# EUROPEAN PATENT APPLICATION

(11) **EP 0 599 265 A1**
(43) Date of publication of application: **01.06.1994**
(21) Application number: 93118809.8
(22) Date of filing: 23.11.1993
(51) Int. Cl.: C07C 271/20, A01N 47/12, C07C 275/14, C07C 333/04, A01N 25/10, C07D 213/38

(54) **Antibacterial, antifungal compound and its production method**

(30) Priority: 24.11.1992 JP 313820/92; 13.10.1993 JP 255861/93
(71) Applicant: Takeda Chemical Industries, Ltd., Osaka-shi, Osaka 541 (JP)
(72) Inventor: Sasaki, Satoshi, Tsukuba-shi Ibaraki (JP); Hata, Yoshio, Tsukuba-shi Ibaraki (JP); Shibata, Sachio, Tsukuba-shi Ibaraki (JP); Hashimoto, Naoto, Suita-shi, Osaka (JP)
(74) Representative: Keller, Günter, Dr.

(57) **Abstract**

A compound represented by the following formula: 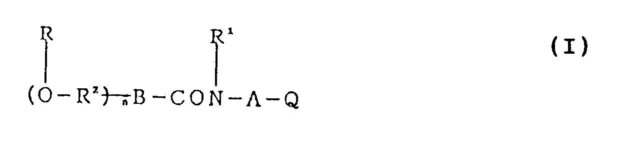
wherein R is an acyl group which may or not be substituted. R¹ is a hydrogen atom, C₁₋₁₀ hydrocarbon group which may or may not be substituted, or an acyl group which may or may not be substituted, R² is a straight chain or branched C₁₋₁₂ alkylene group which may or may not be substituted, A is a straight chain or branched C₁₋₁₀ alkylene group, B is O, NH or S, Q is a tertiary amino or quaternary ammonium group, a nitrogen-containing heterocyclic group which may or may not be substituted, or a nitrogen-containing heterocyclic ammonium group which may or may not be substituted, and n is an integer from 1 to 10, and the method of manufacturing this compound.

## Description

### BACKGROUND OF THE INVENTION

### 1. Field of the Invention

This invention relates to a group of compounds formed by the combination of specific atomic groups having a tertiary amino, quartenary ammonium, nitrogen-containing heterocyclic or nitrogen-containing heterocyclic ammonium group and acyl groups. More specifically, it relates to polymer compounds with antibacterial and antifungal properties, having a tertiary amino, quartenary ammonium, nitrogen-containing heterocyclic or nitrogen-containing heterocyclic ammonium group in a side chain which are useful as materials with low toxicity and long-term antibacterial or antifungal properties, to monomers which are useful as intermediates in the synthesis of said polymers and which themselves exhibit superlative antibacterial, antifungal properties, and to related compounds.

### 2. Description of the Prior Art

In recent years, there has been an increasing demand for greater comfort, cleanliness and convenience in daily life. To achieve this purpose, polymer materials having antibacterial and antifungal properties are for example used in the form of fibers for clothes, or in the form of paints for structural materials and furniture. Many of these materials are made by treating a molded polymer substrate with an antibacterial and antifungal agent, or by mixing such agents with the polymer raw material. There are however many problems associated with their use such as, for example, durability and long-term retention of effect. Also, as these agents have to be highly active in order to have any effect, problems arise as to safety in handling, and they may pose a threat to human health if they leak. Finally, such agents may affect the color and appearance of the substrate. Several polymer compounds having a quartenary ammonium group in a side chain have been proposed as antibacterial or antifungal agents due to their low toxicity (for example Japanese Tokkai Sho 64-26610), however at the present time, there is no material which is really satisfactory.

A polymer having a structure similar to that of the present invention, which constitutes all or part of the vehicle for an antifouling composition, is disclosed for example in Japanese Tokkai Sho 53-124538. This polymer contains a structural unit having a quartenary ammonium salt represented by the following formula: 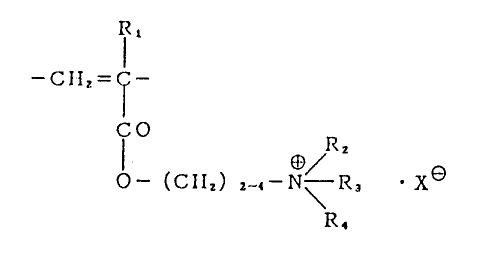
wherein R¹ is H or CH₃, R² and R³ are C₁₋₄ alkyl groups, R⁴ is a C₁₋₁₈ alkyl group, aralkyl group or a -CH₂COOY group (wherein Y is C₁₋₁₈ alkyl or an alkali metal), and X is an anionic group. There is however no mention in this disclosure of the antibacterial and antifungal properties of the polymer.

In view of the aforesaid situation, it is therefore an object of this invention to provide a group of compounds formed by the combination of specific atomic groups having a tertiary amino, quartenary ammonium, nitrogen-containing heterocyclic or nitrogen-containing heterocyclic ammonium group and acyl groups. It is a further object of this invention to provide a polymer which itself has antibacterial and antifungal properties, containing the aforesaid atomic groups in the side chain and can be used to manufacture molded products and paints with the said biological activity in the same way as ordinary polymers. It is a still further object of this invention to provide novel monomers having antibacterial, antifungal properties which are useful as intermediates in the synthesis of said polymers, and related low molecular weight compounds having a tertiary amino, quartenary ammonium, nitrogen-containing heterocyclic or nitrogen-containing heterocyclic ammonium group.

After intensive research to resolve the aforesaid problems associated with conventional materials having antibacterial, antifungal properties, the Inventors synthesized a novel compound having a group represented by the following formula in a side chain: 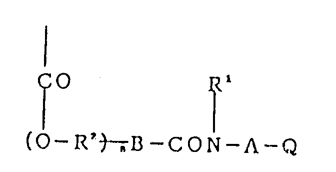
The Inventors discovered that this compound had superlative antibacterial, antifungal properties which exceeded expectations, and that these activities were manifested with every type of the compound of this invention, namely, being indifferent to whether the compound was a polymer, a monomer unit of this polymer, or a compound related to this monomer, and as a result of further research, they formulated the present invention.

### SUMMARY OF THE INVENTION

This invention therefore relates to:
(1) A compound represented by the following formula: 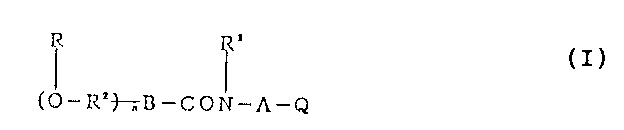 (wherein R is an acyl group which may or may not be substituted, R¹ is a hydrogen atom, C₁₋₁₀ hydrocarbon group which may or may not be substituted, or an acyl group which may or may not be substituted, R² is a straight chain or branched C₁₋₁₂ alkylene group which may or may not be substituted, A is a straight chain or branched C₁₋₁₀ alkylene group, B is O, NH or S, Q is a tertiary amino group, quartenary ammonium group, nitrogen-containing heterocyclic group which may or may not be substituted, or a nitrogen-containing heterocyclic ammonium group which may or may not be substituted, and n is an integer from 1 to 10),
(2) A method of manufacturing a compound having the aforesaid formula (I), and
(3) An antibacterial, antifungal composition comprising a compound having the aforesaid formula (I).

A preferred form of the compound (I) according to this invention is a (co)polymer comprising a repeating unit wherein R is typically represented by the following formula: 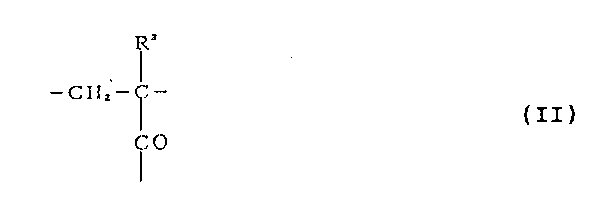
(wherein R³ is a hydrogen atom, or a straight chain or branched C₁₋₆ alkyl group which may or may not be substituted), or the following formula: 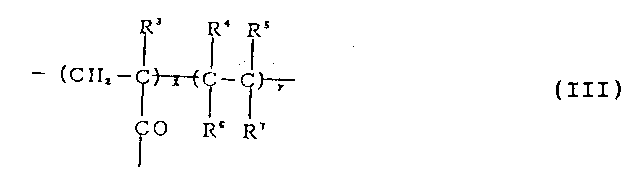
(wherein R³ is a hydrogen atom or a straight chain or branched C₁₋₆ alkyl group, R⁴ is a hydrogen atom, or a straight chain or branched C₁₋₆ alkyl group, R⁵, R⁶, R⁷ are identical or different, and may each respectively be a hydrogen atom, a straight chain or branched C₁₋₆ alkyl group, halogen atom (i.e. chlorine, bromine, iodine or fluorine), cyano group, C₁₋₁₈ alkyl or cycloalkyloxy-carbonyl group, carboxyl group, nitrogen-containing cyclic group which may or may not be substituted, -COOR⁵¹ (wherein R⁵¹ is a hydrogen atom or a hydrocarbon group which may or may not be substituted), -CONR⁵²R⁵³ (wherein R⁵², R⁵³ are respectively hydrogen atoms or C₁₋₁₀ hydrocarbon groups which may or may not be substituted, and R⁵² R⁵³ together form a 4 to 9 membered saturated heterocyclic group with the adjacent nitrogen atom wherein said group may or may not be substituted), -0R⁵⁵ (wherein R⁵⁵ is a hydrogen atom or a C₁₋₁₀ hydrocarbon group which may or may not be substituted), -O-(CH₂CH₂O)ₘ- CH₂CH₂Z (wherein m is an integer from 1 to 10, Z is a hydroxyl, amino, lower acylamino or lower alkoxy group, or a halogen atom), -OCOR⁵⁶ (wherein R⁵⁶ is a hydrogen atom or a C₁₋₁₀ hydrocarbon group which may or may not be substituted), or a group represented by the formula: 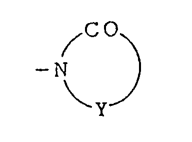
(wherein Y is a C₃₋₁₁ alkylene group which may or may not be substituted), R⁵ and R⁷ may together form a cyclic acid anhydride structure, an N-substituted cyclic imido structure (the substituting group being C₁₋₁₀ alkyl, C₃₋₁₀ cycloalkyl, C₆₋₁₀ aryl, C₇₋₁₀ cycloalkylalkyl or aralkyl) or an unsubstituted cyclic imido structure, and x, y are numbers in mole percent.

In another preferred embodiment of this invention, R in the aforesaid formula (I) is a group typically represented by the formula: 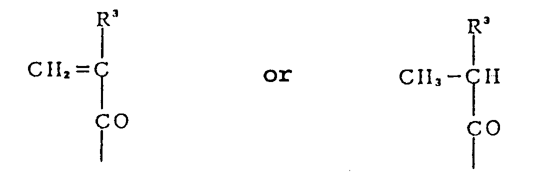
(wherein R³ is a hydrogen atom, or a straight chain or branched C₁₋₆ alkyl group which may or may not be substituted).

The methods of manufacturing the aforesaid preferred compounds and antibacterial, antifungal compositions containing such compounds, are also preferred manufacturing methods and compositions according to this invention.

### DETAILED DESCRIPTION OF THE PREFERRED EMBODIMENTS

The terminology used in this Specification will now be described in further detail.

In this context, the term "hydrocarbon group" refers for example to an alkyl, alkenyl, aryl, aralkyl, cycloalkyl or cycloalkylalkyl group. This "alkyl group' may for example be a straight chain or branched C₁₋₃₀, but more preferably C₁₋₁₈ alkyl group (e.g. methyl, ethyl, n-propyl, i-propyl, n-butyl, i-butyl, tert-butyl, n-pentyl, n-hexyl, n-heptyl, n-octyl, n-nonyl, n-decyl, undecyl, dodecyl, tridecyl, tetradecyl, pentadecyl, heptadecyl, octadecyl). The C₁₋₁₀ alkyl group referred to herein may for example be methyl, ethyl, n-propyl, i-propyl, n-butyl, i-butyl, tert-butyl, n-pentyl, n-hexyl, n-heptyl, n-octyl, n-nonyl or n-decyl. The C₁₋₆ alkyl group referred to herein may for example be methyl, ethyl, n-propyl, i-propyl, n-butyl, i-butyl, tert-butyl, n-pentyl, n-hexyl. The "alkenyl group" may for example be a straight chain or branched C₂₋₁₀, but more preferably C₂₋₄, alkenyl group (e.g. vinyl, allyl, 2-butenyl). The "alkynyl group" may for example be a straight chain or branched C₂₋₁₀, but more preferably C₂₋₄, alkynyl group (e.g. propargyl, 2-butenyl). The "aryl group' may for example be a C₆₋₁₄, but more preferably C₆₋₁₀, aryl group (e.g. phenyl, naphthyl). The "aralkyl group' may for example be:
(1) a C₇₋₁₆ aralkyl group (e.g. a phenyl C₁₋₆ or naphthyl C₁₋₆ alkyl group such as phenylmethyl, phenylethyl, phenylpropyl, phenylbutyl, phenylpentyl, phenylhexyl, α-naphthylmethyl), but more preferably a C₇₋₁₀ aralkyl group (e.g. a phenyl C₁₋₃ alkyl group such as benzyl, phenylethyl, or a diphenyl C₁₋₃ alkyl group such as diphenylmethyl, diphenylethyl),
(2) a C₆₋₁₄ aryl - C₂₋₆ alkenyl group (e.g. a phenyl C₂₋₆ alkenyl group such as styryl, cinnamyl, 4-phenyl-2-butenyl, 4-phenyl-3-butenyl),
(3) C₆₋₁₄ aryl - C₂₋₆ alkynyl (e.g. a phenyl - C₂₋₆ alkynyl group such as phenylethynyl, 3-phenyl-2-propynyl, 3-phenyl-1-propynyl).

The "cycloalkyl group" referred to herein may for example be a C₃₋₁₀ cycloalkyl group (e.g. cyclopropyl, cyclobutyl, cyclopentyl, cyclohexyl, cycloheptyl, cycloctyl, cyclononyl, cyclodecyl) and C₃₋₈ cycloalkyl group (e.g. cyclopropyl, cyclobutyl, cyclopentyl, cyclohexyl, cycloheptyl, cyclooctyl). The "cycloalkylalkyl group" may for example be a C₃₋₈ cycloalkyl - C₁₋₆ alkyl group (e.g. cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl, cycloheptylhexyl).

The "hydrocarbon group" may have one or more substituents.

Groups which may serve as such substituents on this "hydrocarbon group" for example include C₁₋₄ alkyl (e.g. methyl, ethyl, n-propyl, n-butyl, isobutyl, etc.), halogen (e.g. fluorine, chlorine, bromine or iodine), nitro, cyano, hydroxy, C₁₋₄ alkoxy (e.g. methoxy, ethoxy, propoxy, butoxy, isopropyloxy), C₁₋₄ alkylthio (e.g. methylthio, ethylthio, propylthio, isopropylthio, butylthio), amino, mono or di-C₁₋₄ alkylamino (e.g. methylamino, ethylamino, propylamino, dimethylamino, diethylamino, dibutylamino), C₂₋₅ alkylcarbonylamino (e.g. acetylamino, propionylamino, butyrylamino), C₁₋₄ alkylsulfonylamino (e.g. methyl-sulfonylamino, ethylsulfonylamino), C₂₋₅ alkoxycarbonyl, (e.g. methoxycarbonyl, ethoxycarbonyl, propoxycarbonyl, butoxycarbonyl), carboxyl, formyl, C₂₋₇ alkylcarbonyl (e.g. acetyl, propionyl, butyryl, pentanoyl, hexanoyl, heptanoyl), or C₂₋₅ alkylcarbonyloxy (e.g. acetyloxy, propionyloxy). The number of substituent groups is of the order of 1 to 3.

The "C₁₋₁₀ hydrocarbon group" referred to herein is for example a C₁₋₁₀ alkyl, C₁₋₆ alkyl, C₂₋₁₀ alkynyl, C₆₋₁₀ aryl, C₇₋₁₀ aralkyl, C₃₋₁₀ cycloalkyl or C₄₋₁₀ cycloalkylalkyl. In this context, "C₁₋₁₀ alkyl group" refers for example to a straight chain or branched C₁₋₁₀ alkyl group (e.g. methyl, ethyl, n-propyl, i-propyl, n-butyl, i-butyl, tert- butyl, n-pentyl, n-hexyl, n-heptyl, n-octyl, n-nonyl, n-decyl). "C₂₋₁₀ alkenyl group" refers for example to a straight chain or branched C₂₋₁₀, but more preferably a C₂₋₄ alkylene group (e.g. vinyl, allyl, 2-butenyl). "C₂₋₁₀ alkynyl group" refers for example to a straight chain or branched C₂₋₁₀, but more preferably a C₂₋₄ alkynyl group (e.g. propargyl, 2-butynyl). "C₆₋₁₀ aryl group" refers for example to phenyl or naphthyl. "C₇₋₁₀ aralkyl group" refers for example to:
(1) a phenyl - or naphthyl-C₁₋₄ alkyl group (e.g. benzyl, phenylethyl, phenylpropyl, phenylbutyl, phenylpentyl, phenylhexyl, α-naphthylmethyl),
(2) a phenyl - C₂₋₄ alkenyl group (e.g. styryl, cinnamyl, 4-phenyl-2-butenyl, 4-phenyl-3-butenyl),
(3) a phenyl - C₂₋₄ alkynyl group (e.g. phenylethnyl, 3-phenyl-2-propynyl, 3-phenyl-1-propynyl).
"C₃₋₁₀ cycloalkyl group" refers for example to cyclopropyl, cyclobutyl, cyclopentyl, cyclohexyl, cycloheptyl, cyclooctyl or adamantil "C₄₋₁₀ cycloalkylalkyl group" refers for example to cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cyclohexylpropyl, cyclopentylbutyl or cyclohexylbutyl.

Groups which may serve as substituents on this "C₁₋₁₀ hydrocarbon group" are identical to those which are referred to as the substituents on the aforesaid "hydrocarbon group". The number of substituent groups is of the order of 1 to 3.

The "acyl group" referred to herein may for example be the one derived from a carboxylic acid, the one derived from a sulfonic acid, a substituted oxycarbonyl group or a heterocyclic carbonyl group, having 1 to 20, but more preferably 1 to 10, carbon atoms.

The "acyl group derived from a carboxylic acid" may for example be a straight chain or branched C₁₋₁₈, but more preferably C₁₋₁₀ alkylcarbonyl group (e.g. formyl, acetyl, propionyl, isopropionyl, butyryl, pentanoyl, heptanoyl, octanoyl or decanoyl), straight chain or branched C₃₋₁₁, but more preferably C₃₋₅ alkenylcarbonyl group (e.g. acryloyl, methacryloyl, crotonyl), C₄₋₈ cycloalkylcarbonyl group (e.g. cyclopropylcarbonyl, cyclobutylcarbonyl, cyclopentylcarbonyl, cyclohexylcarbonyl), C₃₋₇ cycloalkyl-C₂₋₇ alkylcarbonyl (e.g. cyclopropylmethylcarbonyl, cyclobutylmethylcarbonyl, cyclopentylmethylcarbonyl, cyclohexylmethylcarbonyl, cycloheptylmethylcarbonyl, cyclopropylethylcarbonyl, cyclobutylethylcarbonyl, cyclopentylethylcarbonyl, cyclohexylethylcarbonyl, cycloheptylethylcarbonyl, cyclopropylpropylcarbonyl, cyclobutylpropylcarbonyl, cyclopentylpropylcarbonyl, cyclohexylpropylcarbonyl), C₇₋₁₁ arylcarbonyl group (e.g. benzoyl, 2-naphthoyl), C₈₋₁₁ aralkylcarbonyl group (e.g. benzylcarbonyl, phenylethylcarbonyl), or a C₉₋₁₂ aralkenyl-carbonyl group (e.g. phenylvinyl- carbonyl). The "acyl group derived from a sulfonic acid" may for example be a C₁₋₇ alkylsulfonyl group (e.g. methane sulfonyl, ethane sulfonyl, propane sulfonyl), C₇₋₁₀ aralkylsulfonyl group (e.g. benzylsulfonyl), or C₆₋₁₄ arylsulfonyl group (e.g. benzenesulfonyl, p-toluenesulfonyl). The "substituted oxycarbonyl group" may for example be a C₂₋₁₉ alkoxy-carbonyl group (e.g. methoxycarbonyl, ethoxycarbonyl, tertiary butoxy carbonyl), or a C₈₋₉ aralkyloxycarbonyl group (e.g. benzyloxycarbonyl). The "heterocyclic carbonyl group" may for example be a 5 to 9 member heterocyclic carbonyl group containing 1 to 3 atoms other than carbon such as O, N or S (e.g. 2-furoyl, 3-furoyl, 2-thenoyl, 3-thenoyl, furfuryl-carbonyl, 2- or 3-thenylcarbonyl, thiazolyl-4-carbonyl, imidazol-4-carbonyl, 1,2,3-thiadiazolyl-4-carbonyl, pyridyl-2-(or -3-, or -4-)carbonyl).

Groups which may serve as substituents on this "acyl group" for example include C₁₋₃ alkyl (e.g. methyl, ethyl, propyl, isopropyl), halogen (e.g. fluorine, chlorine, bromine or iodine), nitro, cyano, hydroxy, C₁₋₄ alkoxy (e.g. methoxy, ethoxy, propoxy, butoxy, isopropoxy), C₁₋₄ alkylthio (e.g. methylthio, ethylthio, propylthio, isopropylthio), amino, mono or di-C₁₋₄ alkylamino (e.g. methylamino, ethylamino, propylamino, butylamino, dimethylamino, diethylamino), formylamino, C₁₋₄ alkylcarbonylamino (e.g. acetylamino, propionylamino, butyrylamino), C₁₋₄ alkylsulfonylamino (e.g. methylsulfonylamino, ethylsulfonylamino), C₁₋₄ alkoxycarbonyl, (e.g. methoxycarbonyl, ethoxycarbonyl, propoxycarbonyl, butoxycarbonyl), carboxyl, formyl, C₂₋₇ alkylcarbonyl (e.g. acetyl, propionyl, butyryl), or C₂₋₅ alkylcarbonyloxy (e.g. acetoxy, propionyloxy). The number of substituent groups is of the order of 1 to 3.

The "straight chain or branched C₁₋₆ alkyl group" referred to herein is for example methyl, ethyl, n-propyl, i-propyl, n-butyl, i-butyl, tert-butyl, n-pentyl or n-hexyl.

Groups which may serve as substituents of this "straight chain or branched C₁₋₆ alkyl group" are identical to those which are referred to as the substituent on the aforesaid "hydrocarbon group". The number of substituent groups is of the order of 1 to 3.

In this text, the term "(meth)acrylate" refers to both acrylate and methacrylate. And the term "(meth)acrylamide"refers to both acrylamide and methacrylamide.

In the aforesaid formula, R¹ is a hydrogen atom, C₁₋₁₀ hydrocarbon group which may or may not be substituted, or an acyl group which may or may not be substituted. R¹ is preferably a hydrogen atom, straight chain or branched C₁₋₁₀ alkyl group, C₇₋₁₀ aralkyl group, straight chain or branched C₂₋₁₁ aralkylcarbonyl group, formyl, C₃₋₅ alkenylcarbonyl group (e.g. acryloyl, methacryloyl) or C₄₋₆ cycloalkylcarbonyl group (e.g. cyclo-propylcarbonyl, cyclopropylacetyl, methylcyclopropylcarbonyl). More preferably, R¹ is a hydrogen atom, straight chain or branched C₁₋₄ alkyl group (e.g. methyl, ethyl, propyl, butyl), straight chain or branched C₂₋₉ alkylcarbonyl group (e.g. acetyl, propionyl, isobutyryl, butyryl, pentanoyl, hexanoyl, heptanoyl, octanoyl), formyl, and straight chain or branched C₂₋₅ alkylcarbonyl group (e.g., acetyl, propionyl, isobutyryl, butyryl) are to be preferred.

R² is a straight chain or branched C₁₋₁₂ alkylene group. R² may be methylene, ethylene, 1,3-propylene, 1,2-propylene, 1,4-butylene, 1,3-butylene, 1,5-pentylene, 1,6-hexylene, 1,8-octylene, 1,10-decylene or 1,12-dodecylene. Of these, straight chain or branched C₁₋₆ alkylene groups (e.g. methylene, ethylene, 1,3-propylene, 1,2-propylene, 1,4-butylene, 1,3-butylene, 1,5-pentylene, 1,6-hexylene) are to be preferred, and straight chain or branched C₁₋₄ alkylene groups (e.g. methylene, ethylene, 1,3-propylene, 1,2-propylene, 1,4-butylene) are particularly to be preferred. Ethylene, 1,3-propylene and 1,2-propylene are especially preferably used.

Groups which may serve as substituents on R² are identical to those which are referred to as the substitents on the aforesaid "hydrocarbon group" (excepting C₁₋₄ alkyl). Preferred examples are hydroxyl, formylamino, C₁₋₄ alkylcarbonylamino (e.g. acetylamino, propionylamino, butyrylamino), C₁₋₄ alkoxy (e.g. methoxy, ethoxy, propoxy, butoxy, isopropoxy), and halogen (e.g. fluorine, chlorine, bromine or iodine).

R³ is a hydrogen atom, or a straight chain or branched C₁₋₆ alkyl group. Preferred examples are hydrogen, and C₁₋₃ alkyl (e.g. methyl).

R⁴ is a hydrogen atom, or a straight chain or branched C₁₋₆ alkyl group which may or may not be substituted. Preferred examples are hydrogen, and C₁₋₃ alkyl (e.g. methyl).

R⁵, R⁶, R⁷ are, same or different, a hydrogenatom, a straight chain or branched C₁₋₆ alkyl, a halogen atom (e.g. chlorine, bromine, iodine, fluorine), a cyano group, a C₁₋₁₈ alkyl or cycloalkyloxycarbonyl group, carboxyl group, a nitrogen-containing cyclic group which may or may not be substituted, -COOR⁵¹ (wherein R⁵¹ is a hydrogen atom or a hydrocarbon group which may or may not be substituted), -CONR⁵²R⁵³ (wherein R⁵², R⁵³ are each a hydrogen atom or a C₁₋₁₀ hydrocarbon group which may or may not be substituted, and R⁵², R⁵³ together may form with the adjacent nitrogen atom a 4 to 9 membered saturated heterocyclic group which may or may not be substituted), -0R⁵⁵ (wherein R⁵⁵ is a hydrogen atom or a C₁₋₁₀ hydrocarbon group which may or may not be substituted), -O-(CH₂CH₂O)ₘ-CH₂CH₂Z (wherein m is an integer from 1 to 10, Z is a hydroxyl, amino, lower acylamino or lower alkoxy group, or a halogen atom), or -OCOR⁵⁶ (wherein R⁵⁶ is a hydrogen atom or a C₁₋₁₀ hydrocarbon group which may or may not be substituted), or a group represented by the following formula: 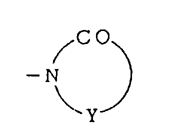
(wherein Y is a C₃₋₁₁ alkylene group which may or may not be substituted).

If R⁵ is halogen, the halogen may be fluorine, chlorine, bromine or iodine.

If R⁵ is a "nitrogen-containing cyclic group which may or may not be substituted", the "nitrogen-containing cyclic group" may for example be a 5 to 9 membered cyclic group having 1 to 3 nitrogen atoms. Specific examples are unsaturated nitrogen-containing cyclic groups such as pyridyl, pyradinyl, quinolyl, imidazolyl, triazolyl, carbazolyl, imidazopyridinyl or isoquinolyl, and saturated nitrogen-containing cyclic groups such as pyrrolidinyl, piperidinyl, hexamethyleneiminyl, heptamethyleneiminyl or piperazinyl. Pyridyl and related compounds are particularly preferred.

The nitrogen ring may or may not have a substituent group on one or more of its C atoms and/or N atoms. Examples of such substituent groups on an N atom are straight chain or branched C₁₋₃₀ alkyl (preferably C₆₋₂₂), straight chain or branched C₂₋₄ alkenyl, straight chain or branched C₂₋₄ alkynyl, aryl (e.g. phenyl, naphthyl), C₇₋₁₀ aralkyl (e.g. benzyl, phenylethyl), and C₇₋₁₀ cycloalkyl-C₁₋₃-alkyl (e.g. cyclohexylmethyl). These groups may be further substituted by about 1 to 3 substituent groups, for example C₁₋₃ alkyl (e.g. methyl, ethyl), halogen (e.g. fluorine, chlorine, bromine, iodine), nitro, cyano, C₁₋₄ alkoxy (e.g. methoxy, ethoxy, propoxy, butoxy, isopropoxy), C₁₋₄ alkylthio (e.g. methylthio, ethylthio, propylthio, isopropylthio), C₂₋₅ alkylcarbonylamino (e.g. acetylamino, propionylamino, butyrylamino), C₁₋₄ alkylsulfonylamino (e.g. methylsulfonylamino, ethylsulfonylamino), C₂₋₅ alkoxycarbonyl (e.g. methoxycarbonyl, ethoxycarbonyl, propoxycarbonyl), C₂₋₇ alkylcarbonyl (e.g. methylcarbonyl, ethylcarbonyl, propylcarbonyl), or C₂₋₅ alkylcarbonyloxy (e.g. acetyloxy, ethylcarbonyloxy). Examples of such substituent groups on a C atom are C₁₋₃ alkyl (e.g. methyl, ethyl), halogen (e.g. fluorine, chlorine, bromine, iodine), nitro, cyano, hydroxyl, C₁₋₄ alkoxy (e.g. methoxy, ethoxy, propoxy, butoxy, isopropoxy), C₁₋₄ alkylthio (e.g. methylthio, ethylthio, propylthio, isopropylthio), C₂₋₅ alkylcarbonylamino (e.g. acetylamino, propionylamino, butyrylamino), carboxamide which may or may not have one or two substituent group, C₁₋₄ alkyl, C₇₋₁₁ aralkyl or phenyl, on its N atom, C₁₋₄ alkyl - sulfonylamino (e.g. methylsulfonylamino, ethylsulfonylamino), C₂₋₅ alkoxycarbonyl (e.g. methoxycarbonyl, ethoxycarbonyl, propoxycarbonyl), formyl, C₂₋₇ alkylcarbonyl (e.g. methylcarbonyl, ethylcarbonyl, propylcarbonyl), or C₁₋₄ alkyl - carbonyloxy (e.g. acetyloxy, ethylcarboxy). The number of these substituent groups is of the order of 1 to 3.

Preferred examples of the aforesaid nitrogen-containing heterocyclic group are 5 to 9 membered saturated or unsaturated nitrogen-containing heterocyclic groups wherein the C atom may or may not be substituted by, for example, C₁₋₃ alkyl group (e.g. methyl, ethyl), halogen atom (e.g. fluorine, chlorine, bromine or iodine), cyano, hydroxy, C₁₋₄ alkoxy (e.g. methoxy, ethoxy, propoxy, butoxy, isopropoxy), C₂₋₅ alkylcarbonylamino (e.g. acetylamino, propionylamino, butyrylamino), carboxamido which may or may not have one or two substituent group, C₁₋₄ alkyl, C₇₋₁₁ aralkyl or phenyl, C₂₋₅ alkoxycarbonyl (e.g. methoxycarbonyl, ethoxycarbonyl, propoxycarbonyl), formyl, C₂₋₇ alkylcarbonyl (e.g. methylcarbonyl, ethylcarbonyl, propylcarbonyl), C₂₋₅ alkylcarbonyloxy (e.g. acetyloxy, ethylcarbonyloxy). Particularly preferred examples of the 5 to 9 membered saturated or unsaturated nitrogen-containing heterocyclic group are C₁₋₃ alkyl (e.g. methyl, ethyl), halogen atom (e.g. fluorine, chlorine, bromine or iodine), C₁₋₄ alkoxy (e.g. methoxy, ethoxy, propoxy, butoxy, iso-propoxy). Specific examples are pyrrolyl, 1-imidazolyl, 2-imidasolyl, 1-methyl-2-imidasolyl, 1-triazolyl, 2-,3-,4-pyridyl, 1-carbazolyl and pyrazinyl. If the N atom in the 5 to 9 membered unsaturated nitrogen-containing ring is substituted, this nitrogen atom will have a positive charge, hence it will be associated with an anionic group having a negative charge. Such negatively charged groups are of the type X⁻ which will be described hereinafter.

R⁵¹ may for example be a hydrogen atom, or a straight chain or branched C₁₋₁₈ alkyl group which may or may not be substituted (e.g. methyl, ethyl, n-propyl, isopropyl, n-butyl, isobutyl, tert-butyl, n-pentyl, n-hexyl, n-heptyl, n-octyl, n-nonyl, n-decyl, undecyl, dodecyl, tridecyl, tetradecyl, pentadecyl, heptadecyl, octadecyl). Straight chain or branched c₁₋₁₀ alkyl groups (e.g. methyl, ethyl, n-propyl, isopropyl, n-butyl, isobutyl, tert-butyl, n-pentyl, n-hexyl, n-heptyl, n-octyl, n-nonyl), and C₇₋₁₀ aralkyl groups, are particularly preferred. Hydroxyl is especially preferred as the substituent group.

The 4 to 9 membered saturated heterocyclic ring which may or may not be substituted, and which may be formed by R⁵², R⁵³ and the adjacent nitrogen atoms, may for example be a 4 to 9 membered saturated heterocyclic group containing, in addition to the said N atom, 1 or 2 heteroatoms such as O, N or S. Specific examples are aziridino, pyrrolidino, piperidino, morpholino, pyrazino or azepino, but 5 to 6 membered saturated heterocyclic rings are preferable. These 4 to 9 membered saturated heterocyclic groups may be substituted by 1 to 3 substituents, for example a straight chain or branched C₁₋₆ alkyl group (e.g. methyl, ethyl, n-propyl, isopropyl, n-butyl, isobutyl, tert-butyl), C₇₋₁₀ aralkyl group (e.g. benzyl), C₆₋₁₀ aryl group (e.g. phenyl, naphthyl), oxo, C₁₋₆ alkoxy (e.g. methoxy, ethoxy), or C₂₋₇ alkylcarbonyl group (e.g. acetyl), formyl or a halogen atom (e.g. fluorine, chlorine, bromine, iodine).

Examples of 4 to 9 membered saturated heterocyclic groups which may or may not be substituted are aziridino, pyrrolidino, piperidino, morpholino, N4-methylpyrazino, azepino, N4-benzylpyrazino, 2-oxo-pyrrolidino and 2-oxo-piperidino.

R⁵² and R⁵³ are, same or different, preferably respectively hydrogen atoms, straight chain or branched C₁₋₁₀ alkyl groups (e.g. methyl, ethyl, n-propyl, isopropyl, n-butyl, isobutyl, tert-butyl, n-pentyl, n-hexyl, n-heptyl, n-octyl, n-nonyl), C₇₋₁₀ aralkyl groups (e.g. benzyl, phenylethyl) or C₆₋₁₀ aryl groups (e.g. phenyl, naphthyl) groups, or alternatively R⁵², R⁵³ form a 4 to 9 membered saturated heterocyclic ring together with the adjacent nitrogen atom wherein this ring may or may not be substituted by a straight chain or branched C₁₋₆ alkyl group (e.g. methyl, ethyl, n-propyl, isopropyl, n-butyl, isobutyl, tert-butyl), C₇₋₁₀ aralkyl group (e.g. benzyl), C₆₋₁₀ aryl group (e.g. phenyl, naphthyl), oxo group, C₁₋₆ alkoxy group (e.g. methoxy, ethoxy) or C₂₋₇ alkylcarbonyl group (e.g. acetyl), formyl or a halogen atom (e.g. fluorine, chlorine bromine, iodine).

R⁵⁵ may for example be a hydrogen atom or a C₁₋₄ alkoxy group. Alternatively, R⁵⁴ may be a straight chain or branched C₁₋₁₈ alkyl group (e.g. methyl, ethyl, n-propyl, isopropyl, n-butyl, isobutyl, tert-butyl, n-pentyl, n-hexyl, n-heptyl, n-octyl, n-nonyl, n-decyl, undecyl, dodecyl, tridecyl, tetradecyl, pentadecyl, heptadecyl, octadecyl), but more preferably a C₁₋₁₀ alkyl group (e.g. methyl, ethyl, n-propyl, isopropyl, n-butyl, isobutyl, tert-butyl, n-pentyl, n-hexyl, n-heptyl, n-octyl, n-nonyl), C₇₋₁₀ aralkyl group (e.g. benzyl) and C₆₋₁₀ aryl group (e.g. phenyl, naphthyl), wherein these groups may or may not be substituted by about 1 to 3 substituent groups such as halogen atoms. It is particularly preferred that R⁵⁴ is a hydrogen atom, C₁₋₄ alkoxy group or C₁₋₁₀ alkyl group which may or may not be substituted by a halogen atom or the like.

m is an integer from 1 to 10, but 1, 2, 3 and 4 are to be preferred.

Z is a hydroxy, amino, formylamino lower acylamino or lower alkoxy group, or a halogen atom. The lower acylamino group may for example be a C₂₋₇ acylamino group (e.g. acetylamino, ethylcarbonylamino). The lower alkoxy group may for example be a C₁₋₆ alkoxy group (e.g. methoxy, ethoxy, propoxy). The halogen atom may for example be fluorine, chlorine, bromine or iodine. It is particularly preferred that Z is a hydroxyl or amino group, or a halogen atom.

R⁵⁶ may for example be a hydrogen atom, or a straight chain or branched C₁₋₁₀ alkyl group (e.g. methyl, ethyl, n-propyl, isopropyl, n-butyl, isobutyl, tert-butyl, n-pentyl, n-hexyl, n-heptyl, n-octyl, n-nonyl), C₇₋₁₀ aralkyl group (e.g. benzyl), or C₆₋₁₀ aryl group (e.g. phenyl, naphthyl). It is preferable that R⁵ is a group represented by the formula: 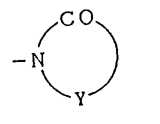
(wherein Y is a C₃₋₁₁ alkylene group which may or may not be substituted, e.g. 1,3-propylene, 1,4-butylene, 1,5-pentylene, 1,6-hexylene, 1,7-heptylene, 1,8-octylene, 1,9-nonylene and 1,10-decylene), 2-oxo-pyridino, 2-oxo-piperidino, 2-oxo-azepino, 2-oxo-1-azacyclooctan-2-yl or 2-oxo-1-azacycloundecan-1-yl, which may or may not be substituted by for example a C₁₋₆ alkyl group (e.g. methyl, ethyl, propyl), halogen atom (e.g. fluorine, chlorine, bromine, iodine), or a C₁₋₄ alkoxy group (e.g. methoxy, ethoxy).

It is more preferred that R⁵ is a group represented by the following formula: 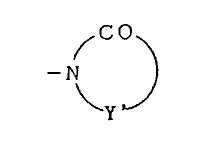
(wherein Y' is a methylene or ethylene group which may or may not be substituted). The resulting group in this case is a 2-oxo-pyrrolidono or a 2-oxo-piperidino group, which may or may not be substituted by for example a C₁₋₆ alkyl group (e.g. methyl, ethyl, propyl), a halogen atom (e.g. fluorine, chlorine, bromine, iodine), or a C₁₋₄ alkoxy group (e.g. methoxy, ethoxy).

R⁵ is preferably a hydrogen atom (e.g. fluorine, chlorine, bromine, iodine) a cyano group, a straight chain or branched C₁₋₆ alkyl group, C₆₋₁₀ aryl group which may or may not be substituted (e.g. phenyl or naphthyl which may be substituted by (1) a C₁₋₆ alkyl group which may be substituted by a halogen atom or a hydroxyl group, (2) a halogen atom, (3) a nitro group, or (4) a C₁₋₆ alkoxy group, examples of such groups being phenyl, methylphenyl, ethylphenyl, chlorophenyl, fluorophenyl, chloromethylphenyl, hydroxymethylphenyl, methoxyphenyl, nitrophenyl and naphthyl), -COOR^{51'} (wherein R^{51'} is a hydrogen atom, a C₁₋₈ alkyl group or a C₇₋₁₀ aralkyl group), -CONR^{52'}R^{53'} (wherein R^{52'}, R^{53'} are, same or different, hydrogen atoms, or C₁₋₆ alkyl, C₃₋₁₀ cycloalkyl, C₇₋₁₀ aralkyl or C₃₋₁₀ cycloalkylalkyl groups, and R^{52'}, R^{53'} may form a 5 to 6 member saturated heterocyclic ring with the adjacent nitrogen atom wherein said ring may or may not be substituted), -OR^{55'} (wherein R^{55'} is a hydrogen atom, or a C₁₋₁₀ alkyl, C₆₋₁₀ aryl or C₇₋₁₀ aralkyl group), -OCOR^{56'} (wherein R^{56'} is a hydrogen atom, C₁₋₁₀ aralkyl group, C₆₋₁₀ aryl group or C₇₋₁₀ aralkyl group), or a group represented by the following formula: 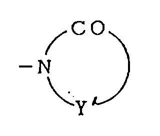
(wherein Y' is a methylene or ethylene group which may or may not be substituted). Of these, hydrogen, C₁₋₆ alkyl, C₆₋₁₀ aryl (e.g. phenyl, naphthyl) and -COOR^{51''} (wherein R^{51''} is a C₁₋₆ alkyl group which may or may not be substituted by hydroxyl), are particularly preferred.

A is a straight chain or branched C₁₋₁₀ alkylene group, for example methylene, ethylene, 1,3-propylene, 1,2-propylene, 1,4-butylene, 1,2-butylene, 1,5-pentylene, 1,6-hexylene, 1,7-heptylene, 1,8-octylene, 1,9-nonylene, 2-methyl-1,3-propylene or 1,10-decylene. Of these, straight chain or branched C₁₋₆ alkylene (e.g. methylene, ethylene, 1,3-propylene, 1,2-propylene, 1,4-butylene or 1,6-hexylene) is to be preferred, with ethylene, 1,3-propylene or 1,2-propylene being particularly preferred.

B is O, NH or S, but O and NH are preferred and O is particularly preferred.

Q is a tertiary amino group, a quaternary ammonium group, a nitrogen-containing heterocyclic group or a nitrogen-containing heterocyclic ammonium group which may or may not be substituted.

If Q is a tertiary amino group, it may for example be a tertiary amino group represented by the following formula: 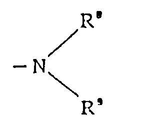
where R⁸, R⁹ are both hydrocarbon groups which may or may not be substituted, or if Q is a quaternary ammonium, it may be a group represented by the following formula: 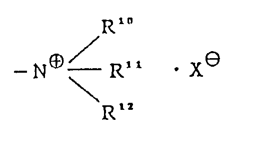
where R¹⁰, R¹¹, R¹² are hydrocarbon groups which may or may not be substituted, and X⁻ is an anionic group.

R⁸, R⁹, R¹⁰, R¹¹ and R¹² are each preferably a C₁₋₃₀ alkyl, C₂₋₁₀ alkenyl, C₇₋₁₈ aralkyl or C₃₋₇ cycloalkyl - C₁₋₆ alkyl group. Any combination of R⁸ and R⁹, R¹⁰, R¹¹ and R¹² is possible, as the each is C₁₋₃₀ alkyl, C₂₋₁₀ alkenyl, C₇₋₈ aralkyl or C₃₋₇ cycloalkyl-C₁₋₆ alkyl group, but the following combinations of R⁸ and R⁹, R¹⁰, R¹¹ and R¹² are particularly preferred:
(1) R⁸ is a C₇₋₁₈ alkyl, C₂₋₄ alkenyl or C₃₋₇ cycloalkyl - C₁₋₆ alkyl group, R⁹ is a C₁₋₃₀ alkyl group.
(2) R⁸ is a C₇₋₁₀ alkyl, C₂₋₄ alkenyl or C₃₋₇ cycloalkyl - C₁₋₆ alkyl group, R⁹ is a C₁₋₃₀ alkyl group.
(3) R⁸ is a C₇₋₁₀ alkyl, C₂₋₄ alkenyl or C₃₋₇ cycloalkyl - C₁₋₆ alkyl group, R⁹ is a C₆₋₂₂ alkyl group.
(4) R⁸ is a C₇₋₁₀ aralkyl group, R⁹ is a C₁₋₃₀ alkyl group.
(5) R⁸ is a C₇₋₁₀ aralkyl group, R⁹ is a C₆₋₂₂ alkyl group.
(6) R⁸ is a benzyl group, R⁹ is a C₆₋₂₂ alkyl group.
(7) R¹⁰ is a C₇₋₁₈ alkyl, C₂₋₄ alkenyl or C₃₋₇ cycloalkyl - C₁₋₆ alkyl group, R¹¹ is a C₁₋₃₀ alkyl group, R¹² is a C₁₋₆ alkyl.
(8) R¹⁰ is a C₇₋₁₀ alkyl, C₂₋₄ alkenyl or C₃₋₇ cycloalkyl - C₁₋₆ alkyl group, R¹¹ is a C₁₋₃₀ alkyl group, R¹² is a C₁₋₆ alkyl group.
(9) R¹⁰ is a C₇₋₁₀ alkyl, C₂₋₄ alkenyl or C₃₋₇ cycloalkyl - C₁₋₆ alkyl group, R¹¹ is a C₆₋₂₂ alkyl group, R¹² is a C₁₋₆ alkyl group.
(10) R¹⁰ is a C₇₋₁₈ alkyl group, R¹¹ is a C₁₋₃₀ alkyl group, R¹² is a C₁₋₆ alkyl group.
(11) R¹⁰ is a C₇₋₁₀ alkyl group, R¹¹ is a C₁₋₃₀ alkyl group, R¹² is a C₁₋₆ alkyl group.
(12) R¹⁰ is a C₇₋₁₀ alkyl group, R¹¹ is a C₆₋₂₂ alkyl group, R¹² is a C₁₋₆ alkyl group.
(13) R¹⁰ is a benzyl group, R¹¹ is a C₆₋₂₂ alkyl group, R¹² is a C₁₋₆ alkyl group.

Herein, the C₇₋₁₀ aralkyl group may be a phenyl - C₁₋₃ alkyl group such as benzyl or phenylethyl. The C₁₋₆ alkyl group may for example be methyl, ethyl, n-propyl, i-propyl, n-butyl, i-butyl, n-pentyl or n-hexyl. The C₂₋₄ alkenyl group may for example be allyl or 2-butenyl. The C₆₋₂₂ alkyl group may for example be hexyl, heptyl, octyl, nonyl, decyl, undecyl, dodecyl, tridecyl, tetradecyl, pentadecyl, octadecyl, nonadecyl, eicosyl, eicosaundecyl, eicosadodecyl or 2-ethylhexyl. The number of carbon atoms in these groups is preferably of the order of 10 or more, and more preferably 12 to 18.

Groups which may substitute R⁸, R⁹, R¹⁰, R¹¹ and R¹² are each, for example, C₁₋₃ alkyl (e.g. methyl, ethyl, propyl), halogen (e.g. fluorine, chlorine, bromine, iodine), or nitro, cyano, hydroxy, C₁₋₄ alkoxy (e.g. methoxy, ethoxy, propoxy, butoxy, isopropoxy), C₁₋₄ alkylthio (e.g. methylthio, ethylthio, propylthio, isopropylthio butylthio), C₂₋₅ alkylcarbonylamino (e.g. acetylamino, propionylamino, butyrylamino), C₁₋₄ alkylsulfonylamino (e.g. methylsulfonylamino, ethylsulfonylamino), formyl, C₂₋₇ alkylcarbonyl (e.g. methylcarbonyl, ethylcarbonyl, propylcarbonyl), or C₂₋₅ alkylcarboxy (e.g. acetylcarboxy, ethylcarboxy). The number of substituent groups is of the order of 1 to 3.

X⁻ is an anionic group. X⁻ may for example be an anionic group derived from a halogen atom (e.g. F⁻, Cl⁻, I⁻, Br⁻), or a lower alkylsulfonate ion (e.g. methylsulfonate ion). F⁻, Cl⁻, I⁻, Br⁻ and methylsulfonate ion are particularly to be preferred.

The "nitrogen-containing heterocyclic group" of the "nitrogen-containing heterocyclic group which may or may not be substituted" represented by Q, may for example be a 5 to 9 membered heterocyclic group containing 1 to 3 nitrogen atoms represented by the following formula: 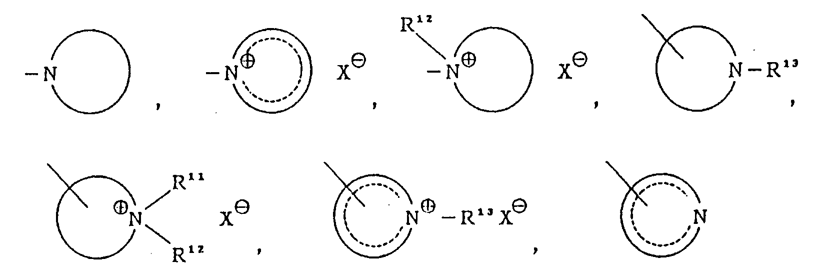
Herein, R¹³ is a hydrocarbon group which may or may not be substituted. X is an anionic group.

Examples are unsaturated nitrogen-containing heterocyclic groups such as pyridyl, pyrazinyl, pyrimidinyl, quinolyl, imidazolyl, triazolyl, carbasolyl, imidazopyridinyl and isoquinolyl, or saturated nitrogen-containing heterocyclic groups such as pyrrolidinyl, piperidinyl, hexamethyleneiminyl, heptamethyleneiminyl and piperazinyl. Pyridyl is particularly preferred.

The nitrogen ring may or may not have a substituent group on one or more of its C atoms and/or N atoms. Examples of such substituent groups on an N atom are straight chain or branched C₁₋₃₀ alkyl (preferably C₆₋₂₂), straight chain or branched C₂₋₄ alkenyl, straight chain or branched C₂₋₄ alkinyl, aryl (e.g. phenyl, naphthyl), C₇₋₁₀ aralkyl (e.g. benzyl, phenylethyl), and C₇₋₁₀ cycloalkyl- alkyl (e.g. cyclohexylmethyl). These groups may be further substituted by about 1 to 3 substituent groups, for example C₁₋₃ alkyl (e.g. methyl, ethyl), halogen (e.g. fluorine, chlorine, bromine, iodine), or nitro, cyano, C₁₋₄ alkoxy (e.g. methoxy, ethoxy, propoxy, butoxy, isopropoxy), C₁₋₄ alkylthio (e.g. methylthio, ethylthio, propylthio, isopropylthio), C₂₋₅ alkylcarbonylamino (e.g. acetylamino, propionylamino, butyrylamino), C₁₋₄ alkylsulfonylamino (e.g. methylsulfonylamino, ethylsulfonylamino), C₂₋₅ alkoxycarbonyl (e.g. methoxycarbonyl, ethoxycarbonyl, propoxycarbonyl), C₂₋₇ alkylcarbonyl (e.g. methylcarbonyl, ethylcarbonyl, propylcarbonyl), or C₂₋₅ alkylcarbonyloxy (e.g. acetyl, ethylcarboxy). Examples of such substituent groups on a C atom are C₁₋₃ alkyl (e.g. methyl, ethyl), halogen (e.g. fluorine, chlorine, bromine, iodine), or nitro, cyano, hydroxy, C₁₋₄ alkoxy (e.g. methoxy, ethoxy, propoxy, butoxy, isopropoxy), C₁₋₄ alkylthio (e.g. methylthio, ethylthio, propylthio, isopropylthio), C₂₋₅ alkylcarbonylamino (e.g. acetylamino, propionylamino, butyrylamino), C₁₋₄ alkylsulfonylamino (e.g. methylsulfonylamino, ethylsulfonylamino), C₂₋₅ alkoxycarbonyl (e.g. methoxycarbonyl, ethoxycarbonyl, propoxycarbonyl), C₂₋₇ alkylcarbonyl (e.g. methylcarbonyl, ethylcarbonyl, propylcarbonyl), or C₂₋₅ alkylcarbonyloxy (e.g. acetyl, ethylcarboxy). The number of these substituents is of the order of 1 to 3.

Preferred examples of the aforesaid nitrogen-containing heterocyclic group are 5 to 9 membered unsaturated nitrogen-containing heterocyclic groups wherein the N atom may or may not be substituted by, for example, a straight chain or branched C₁₋₃₀ alkyl group (particularly C₆₋₂₂ alkyl), straight chain or branched C₂₋₄ alkenyl group, straight chain or branched C₂₋₄ alkynyl group, aryl group (e.g. phenyl, naphthyl), C₇₋₁₀ aralkyl group (e.g. benzyl, phenylethyl), or C₇₋₁₀ cycloalkylalkyl group (e.g. cyclohexylmethyl). Particularly preferred are (1) unsubstituted pyridyl (2-, 3- or 4- pyridyl), or (2) pyridyl or pyridinium wherein the N atom is substituted by a straight chain or branched C₆₋₂₂ alkyl group. If the N atom in the 5 to 9 member unsaturated nitrogen-containing heterocyclic ring is substituted, this nitrogen atom will have a positive charge, hence it will be associated with an anion having a negative charge. Such anions are of the aforesaid type X⁻.

x and y are numbers in mole percent. Preferred values of x:y are from 100:0 to 0.01:99.99, from 100:0 to 0.1:99.9, from 100:0 to 1:99, from 20:80 to 1:99, and from 10:90 to 3:97. Of these, the values from 20:80 to 1:99 and from 10:90 to 3:97 are particularly to be preferred, 50:50 being especially common.

R is an acyl group which may or may not be substituted. The acyl group referred to herein is preferably an acyl group derived from a carboxylic acid or an acyl group derived from a sulfonic acid having 1 to 20, but more preferably 1 to 10, carbon atoms. Examples are a straight chain or branched C₂₋₁₉, but more preferably C₂₋₁₁ alkylcarbonyl group, a straight chain or branched C₃₋₁₁, but more preferably C₃₋₅ alkenylcarbonyl group, a C₄₋₈ cycloalkylcarbonyl, C₃₋₇ cycloalkyl - C₂₋₇ alkylcarbonyl, C₇₋₁₁ aryl - carbonyl, C₈₋₁₁ aralkylcarbonyl, C₇₋₁₀ aralkenyl - carbonyl, straight chain or branched C₁₋₇ alkylsulfonyl, C₇₋₁₀ aralkylsulfonyl or C₆₋₁₄ arylsulfonyl group, or a 5 to 9 membered heterocyclic carbonyl group containing 1 to 3 atoms other than carbon such as O, N or S (e.g. 2-furoyl, 2-thenoyl, 4-thiazolylcarbonyl, 4-imidasolylcarbonyl, 1,2,3-thiadiazolyl-4-carbonyl, or 2-, 3- or 4-pyridylcarbonyl). Of these, a straight chain or branched C₂₋₁₉ alkylcarbonyl, straight chain or branched C₃₋₁₁ alkenylcarbonyl, C₄₋₈ cycloalkylcarbonyl, C₃₋₇ cycloalkyl - C₂₋₇ alkylcarbonyl, C₇₋₁₅ arylcarbonyl, C₈₋₁₁ aralkyl - carbonyl, or C₉₋₁₂ aralkenylcarbonyl group is preferred, and a straight chain or branched C₂₋₁₁ alkylcarbonyl, C₃₋₁₁ alkenylcarbonyl or C₃₋₅ alkenylcarbonyl (e.g. acryloyl, methacryloyl) group is particularly preferred. Further, the acyl group which may or may not be substituted in the compound of this invention is preferably represented by the formula: 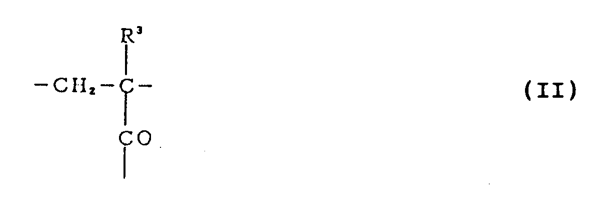
(wherein R³ has the same significance as hereintofore defined), or by the formula: 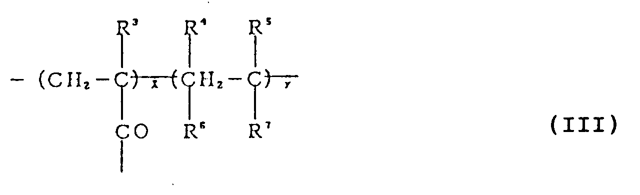
(wherein R³, R⁴, R⁵, R⁶, R⁷, x and y have the same significance as hereinbefore defined). In these cases, the compound of this invention is a (co)polymer comprising a repeating unit having the formula (I) wherein R is the group (II) or (III).

If R is non-polymeric, these acyl groups, which is represented by R, may or may not have 1 to 3 substituent groups, for example C₁₋₃ alkyl (e.g. methyl, ethyl), halogen (e.g. fluorine, chlorine, bromine, iodine), nitro, cyano, hydroxy, C₁₋₄ alkoxy (e.g. methoxy, ethoxy, propoxy, butoxy, isopropoxy, phenoxy), C₁₋₄ alkylthio (e.g. methylthio, ethylthio, propylthio, isopropylthio), amino, mono or di-C₁₋₄ alkylamino (e.g. methylamino, ethylamino, propylamino, dimethylamino, diethylamino), formylamino, C₁₋₄ alkylcarbonylamino (e.g. acetylamino, propionylamino, butyrylamino), C₁₋₄ alkylsulfonylamino (e.g. methylsulfonylamino, ethylsulfonylamino), C₂₋₅ alkoxycarbonyl, (e.g. methoxycarbonyl, ethoxycarbonyl, propoxycarbonyl), carboxyl, formyl, carbamoyl, C₂₋₇ alkylcarbonyl (e.g. methylcarbonyl, ethylcarbonyl, propyl-carbonyl), C₂₋₅ alkylcarbonyloxy (e.g. acetyloxy, ethylcarbonyloxy), or a 5 to 9 membered heterocyclic group (e.g. furyl, pyridyl, thienyl, thiazolyl, imidazolyl) which may or may not be substituted .

Specific examples of R are formyl, acetyl, propionyl, butyryl, isobutyryl, sec-butyryl, pentanoyl, hexanoyl, octanoyl, nonanoyl, decanoyl, tetradecanoyl, hexadecanoyl, octadecanoyl, dodecanoyl, cyclopropanecarbonyl, cyclobutanecarbonyl, cyclohexanecarbonyl, cyclo-hexylacetyl, adamantanecarbonyl, chloroacetyl, dichloroacetyl, iodoacetyl, acryloyl, methacryloyl, cinnamoyl, p-hydroxycinnamoyl, p-chlorocinnamoyl, p-methoxycinnamoyl, benzoyl, toluoyl, phenylacetyl, p-chlorophenylacetyl, phenylpropionyl, naphthalenecarbonyl, methoxycarbonyl, ethoxycarbonyl, phenoxyacetyl, p-methoxyphenoxyacetyl, p-hydroxyphenoxypropionyl, furan-2-carbonyl, thiophene-2-carbonyl, furfurylcarbonyl, tetrahydrofurfurylcarbonyl, tetrahydrothenoylcarbonyl 2-acetamidothiazole-4-carbonyl, 2-methylimidazol-4-carbonyl, 1-, 2-, 3-thiazole-4-carbonyl, pyrimidine-2-(or -3-, -4-)carbonyl, 2-(2-formamido-4-thiazolyl)acetyl, methanesulfonyl, propane-sulfonyl, phenylsulfonyl, p-toluenesulfonyl, and benzylsulfonyl. Of these, straight chain or branched C₃₋₁₁ alkenylcarbonyl groups such as acryloyl and methacryloyl are particularly often used.

The compound having the formula (I), and the repeating unit having the formula (I) wherein R is the group (II) or (III), is preferably a combination of the aforesaid groups within suitable limits.

Of the compounds (I), those having a polymerizable reactive double bond are typified by the formula: 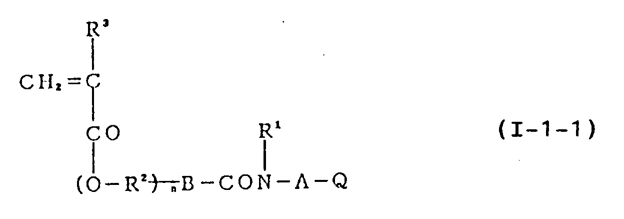
(wherein the symbols have the same significance as hereinbefore defined).

These compounds may themselves be used as antibacterial, antifungal agents, or as intermediates which, by polymerising with themselves or with one, two or more other polymerisable vinyl monomers, give the (co)polymers of this invention having antibacterial, antifungal properties. The compounds (I) of this invention, i.e. the polymers or copolymers having the formula (I), comprising a repeating unit wherein R is the group (II) or (III), and the non-polymeric compounds (I-1) represented by the formula: 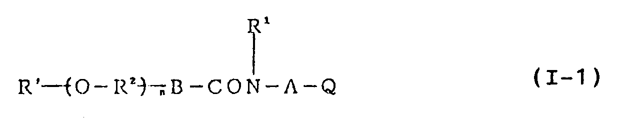
(wherein R' is a non-polymerizable acyl group which may be substituted or non-substituted and denoted by R except (II) and (III)), all have superlative antibacterial, antifungal properties.

Next, the method of manufacturing the compounds of this invention, i.e. the polymers or copolymers comprising a repeating unit containing the groups (II) or (III), and the non-polymeric compound (I-1), will be described.

Firstly, the compound (I-1) may be manufactured according to the methods described in the art (e.g. USP 4,126,747, Union Carbide Corporation) by the following reactions [1] to [4].

### [Reaction 1]

Firstly, the diamine (IV) represented by the following formula: 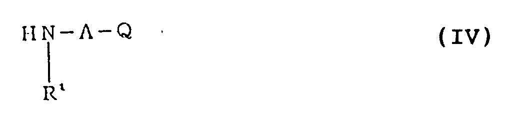
(wherein the symbols have the same significance as that previous defined), is reacted with ethylene carbonate or propylene carbonate so as to obtain the N-(amino-substituted) carbamic acid ester (V): 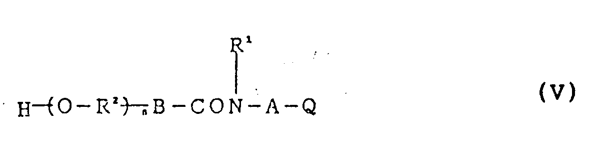
(wherein the symbols have the same significance as that previously defined. This compound is then subjected to an acylation reaction, and then, if necessary, an alkylation reaction, so as to obtain a quaternary derivative.

The reaction of the compound (IV) with ethylene carbonate or propylene carbonate to obtain the compound (V), may be performed for example according to the method described in the aforesaid USP 4,126,747, Japanese Tokkai Hei 4-288050 and J. Probst., G. Kolb, Makromol. Chem., 177, 2681 (1976), but compound (IV) is preferably reacted for example with ethylene carbonate under conditions with or without solvent. It is preferable to use 1.0 - 1.3 mole of ethylene carbonate per 1 mole of compound (IV). The solvent may be an ether such as tetrahydrofuran, an aromatic hydrocarbon such as toluene, or a halogenated hydrocarbon such as chloroform. The reaction temperature may be any temperature from room temperature to approx. 100°C, but is preferably 50 to 70°C. The reaction time is 1 to 24 hours, but is preferably 3 to 10 hours. The reaction product may be isolated and purified by column chromatography using ordinary techniques.

The acylation reaction of the compound (V) obtained as described hereintofore, may be performed by reacting the compound (V) with an organic carboxylic acid or an organic sulfonic acid represented by R-OH, or with their reactive derivatives, by known methods or methods similar to them.

The acylation (esterification) of the compound (V) may for example be performed using an organic carboxylic acid according to USP 4,126,747 and the method cited in the Patent, but is more preferably carried out using a chloride of an organic carboxylic acid (e.g. acryloyl chloride, methacryloyl chloride, acetyl chloride, isobutyl chloride, benzoyl chloride, phenylacetyl chloride or cinnamoyl chloride) in the presence or in the absence of an added base according for example to the method of J. Prost, G. Kolb (mentioned hereintofore). The reaction is preferably carried out by reacting approx. 1 to 1.5 mole of an organic carboxylic acid chloride and 1 mole of compound (V) in a solvent. Any solvent may be used provided it does not interfere with the reaction, but halogenated hydrocarbons, ketones such as acetone, or ethers such as tetrahydrofuran are to be preferred. The reaction temperature is approx. -60 to 100°C, but preferably approx. -30 to 60°C and more preferably approx. -20 to 50°C. The reaction time is approx. 0.5 to 24 hours, but preferably approx. 1 to 12 hours and more preferably approx. 3 to 8 hours. The reaction may be performed without the addition of any substances apart from those mentioned hereintofore, but in order to make the reaction proceed more smoothly and suppress the formation of by-products, bases (e.g. amines such as triethylamine, pyridine, dimethylaniline or diazabicyclooctane) may be added. The amount of these bases added is approx. 1 to 20 mole equivalents, but preferably approx. 2 to 10 mole equivalents and more preferably approx. 2 to 5 mole equivalents with respect to the amount of V. After the reaction, if necessary, the solvent and other low boiling constituents are removed as far as possible by distillation under reduced pressure or a similar method, and the desired product is obtained by usual techniques such as purification of the residue by column chromatography.

This acylation reaction may also be carried out by a method using an reactive derivative of an organic carboxylic acid other than the corresponding chloride. Examples of such reactive derivatives of organic carboxylic acids are carboxylic acid anhydrides (e.g. acetic anhydride, propionic anhydride), mixed carboxylic acid anhydrides (e.g. acetic formic anhydride), carboxylic acid esters (e.g. methyl or ethyl esters or aromatic esters such as p-nitrophenyl or 2,4-dichlorophenyl esters, heterocyclic amides of carboxylic acids (e.g. imidazolide or triazolide of carboxylic acids), and N-hydroxy heterocyclic derivatives of carboxylic acids (e.g. N-hydroxysuccinimide or N- hydroxybenzotriazol derivatives).

The reactive acylating derivatives themselves may be used after separating them when possible, but normally, the carboxylic acid and compound (V) are reacted together in the presence of dehydration condensation agents and, if necessary the starting materials of the reactive derivative such as p-nitrophenol or N-hydroxysuccinimide.

The dehydration condensation agent may be a carbodiimide or carbodiimidazole, or a combination of a chloroformic acid ester with a tertiary amine, but a carbodiimide is to be preferred.

Examples of carbodiimides are dicyclohexylcarbodiimide, diphenylcarbodiimide, di-o-tolylcarbodiimide, di-p-tolylcarbodiimide, and di-tert-butylcarbodiimide.

The amount of these dehydration condensation agents may be adjusted as appropriate. In the case of for example carbodiimides, this amount is approx. 1 to 20 mole equivalents but more preferably approx. 1 to 5 mole equivalents with respect to the carboxylic acid represented by R-OH.

It is preferable that the acylation reaction using the aforesaid carbodiimide is performed in a solvent, this solvent preferably being the same as that for the acylation reaction using the aforesaid acid chloride. The acylation reaction may be rendered more efficient by the addition of a suitable basic catalyst, e.g. 4-dimethylaminopyridine or 4-pyrrolidinopyridine. The reaction temperature is normally in the range of approx. -10 to 120°C, but more preferably approx. 0 to 80°C. The reaction time is approx. 0.5 to 48 hours, but more preferably approx. 1 to 24 hours.

After the reaction is complete, a compound having a hydroxyl group such as water or an alcohol is added to the reaction solution so as to deactivate any remaining condensation agent or reactive derivative. Insolubles are then filtered off, and after removing low boiling constituents by distillation under reduced pressure or a similar method, the residue is then for example subjected to column chromatography so as to obtain the desired product.

In order to acylate the compound (V) by an organic sulfonic acid, the compound (V) is reacted in the same way with an organic sulfonyl halide as described in the prior art. Examples of organic sulfonyl halides that can be used are alkylsulfonyl chlorides such as methylsulfonyl chloride or ethylsulfonyl chloride, aromatic sulfonyl chlorides such as p-toluenesulfonyl chloride or benzenesulfonyl chloride, benzylsulfonyl chloride or allylsulfonyl chloride. The reaction may be conducted in a solvent, suitable solvents being for example the same as those used for the acylation reaction using the aforesaid carboxylic acids and their reactive derivatives. Aromatic heterocyclic compounds (e.g. pyridine, picoline, quinoline), and tertiary amines such as triethylamine, dimethylaniline and diethylaniline, may also be used as solvents either alone or in admixture with other solvents.

Amounts of reactants, reaction temperatures and reaction times may be suitably chosen from ranges based on the acylation using the aforesaid carboxylic acids and their reactive derivatives.

Next, the acylated carbamic acid ester obtained as described hereinabove, is if necessary converted to a quartenary derivative.

In this conversion reaction, the acylated carbamic acid ester is reacted with a suitable alkylating agent corresponding to R¹⁰, R¹¹ or R¹². Examples of alkylating agents are corresponding alkyl halides (e.g. methyl iodide, ethyl iodide, butyl chloride, hexyl bromide, octyl bromide, decyl bromide, dodecyl bromide, trifluoroethyl iodide), aralkyl halides (e.g. benzyl bromide, benzyl chloride, p-chlorobensyl chloride, furfuryl chloride, thienyl chloride), allyl halides (e.g. allyl bromide, allyl iodide), propargyl halides (e.g. propargyl bromide, propargyl iodide), halogenated carboxylic acids (e.g. chloroacetic acid, bromoacetic acid, iodoacetic acid, chloroacetic acid methyl ester, chloroacetic acid ethyl ester, chloropropionic acid), trialkyloxoniumtetrafluoro-borates (e.g. trimethyloxoniumtetrafluoroborate, triethyloxoniumtetrafluoroborate), alkylsulfonic acid esters (e.g. dimethylsulfate, diethylsulfate), and epihalohydrins (e.g. epichlorohydrin, epibromohydrin). As this alkylation (quaternisation) reaction is largely affected by steric crowdedness on the terminal nitrogen atom which is alkylated, it is essential to use highly reactive alkylating agents or alkylating agents of small bulk (e.g. methyl iodide, benzyl bromide, allyl bromide). This quaternization reaction may be carried out without a solvent, but it is normally preferable to use a solvent. Any solvent may be used provided it does not interfere with the reaction, for example alcohols (e.g. methanol, ethanol, tertiary butanol). halogenated hydrocarbons (e.g. dichloromethane, chloroform, dichloroethane), nitriles (e.g. acetonitrile, propionitrile), ketones (e.g. acetone, methylethylketone), ethers (e.g. diethylether, dimethoxyethane, dioxane, tetrahydrofuran), amides (e.g. acetamide, N,N-dimethylformamide, N,N-dimethylacetamide, 1-methyl-2-pyrrolidone), sulfoxides (e.g. dimethylsulfoxide), sulfones (e.g. sulfolane), aromatic hydrocarbons (e.g. toluene, xylene), or a mixture of these solvents. The solvent chosen should be able to dissolve the starting compounds. If the starting material compound has a low molecular weight, alcohols or nitriles are suitable, while if it has a high molecular weight, halogenated hydrocarbons or dimethylformamide are suitable.

The reaction temperature is normally chosen to be in the range of approx. -30 to 120°C, but it should preferably be approx. -10 to 100°C and more preferably approx. 20 to 80°C. If low boiling point alkylating agents are reacted at a relatively high temperature, it is convenient to carry out the reaction in a vessel which can be sealed. And even when general alkylating agents are used, it is reasonable to carry out the reaction under pressure for acceleration of quaternisation. The reaction time should be suitably chosen depending on the reactivity of the starting material, acylated carbamate, and the alkylating agent, and on the reaction temperature. Normally, it is approx. 0.5 hour to 7 days, but preferably 8 hours to 5 days and more frequently approx. 24 hours to 3 days. If the alkylating agent is a bromide or chloride, it is occasionally advantageous to add an alkali metal iodide (e.g. potassium iodide, sodium iodide) as a catalyst to promote the reaction. The amount of these catalysts may be suitably chosen, but it is normally approx. 0.01 to 100 mole equivalents, and more preferably approx. 0.1 to 10 mole equivalents, with respect to the amount of alkyl bromide or chloride. Further, if iodides or iodinated compounds are used, normal precautions should be taken such as suitably covering the reaction vessel to prevent entry of light.

The alkylated (quaternized) reaction product is accompanied by an anion. This anion may be identical to the aforesaid X⁻. Moreover, the anion may be converted to any desired anion by ordinary ion exchange methods, such as allowing it to come into contact with an excess of a salt solution containing the other anion, or first changing it to a hydroxyl ion, and then allowing it to come into contact with a salt solution containing the other anion. In special cases, -COO⁻ or -SO₃⁻ in a comonomer may be used as the anion.

### [Reaction 2]

The acylated carbamate which is compound (III) may be manufactured also by reacting a carboxylic acid ester represented by the formula:

R-(O-R²-)n-BH (VI)

(wherein the symbols have the same significance as that previously defined), which has a terminal hydroxyl, mercaptol or amino group, with an isocyanic acid ester or its salt represented by the formula:

OCN-A-Q (VII)

(wherein the symbols have the same significance as that previously defined), which contains a substituted amino group, and then, if necessary, alkylating the product to convert it to a quaternized derivative. Examples of the carboxylic acid esters (VI) having a terminal hydroxol, mercaptol or amino group are 2-hydroxy-ethyl (meth)acrylate, 2-hydroxypropyl (meth)acrylate, 2-hydroxybutyl (meth)acrylate, 3-hydroxypropyl (meth)acrylate, 2-aminoethyl (meth)acrylate, 2,3-dihydroxypropyl-(meth)acrylate, 3-chloro-2-hydroxypropyl-(meth)acrylate, 2,3-dimercaptopropyl (meth)acrylate, diethyleneglycol (meth)acrylate, triethyleneglycol (meth)acrylate, polyethyleneglycol mono(meth)acrylate, or 2-(2-glycosyl)ethyl (meth)acrylate, which are commercially available as starting material compounds, and polymers or copolymers obtained from them. The polymers or copolymers may be obtained from these commercially available starting materials, and if necessary other polymerizable monomers, according to known methods or methods based on them. Other monomers which can be used will be described hereinafter. Examples of isocyanic acid esters or their salts containing a substituted amino group represented by the compound (VII) are 2-dimethylaminoethyl isocyanate, 3-dimethylaminopropyl-isocyanate, 2-(N-benzyl-N dodecyl)aminoethyl isocyanate or 1-methyl-4-diethylaminobutyl isocyanate, and salts of these compounds. The salts may be hydrochlorides or hydrobromides, but hydrochlorides are especially to be preferred. These isocyanic acid esters containing a substituted amino group or their salts may be manufactured according to known methods or methods based on them. Examples of such methods are reaction of the corresponding alkylamine containing a substituted amino group with phosgene (e.g. H. Staudinger and R. Endle, Ber. 50, 1042 (1917), C. Naegli et al, Helv. 21, 1100 (1938)), and thermal decomposition of the corresponding N-(substituted amino alkyl substituted) thiol carbamoyl aralkyl ester (e.g. Belg. 631, 961 (Chem, Abstr, 61, 6919g)), but it is more preferable to react the corresponding substituted amino alkylamine with trichloromethyl chloroformate according to the method disclosed in e.g. Organic Synth. Vol. 59, p. 195 and references quoted therein, or with bis(trichloromethyl carbonate) according to the disclosure in e.g. H. Echert and B. Forster et al (Angew. Chem. Int. Ed. Engl. 26, 894 (1987)).

The reaction of the compound (VI) with the compound (VII) may be carried out without a solvent, but it is normally preferable to use a solvent. Any solvent may be used provided it does not interfere with the isocyanate group, for example halogenated hydrocarbons (e.g. dichloromethane, chloroform), ethers (e.g. diethylether, dimethoxyethane, dioxane, tetrahydrofuran, ethylene glycol dimethylether), aromatic hydrocarbons (e.g. benzene, toluene, xylene), nitriles (e.g. acetonitrile), amides (e.g. acetamide, N,N-dimethylacetamide), N,N-dimethylformamide, N-methylpyrrolidone), or tertiary amines (e.g. triethylamine, pyridine). The amount of compound (VII) used is normally chosen to be about 1 to 1.5 mole equivalents or more preferably 1 to 1.1 mole equivalents per 1 mole of compound (VI) if compound (VI) has a low molecular weight, and about 0.001 to 1.5 mole equivalents or more preferably 0.01 to 0.5 mole equivalents per 1 mole of compound (VI) if compound (VI) is an oligomer or polymer. The reaction temperature is approx. -30 to 100°C, but more preferably approx. -20 to 50°C. The reaction time is approx. 0.5 to 48 hours, but more preferably approx. 1 to 20 hours. A tertiary amine (e.g. triethylamine, tributylamine, N-methylmorpholine, N-ethylmorpholine, N,N ,N',N'-tetramethylethylenediamine, pyridine, diazabicyclononane, 4-dimethylaminopyridine, 4-pyrrolidinopyridine, dimethylaniline and 1,2-dimethylimidasole), or a tin salt of a carboxylic acid (e.g. tin (II) acetate, tin (II) octanate, tin (II) ethylhexanate, tin (II) laurate, dibutyltin (IV) oxide, dibutyltin (IV) dihydrochloride, dibutyltin (IV) diacetate, dibutyltin (IV) dilaurate, dibutyltin (IV) maleate and dioctyltin (IV) diacetate), may be used as a catalyst to promote the reaction. The amount of catalyst used is 0.01 to 100 mole equivalents, but more preferably 0.05 to 20 mole equivalents, with respect to the amount of isocyanate.

The conversion to a quaternary ammonium derivative of the low molecular weight or high molecular weight N-(substituted aminoalkyl) carbamate compound, may be performed according to the method described hereinbefore.

### [Reaction 3]

The low molecular weight or high molecular weight N-(substituted aminoalkyl)carbamate of this invention may also be obtained by reacting a carboxylic acid ester represented by the formula:

R-(O-R²-)ₙ-OH (VIII)

(wherein the symbols have the same significance as that previously defined), which has a terminal hydroxyl group, with a chloroformic acid ester, followed by reacting the reaction product (IX) with the diamine represented by the formula (IV), and if necessary alkylating so as to convert the product to a quaternary ammonium derivative.

Examples of the carboxylic acid esters (VIII) having a terminal hydroxyl group that can be used in this method are e.g. the aforesaid compounds (VI) wherein the terminal group is hydroxyl.

Examples of chloroformic acid esters that can be used are the ethyl, trichloro and trifluoro, phenyl, nitrophenyl and pentachlorophenyl esters of chloroformic acid.

The reaction of the compound (VIII) with chloroformic acid esters may be performed according to known methods under the same conditions as those used for the aforesaid acylation of the N-(substituted aminoalkyl)carbamic acid ester (V) by a carboxylic acid halide. The reaction product (IX) may be isolated and purified by column chromatography using ordinary techniques, alternatively the reaction solution may be directly used for the following reaction.

The reaction of the compound (IX) and the diamine (IV) may be carried out in a suitable solvent added, or carried out without a solvent. Alternatively, the diamine (IV) may be added to the reaction solution in which the compound (IX) is obtained.

The reaction temperature is normally approx. -30 to 120°C, but more preferably approx. 0 to 60°C. The reaction time is approx. 0.5 to 24 hours, but more preferably approx. 1 to 8 hours. The extraction and purification of the product, and its conversion to a quaternary ammonium derivative, are performed as described hereinbefore.

### [Reaction 4]

The compound of this invention may also be manufactured by reacting an isocyanic acid ester represented by the formula (X):

R-(O-R²-)ₙ-NCO (X)

(wherein the symbols have the same significance as that previously defined), with a diamine represented by the formula (IV), and if necessary, performing an alkylation.

An example of an isocyanic acid ester that can be used is 2-isocyanate ethyl(meth)acrylate.

The reaction of the compound (X) and the diamine (IV) is carried out by the same method as that used for the reaction of, for example, the aforesaid compounds (VI) and (VII). An alkylation reaction (conversion to quaternary ammonium derivative) may also be performed according to the method as described for [Reaction 1 - 3].

The compound (I) wherein R is represented by the formula: 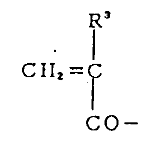
(wherein the symbols have the same significance as that hereinbefore defined), is the aforesaid compound (I-1-1). A homopolymer of the compound (I-1-1) may be manufactured by polymerising the compound (I-1-1) alone, or a copolymer of the compound (I-1-1) may be manufactured by polymerizing the compound (I-1-1) in the presence of one, two or more copolymerizable compounds. The polymer of this invention may therefore include a homopolymer and a copolymer, a substance obtained by a polymer modifying reaction, or a crosslinked polymer wherein the polymer chains of these polymers are crosslinked.

There is no particular limitation on the aforesaid copolymerizable compounds provided that they are capable of being copolymerized with the compound (I-1-1), but preferred examples of them are copolymerizable vinyl compounds having the formula (XI) given hereinafter. The copolymer of this invention may be a random copolymer, block copolymer or graft copolymer.

Homopolymers and copolymers of these compounds (I-1-1) may be manufactured by the method of the following reaction [5], but may also be obtained for example by a modification reaction of a polymer (or an oligomer), i.e. wherein a polymer (or an oligomer) is used as the compound represented by R-, R-(O-R²-)ₙ-BH or R-(O-R²-)ₙ-NCO in the aforesaid reactions [1] - [4]. In these cases, R is a polymer chain comprising a repeating unit having the formula (I) and containing a group (II) or (III), this polymer being a compound wherein the group: 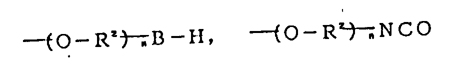
is attached as a pendant to the polymer chain. The compound (I-1-1) may be any polymerizable monomer which can be used to perform, via known methods, a modification by graft polymerisation on a polymer, polymer material or inorganic material when the compound is used alone, or together with one, two or more other polymerizable monomers. In these cases, if the side chain of the product obtained is terminated by a group Q which is tertiary amino, it will be evident that an antibacterial, antifungal polymer according to this invention may also be obtained by converting this group Q to quaternary ammonium group by means of alkylation reaction as described hereinafter.

It appears that in the case of polymers according to this invention, their antibacterial, antifungal action is manifested merely when they come into contact with the microorganisms to be eliminated. However, as it is not essential, in order to manifest this activity, that they should be homopolymers, for them to be able to achieve a practical level of antibacterial, antifungal activity, but it is merely indispensable to incorporate a small amount of antibacterial, anti-fungal structural units, consistent with economic requirements, into the surface layer (i.e. the layer susceptible to come into contact with the microorganisms) of the polymer. Form the viewpoint of obtaining bulk physical properties suited to a specific application, it is desirable that these polymers are copolymers or graft polymers. Further, from the viewpoint of convenience of application, it is desirable to use these copolymers or graft polymers in a composition containing other polymers.

### [Reaction 5]

A method of manufacturing homopolymers and copolymers of the compound (I-1-1) will now be described.

A copolymer of the compound (I-1-1) may be obtained by polymerizing the compound (I) having a group R-represented by the formula: 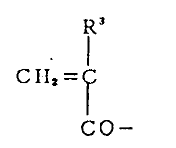
(wherein the symbols have the same significance as that hereinbefore defined), in the presence of the compound (XI) having the formula: 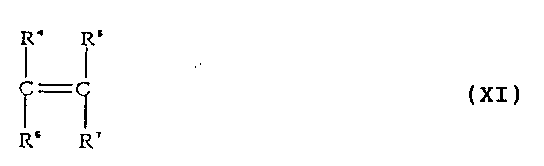
(wherein the symbols have the same significance as that hereinbefore defined), and if necessary subjecting the product to a quaternization reaction. In this case, the reaction system may contain one, two or more compounds of the type (XI). The compound (XI) may be manufactured by any of the known methods, or a method based on known methods.

The compound (I-1-1) is a (meth)acrylate type polymerizable monomer. If this is polymerized alone, any of the ordinary methods for polymerizing (meth)acrylate type monomers may be used, e.g. ion polymerization or radical polymerization. If ion polymerization is used, anionic polymerization is preferred. Further, the copolymerization can be carried out using any polymerizable monomer or macromer which is generally capable of coplymerizing with (meth)acrylate type monomers.

Examples of such polymerizable monomers which can undergo anionic polymerization with the compound (I-1-1) are aromatic vinyl monomers (e.g. styrene, α-methylstyrene), diene monomers (e.g. butadiene, isoprene), (meth)acrylic acid esters (e.g. methyl (meth)acrylate), α-substituted acrylic acid esters (e.g. α-ethyl, α-propyl, α-phenyl, α-chloro, α-bromo-or α-cyanoacrylic acid methyl esters), vinylketones (e.g. vinyl t-butyl ketone, isopropenyl t-butyl ketone), vinylcyanides (e.g. acrylonitrile, methacrylonitrile), unsaturated amides (e.g. maleimides, acrylamide, methacrylamide, N,N-dimethylacrylamide), ethylene, vinylpyridine, dimethyl methylenemalonate, or vinylidene cyanide.

The homo- or co-anionic polymerization of the compound (I-1-1) is carried out in a solvent by the addition of an initiator. The solvent may be an aromatic hydrocarbon such as toluene, a hydrocarbon such as cyclohexane, an ether such as dimethoxyethane, a halogenated hydrocarbon such as dichloroethane, a nitrile such as acetonitrile or an amide such as dimethylformamide. The initiator may be lithium, an organometallic compound such as n-butyllithium, secbutyllithium, tert-butyllithium, 9-fluorenyl sodium, sodium naphthalene, cumylpotassium, octylpotassium, 1,1-diphenylhexyllithium, or 1,1-diphenyl-3-methylheptyllithium, a complex of an organolithium compound with an organoaluminum compound such as tert-butyllithium, tri-n-butylaluminum, an alkali metal amide or alcoholate such as sodium amide, sodium alcoholate, lithium tert-butoxide, sodium polyethylene oxide or sodium alkoxide, a Cryptand complex such as sodium hydroxyborate, a Grignard reagent such as n-butylmagnesium chloride, tert-butylmagnesium bromide or phenylmagnesium bromide, sec-butyllithium sodium chloride, n-butyl, phenyl or 2-trimethylsiloxyethylmercapto-tetra-n-butylammonium, or ethyl dimethylmalonate-tetra-n-butylammonium.

The reaction may be performed in air in an atmosphere from which carbon dioxide and moisture are excluded. The reaction temperature is normally approx. -100 to 100°C, but more preferably approx. -70 to 50°C. The reaction time is approx. 0.1 to 12 hours, but more preferably approx. 2 to 8 hours. After the reaction is complete, the reaction is terminated by the addition of water, alcohol or the like, and the product may be separated, purified and extracted by the usual methods.

The radical polymerization of the compound (I-1-1) may also be carried out by general techniques for radical polymerization. Radiation may be used to generate radicals, but it is normally more convenient to use a radical initiator chemicals.

There is no particular restriction on the polymerizable monomer which can be made to undergo radical polymerization provided that it is compatible with the monomer of this invention. Examples are unsaturated aliphatic esters (e.g. alkyl esters of (meth)acrylic acid such as methyl (meth)acrylate, ethyl (meth)acrylate, butyl (meth)acrylate, hexyl (meth)acrylate, octyl (meth)acrylate, nonyl (meth)acrylate, decyl (meth)acrylate, dodecyl (meth)acrylate, cetyl (meth)acrlyate, stearyl (meth)acrylate or 2-ethylhexyl (meth)acrylate, hydroxyalkyl esters of (meth)acrylic acid such as 2-hydroxyethyl (meth)acrylate, 2-hydroxypropyl (meth)acrylate or 2-hydroxybutyl (meth)acrylate, 4-hydroxybutyl(meth)acrylate, cycloalkyl esters of (meth)acrylic acid such as cyclohexyl (meth)acrylate, methylcyclohexyl (meth)acrylate, adamantyl (meth)acrylate, tricyclo [5.2.1.0²,⁶]deca-8-yl (meth)acrylate, isobornyl (meth)acrylate or norbornyl (meth)acrylate, aromatic esters of (meth)acrylic acid such as phenyl (meth)acrylate, 1-naphthyl (meth)acrylate, fluorophenyl (meth)acrylate, chlorophenyl (meth)acrylate, bromophenyl (meth)acrylate, tribromophenyl (meth)acrylate, methoxyphenyl (meth)acrylate or biphenyl (meth)acrylate, aralkyl esters of (meth)acrylic acid such as benzyl (meth)acrylate or bromobenzyl (meth)acrylate, monomers containing an oxilane group such as glycidyl (meth)acrylate, glycidylpropyl (meth)acrylate or glycidylbutyl (meth)acrylate, or monomers containing a sugar group such as 2-(2-glycosylethyl) (meth)acrylate; unsaturated acid compounds (e.g.(meth)acrylic acid), aromatic vinyl compounds (e.g. styrene, 2-, 3-, 4-pyridine, and α-substituted styrenes such as α-methyl styrene, α-ethyl styrene, nuclear substituted styrenes such as chlorostyrene, vinyltoluene, tert-butylstyrene), cyano derivatives of vinyl compounds (e.g. acrylonitrile, methacrylonitrile), viny halides (e.g. vinyl chloride), vinylidene halides (e.g. vinylidene chloride), vinyl compounds such as vinyl chloride, vinyl acetate, N-vinylpyrrolidone, N-vinylimidazole, 2-vinylimidazole or N-methyl-N-vinylimidazole; vinylalkylethers such as vinylmethylether, vinylethylether, vinylpropylether, vinylbutylether, vinyllaurylether, vinylstearylether, fluorine-containing olefins, such as 1, 1-difluoroethylene, tetrafluoroethylene, chlorotrifluoroethylene, hexafluoropropylene, fluoroalkyl group containing (meth)acrylates, such as 2, 2, 2-trifluoroethyl (meth)acrylates, mono-(meth)acrylic acid esters of polyols such as glycerol or trimethylolpropane or N-methylol-(meth)acrylamides and their alkyl ether derivatives; unsaturated amides such as (meth)acrylamide, N,N-dimethyl (meth)acrylamide or N,N-diethylacrylamide; dialkylesters of maleic acid; N-substituted-maleimides (e.g. aliphatic maleimides such as N-methyl-, N-ethyl-, N-isopropyl-, N-butyl- or N-lauryl-maleimide, alicyclic N-substituted-maleimides such as N-cyclohexylmaleimide, or aromatic N-substituted-maleimides such as N-phenyl-, N-methylphenyl-, N-chlorophenyl- or N-methoxyphenylmaleimide; monoalkyl esters of α, β-unsaturated carboxylic acids such as (meth)acrylic acid, or of α,β-unsaturated dicarboxylic acids such as fumaric acid, maleic acid, maleic acid anhydride or itaconic acid.

In addition to the above compounds, other polymerizable momomers which can be used as comonomers are polyfunctional crosslinkable monomers with two or more olefinic double bonds. Examples of such monomers are dimethacrylates or diacrylates such as ethylene glycol dimethacrylate, diethylene- glycol di(meth)acrylate, triethyleneglycol di(meth)acrylate, tetraethyleneglycol di(meth)acrylate, tripropyleneglycol di(meth)acrylate, 1,3-butylenediglycol (meth)acrylate, 1,4-butanediol di(meth)acrylate, 1,5-pentanediol di-(meth)acrylate, 1,6-hexanediol di(meth)acrylate, neopentylglycol di(meth)acrylate, hydroxypivalic acid neopentyl ester (meth)acrylate; oligoester (meth)acrylates, such as poly-butadiene (meth)acrylate, 2,2-bis (4-(meth)acryloyloxy-phenyl)propane, 2,2-bis(4-( (meth)acryloyloxypolyethoxy)phenyl)propane, 2,2-bis(4-( -(meth)acryloyloxypolyethoxy)-dibromophenyl)propane, 2,2-bis(4- -(meth)acryloyloxypropoxy)dibromophenyl)propane, or bis(4-( (meth)acryloyloxypolyethoxy)phenylmethane; bifunctional crosslinkable monomers such as diallylterephthalate, divinylbenzene, N,N'-m-phenylene bismaleimide; trifunctional crosslinked monomers such as trimethyloylethane tri(meth)acrylate, trimethyloylpropane tri(meth)acrylate, pentaerythritol (meth)acrylate, tri(meth)allylisocyanurate, triallyl trimellitate, diallylchlorendate; and tetrafunctional crosslinked monomers such as pentaerythritol tetra(meth)acrylate.

The homopolymerization or copolymerisation of the aforesaid compound (I-1-1) may be carried out using the compound (I-1-1) and one, two or more types of the copolymeriziable monomers given above.

The radical homo- or copolymerization of the compound (I-1-1) may be performed according to known methods by the action of heat, microwaves, infrared light, ultraviolet light or high-energy radiation, or by using a radical initiator as a catalyst which generates free radicals by means of an oxidation-reduction reaction. In general, the polymerization may be carried out in either manner of reactions known as bulk polymerization, solution polymerization, emulsion polymerization or suspension polymerization. The initiator may for example be an azo compound such as azobisisobutyronitrile (AIBN), azobis (sec-valeronitrile), azobiscyclohexanecarbonitrile or azobis (4-methoxy-2,4-dimethylvaleronitrile); a peroxide such as cumene hydroperoxide, tert-butylhydroperoxide, dicumyl peroxide, di (tert-butyl) peroxide, benzoyl peroxide, lauroylperoxide, potassium peroxide or ammonium peroxide, or a redox initiator such as hydrogen peroxide - ferrous sulfate, persulfates - sodium bisulfite, cumene hydroperoxide - ferrous salt, or benzoyl peroxide - dimethylaniline. These compounds are used in a proportion of approx. 0.001 to 10 weight %, but more preferably 0.05 to 5 weight %, with respect to the total weight of monomer. The polymerization temperature is normally approx. -10 to 200°C, but more preferably approx. 50 to 120°C. The reaction time is of the order of approx. 0.1 hours to 2 weeks, but more preferably approx. 1 hour to 6 days.

If a solvent is used, there is no particular restriction on the solvent provided that it can dissolve the compound (I-1-1) or the copolymerizing monomer. Examples are aromatic hydrocarbons (e.g. benzene, toluene, xylene), nitriles (e.g. acetonitrile), ketones (e.g. acetone, methylethylketone, methylisobutylketone), ethers (e.g. tetrahydrofuran, dioxane, dimethoxyethane), esters (e.g. ethyl acetate, butyl acetate, cellosolve acetate, cellosolve lactate), halogenated hydrocarbons (e.g. 1.2-dichloroethane, dichloromethane, chloroform, dichloroethane), amides (e.g. dimethylformamide, dimethylacetamide), sulfoxides (e.g. dimethylsulfoxide), triamide phosphates (e.g. hexamethylphosphorotriamide), alcohols (e.g. 1- and 2-butanol, 1- and 2-propanol, tert-butanol), water, or mixtures of these solvents.

It is preferable, and particularly preferable if the initiator concentration is low, that the reaction be conducted in vacuo, or in an apparatus where the air has been replaced by an inert gas (e.g. nitrogen, argon), so as to completely exclude oxygen.

If necessary, polymerization regulators (e.g. mercaptans, thioglycols, carbon tetrabromide or α-methylstyrene dimer), suspension agents (e.g. polyvinyl alcohol, methyl cellulose, polyacrylamide, calcium phosphate, magnesium pyrophosphate), or dispersion agents (e.g. anionic surfactants such as sodium dodecyl benzenesulfonate) may also be used.

After the polymerization reaction, the reaction solution is treated with a large volume of a solvent in which the product polymer is only poorly dissolved (a suitable substance being chosen according to the type of product, e.g. methanol, acetone, ether, hexane or petroleum ether) in order to precipitate the product polymer. The precipitate is then extracted, and purified to give the target compound.

As described hereintofore, the scope of the invention also includes antibacterial, antifungal polymers obtained by graft copolymerization, on a homopolymer, copolymer, preformed polymer material or inorganic material (e.g. montmorillonite) of monomers having the structural units of the compound (I), either alone or in admixture with one, two or more polymerizable monomers.

Examples of materials which can be used for this graft co-polymerization are synthetic and naturally occurring polymer materials, wood, glass and clay minerals. There is no particular limitation on synthetic polymer materials, examples being polyolefins (e.g. high density polyolefins, low density polyolefins, polypropylene), ethylene-propylene copolymer, ethylene-vinyl acetate copolymer, polyvinyl chloride, polyvinyl alcohol, polystyrene, polyacrylo-nitrile, polymethylmethacrylate, styrene-butadiene (block) copolymer, acrylonitrile-butadiene-styrene (block) copolymer, polybutadiene, polyisoprene, polytetrafluoroethylene, polyethyleneterephthalate, polybutyleneterephthalate, polyethylester (block) copolymer, polycarbonates, polyurethanes (e.g. polyurethane, polyetherurethane, polyurethaneurea), polyamides (e.g. Nylon 6, Nylon 66) and polysulfones (e.g. polysulfone, polyethersulfone), silicones, and cellulose acetate. Of these materials, polyethylene, polyvinyl chloride, polyurethanes, polyamides and polysulfones are particularly preferred. Examples of naturally occurring polymer materials are cellulose, paper, cotton, wool, down, leather, silk, hemp and rubber. Further, to convert inorganic materials such as glass to complex materials by graft copolymerization, known methods may be used such as treatment with a silane coupling agent having reactive groups such as vinyl, epoxy, methacryloyl or alkyl substituted by amino, or cathration by metal ions which give a polymerization intiation point.

In this graft copolymerization, a polymerization initiation point is created on the surface of the polymer substrate. The monomer is then polymerized and the graft chain grows from the point. To create the polymerization initiation point, the following methods may be employed:
(1) plasma irradiation,
(2) irradiation by Gamma or other rays,
(3) irradiation by light (UV, visible (with concurrent use of optical sensitizers),
(4) addition of peroxides such as ozone,
(5) redox methods using peroxides, Fe²⁺ ions,
(6) methods using oxides of metals such as Ce⁴⁺, Cr⁶⁺ or Mn⁷⁺.

Of these methods, it is convenient to employ redox systems, ozone, and Ce ion.

An alternative method of graft copolymerization is to form reactive functional groups on the surface of the polymer by means of acids, alkalis or other reagents as described in the prior art. The antibacterial, antifungal polymer of this invention may then be obtained by graft copolymerizating or coupling a monomer or coupling agent having reactive functional groups on a polymer material, followed by graft copolymerizing the monomer of this invention on the product.

Next, the polymer obtained is if necessary converted to a quaternary ammonium derivative.

This conversion to a quaternary ammonium derivative may be performed according to the method used for the conversion of the aforesaid acylated carbamic acid ester to a quaternary derivative. The solvent chosen should preferably be able to dissolve or fully expand the starting polymer. Examples of such solvents are usually nitriles (e.g. acetonitrile, propionitrile), ketones (e.g. acetone, methylethylketone), ethers (e.g. diethyl ether, dioxane, tetrahydrofuran), amides (acetamide, N,N-dimethylformamide, N,N-diethyl-acetamide, 1-methyl-2-pyrrolidone), sulfoxides (e.g. dimethylsulfoxide) or sulfones (sulfolane). N,N- dimethylformamide, N,N-dimethylacetamide, 1-methyl-2- pyrrolidone, dimethylsulfoxide and sulfolane are particularly to be preferred.

Another method of obtaining a quaternary ammonium derivative of the polymer (compounds I, II wherein Q = quaternary ammonium), is to use a quaternary ammonium monomer as starting material for the polymerization reaction. The quaternary ammonium monomer used in this case may be obtained by alkylation, according to the aforesaid method, of the compound (III) wherein Q = tertiary amino.

The number average molecular weight (Mn) of the antibacterial, antifungal (co)polymer of this invention (based on polystyrene standards) is approx. 2,000 to 2,000,000, but more preferably 5,000 to 200,000, and the ratio Mw/Mn (Mw: Weight average molecular weight) is preferably approx. 1.1 to 3.0.

Of the polymers according to this invention comprising a repeating unit having the formula (I) wherein R is represented by (III), X-ray photoelectron spectroscopy (ESCA) was performed on the copolymer (formula XIII) of the monomer of the formula (XII): 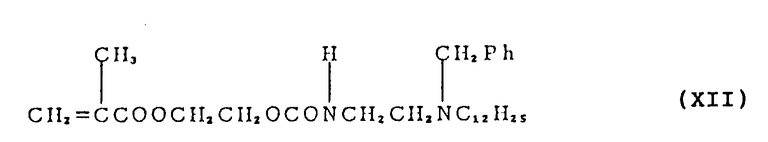
with butyl methacrylate, and on the corresponding poly-(methyl iodide) compound. From the results, it was found that the value of x/y in the compound represented by the formula: 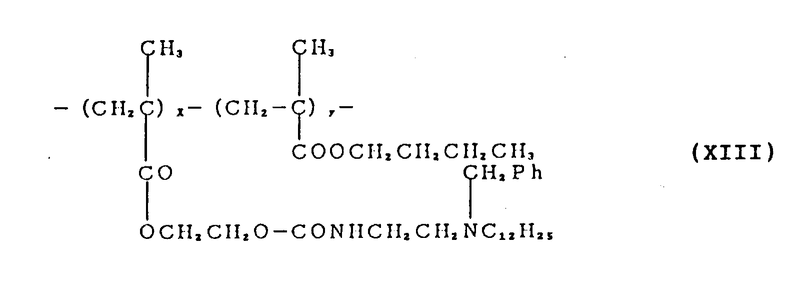
was substantially identical to the mole ratio of the compound (XII) and butyl methacrylate used. Further, there was little scattering in the value of x/y between different parts of the measurement sample, showing the compound (XIII) to be a random polymer or a block polymer.

The compound (I) comprising a polymer or copolymer according to this invention obtained as hereinbefore described has superlative antibacterial, antifungal properties. It can therefore be used to form various antibacterial, antifungal materials, and to carry out antibacterial, antifungal treatment on a variety of materials according to methods known in the art.

For example, of the (co)polymers of this invention, compounds wherein Q = a group containing a tertiary amino group are all soluble in chloroform, dichloromethane, dimethylformamide or dimethylsulfoxide, but insoluble in water, methanol or ethanol. Further, of these copolymers, compounds wherein Q = quaternary ammonium are generally soluble in chloroform, dimethylformamide or dimethylsulfoxide, methanol or ethanol, but insoluble in water. An antibacterial, antifungal material may therefore be obtained by coating, spraying, impregnating or affixing these (co)polymers, in the form of a solution in an organic solvent, onto another substrate (e.g. a plastic, natural or synthetic fiber (such as for example the synthetic or natural polymers used for graft copolymerization hereintofore described, the synthetic or natural polymers used for polymer blends hereinafter described, and products made from such polymers), rubber, leather, wood, paper or glass). Further the (co)polymer of this invention, either alone or in admixture with other compatible or dispersible (co)polymers as described in detail hereinafter, may be used to form common molded products having antibacterial, antifungal properties, or cast onto a glass, polystyrene or polyimide film so as to obtain a film having antibacterial, antifungal properties. Alternatively, it may be extrusion molded, or a fibrous material obtained by solidification of a solution in a solvent poorly dissolve it, so as to obtain filaments having antibacterial, antifungal properties.

In particular, when the compound of this invention is a copolymer obtained by copolymerization of a monomer having antibacterial, antifungal properties with one, two or more other monomers, the following methods may be employed to enhance compatibility with objects on which it is desired to confer antibacterial, antifungal properties and the stability of the resulting composition so as to enlarge its range of application while maintaining a practical level of antibacterial, antifungal properties: (1) changing the compositional ratio of the monomer with antibacterial, antifungal properties in the copolymer,
(2) blending two or more copolymers, or
(3) blending the resulting copolymer with one, two or more other (co)polymers to form a new composition.

In the manufacture of this copolymer or polymer blend, manufacturing costs can be reduced by using more economical comonomers or (co)polymers. This polymer blend technology may be applied in a similar way also to homopolymers of monomers having antibacterial, antifungal properties.

There is no restriction on the (co)polymer used in the manufacture of the aforesaid polymer blend provided it is compatible with the (co)polymer having antibacterial, antifungal properties according to this invention, and provided it can be blended by adding a blending agent such as a solvent, compatibilizing agent, plasticizer or dispersion agent. Typical examples are nitrocellulose, vinyl chloride-vinyl acetate copolymer, polyester resins obtained from polyvinylacetal or polyethylene glycol and phthalic acids or adipic acid, acrylic resins such as poly(meth)acrylic acid, polymethyl(meth)acrylate or polybutyl(meth)acrylate, polytetrafluoroethylene resin, unsaturated polyester resins obtained by copolymerization of fumaric acid or maleic acid with styrene or the like, urethane resins obtained from diols or polyols such as polyethylene glycol, polypropylene glycol, polyoxypropylene triol or butanediol and di- or triisocyanates such as diphenylmethane-4,4'-diisocyanate, hexamethylenediisocyanate, tolylenediisocyanate or cyclohexane-1,3,5-triisocyanate, epoxy resins, aminoalkyd resins, poly[2-hydroxyethyl (meth)acrylate], polyacrylamide, poly[N,N-dimethylamino ethyl (meth)acrlyate], polyethylene glycol, polypropylene glycol, polyvinylpyrrolidone, polyvinylpyridine, polyamides such as Nylon, polyvinyl alcohol, polyvinyl acetoamide, polyvinylamine, poly(2-glucosylethyl methacrylate), or silicone resins.

According to this invention, various additives may also be blended with the aforesaid substances to the extent that they do not interfere with their intended purpose. These additives may for example be curing agents, adhesives, antioxidants, surfactants, weatherproofing agents, carbon black, pigments, dyes, UV absorption agents, inorganic fillers or flame retardants.

Of low molecular weight compounds of this invention, those having Q = tertiary amino are all difficultly soluble in water, while those having Q = quaternary ammonium are all difficultly soluble in water and diethyl ether. However, both types are soluble in alcohols (e.g. methanol, ethanol, butanol), ketones such as acetone or methylethylketone, ethers such as tetrahydrofuran, dioxane or dimethoxyethane, nitriles such as acetonitrile, esters such as methyl acetate, ethyl acetate, butyl acetate, halogenated hydrocarbons such as chloroform or dichloromethane, amides such as dimethylformamide and dimethylsulfoxide. As in the case of the polymers, solutions in these solvents may be added or applied to, or impregnated on, other substrates so as to obtain antibacterial, antifungal materials. When compounds of this invention has low molecular weight, those compounds are blended to the aforesaid polymer or copolymer and used to obtain common molded products, film, fiber or paints having antibacterial and antifungal properties.

In order to obtain a film having adequate antibacterial and antifungal properties using a polymer or copolymer containing a structural unit having the group (I), in case R represents (III), of this invention, the proportion (as a mole fraction x) should be at least approx. 0.01 mole %. Further, in manufacturing the antibacterial, antifungal material of this invention, the polymer or copolymer containing a structural unit having the group (I) or (II), and compound (III), may be used in conjunction with other additives. Examples of additives that can be used are pigments such as titanium oxide, zinc white, red ion oxide, minium or carbon black, dyes such as Cyanin Green, fillers such as bentonite or talc, drying agents such as cobalt naphthenate or manganese naphthenate, and solvents. Such solvents may be hydrocarbons such as benzene, toluene, xylene, cyclohexane, hexane or heptane, alcohols such as methanol and ethanol, ketones such as methylethylketone or methylisobutylketone, esters such as ethyl acetate or butyl acetate, halogenated hydrocarbons such as trichloroethane or trichloroethylene, petroleum hydrocarbons such as lignoin, petroleum benzine or naphtha, water and dimethylsulfoxide.

The coating amount of a film of the compound (I) on material substrates to which it is applied, is approx. 100 to 0.005 g, but more preferably approx. 100 to 0.5 g per 1m² of the substrate. This amount is however normally approx. 10 to 0.5 g, an amount of approx. 10 to 1 g being particularly preferred. The antibacterial, antifungal material thus obtained may for example be used as a material for the inner lining of tanks (i.e. in papermaking factories), pipes or as an agent to confer antibacterial, antifungal and antifouling properties on same, for the interiors (e.g. walls, ceilings and passages) of hospitals, hotels, schools, food processing plants or communal residences, for automobiles or interior parts of trains, airplanes or ships, for fishing nets and ship hulls, as an antibacterial, antifungal and antifouling coating for cooling towers, for antibacterial mats in swimming pools and entrance halls, curtains, PVC wallpaper, urethane foam, artificial leather (e.g. for furniture, interiors, shoe, shoe insoles), bathroom units, PVC film for agricultural use, polypropylene or polyethylene terephthalate fiber, plastic storage boxes, as an antibacterial, antifungal and antifouling material for plastic in sportsware or for conferring similar properties on same, as a food packaging, as an antibacterial, antifungal treatment for fiber products such as clothing or hospital sheets, luster coating agent, paper, tissues (hygienic paper), to confer antibacterial, antifungal and antisoiling properties on wooden parts (e.g. prevention of blackening), disposable face towels, cleaning cloths or as a disinfectant in deodorizing, antibacterial sprays.

This invention (i.e. the antibacterial, antifungal polymer of this invention) therefore provides a material which has superlative antibacterial, antifungal properties, high stability and long-term activity in a wide range of applications.

If necessary, the antibacterial, antifungal polymer of this invention may also be blended with other antibacterial, antifungal agents known in the art to the extent that this does not impair the stability and long-term activity which is characteristic of this invention. Examples of such known antibacterial, antifungal agents are silver, copper-based inorganic antibacterial agents, 1-substituted imidazole or triazole compounds (e.g. clotrimazole, miconazole), benzoimidazole compounds (e.g. 2 (4-thiazolyl) benzoimidazole), thiocarbamate or dithiocarbamate compounds (e.g. tolnaftate, ethylenethiuram disulfide), phthalimide compounds (e.g. N-fluoro-dichloromethylthiophthalimide, phenols (e.g. o-phenylphenol), phenoxalsin compounds (e.g. 10,10'-phenoxalsin-oxybis), or quaternary ammonium salts.

### Method of Manufacturing Intermediate 1: Manufacture of a 2-(N-benzyl-N-alkylamino)ethanol

N-Benzylethanolamine (15.1 g), hexyl bromide (16.5 g) and anhydrous potassium carbonate (6.9 g) were added to 250 ml of ethanol, and the mixture was heated at 80°C for 4 to 20 hours with stirring. The ethanol was distilled off, the product extracted with dichloromethane, and the extract washed with water. After drying, the solvent was distilled off. The residue was purified by distillation under reduced pressure to give 2-(N-benzyl-N-hexylamino)ethanol. The yield was 18.0 g (76.5%).

N-Benzylethanolamine (15.1 g) and dodecyl bromide (24.9 g) were reacted in the same way, and the product was purified by silica gel chromatography (solvent: ethyl acetate) to give 2-(N-benzyl-N-dodecylamino)ethanol, yield 22.0 g (69.1 g).

N-Benzylethanolamine (15.1 g) and hexadecyl bromide (30.5 g) were reacted in the same way, and the product was purified by silica gel chromatography (solvent: ethyl acetate) to give 2-(N-benzyl-N-hexadecylamino) ethanol. The yield was 18.5 g (49.3 g).

### Method of Manufacturing Intermediate 2: Manufacture of a N-benzyl-N-higher-alkyl ethylenediamine

I. Each 2-(N-Benzyl-N-(higher-alkyl)amino)ethanol, i.e. 1.12 g of 2-(N-benzyl-N-hexyl)ethanol, 1.00 g of 2-(N-benzyl-N-dodecyl)ethanol, or 1.02 g of 2-(N-benzyl-N-hexadecyl)ethanol, was separately dissolved together with each 1.23 g of triphenylphosphine and 0.69 g of phthalimide in 20 ml of dry tetrahydrofuran. The solution was cooled in ice water, and a solution of 0.82 g diethyl azadicarboxylate in 5 ml of dry tetrahydrofuran was added dropwise. After stirring at room temperature for 4 to 5 hours, the tetrahydrofuran was distilled off under reduced pressure, the residue was extracted with dichloromethane, and the extract was washed with water. After drying, the product was separated and purified by distillation under reduced pressure, or by silica gel chromatography (solvent:dichloromethane:
   diethyl ether = 30:1), so as to obtain N-benzyl-N-higher-alkyl-N-(2-phthalimidoethyl)amine The yield of N-benzyl-N-hexyl derivative was 1.31 g.
   IR spectrum (neat) cm⁻¹: 1773, 1715 (no absorption due to ν_{OH}).
   ¹H-NMR spectrum (in deutero-chloroform): δppm:
   0.81(3H,t),
   1.16(6H,s), 1.39(2H,m), 2.46(2H,t), 2.71(2H,t), 3.59(2H,s), 3.76(2H,t), 7.12(5H,m), 7.70(2H,m), 7.80(2H,m).
   N-Benzyl-N-dodecyl derivative, 1.10 g.
   IR spectrum (neat) cm⁻¹: 2928, 2858, 1775, 1715, 1396, 1087, 719, 698, 530.
   ¹H-NMR spectrum (in deutero chloroform): δppm:
   0.88(3H,t), 1.16(6H,s), 1.25(12H,s), 1.40(2H,m), 2.46(2H,t), 2.71(2H,t), 3.59(2H,s), 3.76(2H,t), 7.14(5H,m), 7.72(2H,m), 7.80(2H,m).
   N-Benzyl-N-hexadecyl derivative, 0.90 g.
   IR spectrum (neat) cm⁻¹: 2926, 2856, 1775, 1717, 1396, 1089, 719, 698, 530.
   ¹H-NMR spectrum (in deutero chloroform): δppm:
   0.88(3H,t), 1.16(6H,s), 1.26(20H,s), 1.40(2H,m), 2.46(2H,t), 2.71(2H,t), 3.59(2H,s), 3.76(2H,t), 7.14(5H,m), 7.72(2H,m), 7.80(2H,m).
II. N-Benzyl-N-hexadecyl-N-(2-phthalimidoethyl)amine, 2.70 g (5.36 mmol) was dissolved in 60 ml ethanol with heating, 0.54 g (10.72 mmol) of hydrazine hydrate was added, and the mixture stirred at 70°C for 4 hours. The mixture was concentrated under reduced pressure, the residue diluted with water, and the pH adjusted to 1 to 2 with hydrochloric acid. The solution was then extracted with ether, the extract dried with anhydrous magnesium sulfate, and the solvent distilled off. The residue was separated and purified by silica gel chromatography (solvent:dichloromethane:
   methanol = 10:1) to give 1.39 g of N- benzyl-N-hexadecylethylenediamine. The yield was 69.3%.
   ¹H-NMR spectrum (in deutero-chloroform): δppm:
   0.88(3H,t), 1.25(26H,s), 1.36(2H,s), 1.47(2H,m), 2.42(2H,t), 2.47(2H,t), 2.71(2H,t), 3.56(2H,s), 7.30(5H,m).
   N-Benzyl-N-hexylethylenediamine was obtained from N-benzyl-N-hexyl-N-(2-phthalimidoethyl)amine in the same way (yield 60.5%).
   ¹H-NMR spectrum (in deutero-chloroform): δppm:
   0.87(3H,t), 1.28(6H,s), 1.34(2H,s), 1.47(2H,m), 2.42(2H,t), 2.47(2H,t), 2.71(2H,t), 3.56(2H,s), 7.30(5H,m).
   N-Benzyl-N-dodecylethylenediamine was obtained from N-benzyl-N-dodecyl-N-(2-phthalimidoethyl)amine in the same way (yield 39.6%).
   ¹H-NMR spectrum (in deutero-chloroform): δppm:
   0.88(3H,t), 1.24(18H,s), 1.47(2H,m), 2.43(2H,t), 2.50(2H,t), 2.53(2H,s), 2.73(2H,t), 3.56(2H,s), 7.30(5H,m).

### Method of Manufacturing Intermediate 3: Manufacture of 2-hydroxyethyl N-(2-(benzylhexylamino)ethyl)carbamate

A mixture of 0.51 g of N-benzyl-N-hexylethylenediamine and an equimolar amount of ethylene carbonate (0.20 g) was stirred at 60°C for 4 to 8 hours. The product was purified by silica gel chromatography (solvent: dichloromethane: methanol = 10:1) to give 2-hydroxyethyl N-(2-benzylhexyl amino)ethylcarbamate. The yield was 81.7%.
¹H-NMR spectrum (in deutero-chloroform): δppm:
0.88(3H,t), 1.26(6H,s), 1.48(2H,m), 2.44(2H,t), 2.55(2H,t), 3.21 (2H,q), 3.57(2H,s), 3.80(2H,t), 4.18(2H,t),
5.23(1H,br), 7.30(5H,m).

### Method of Manufacturing Intermediate 4: Manufacture of 2-Hydroxyethyl N-(2-(benzyldodecylamino)ethyl)-carbamate

2-Hydroxyethyl N-(2-benzyldodecylamino)ethyl-carbamate was obtained from 0.31 g of N-benzyl-N-dodecylethylenediamine and an equimolar amount (0.09 g) of ethylene carbonate in the same way as in Method of Manufacturing Intermediate 3. The yield was 83.5%.
¹H-NMR spectrum (in deutero-chloroform): δppm:
0.88(3H,t), 1.25(18H,s), 1.47(2H,m), 2.43(2H,t), 2.54(2H,t), 3.21 (2H,q), 3.56(2H,s), 3.77(2H,t), 4.18(2H,t), 5.23(1H,br), 7.29(5H,m).

### Method of Manufacturing Intermediate 5: Manufacture of 2-Hydroxyethyl N-(2-(benzylhexadecylamino)ethyl)-carbamate

2-hydroxyethyl N-(2-benzylhexadecylamino)ethyl-carbamate was obtained from 1.39 g of N-benzyl-N-hexadecylethylenediamine and an equimolar amount (0.33 g) of ethylene carbonate in the same way as in Method of Manufacturing Intermediate 3. The yield was 84.9%.

### Method of Manufacturing Intermediate 6: Manufacture of 2-(N-benzyl-N-(higher alkyl)amino)ethanol

### (1) 2-(N-Benzyl-N-decylamino)ethanol

N-Benzylethanolamine (7.55 g) and decyl bromide (11.05 g) were reacted in the same way as in Method of Manufacturing Intermediate 1, and the product purified by silica gel chromatography (solvent:ethyl acetate:hexane=1:1) to give 10.42 g (71.50%) of the target compound.
¹H-NMR spectrum (in deutero-chloroform): δppm:
0.88(3H,t), 1.24(14H,s), 1.48(2H,m), 2.46(2H,t), 2.61(2H,t), 3.55 (2H,t), 3.61(2H,s), 7.30(5H,m).

### (2) 2-(N-benzyl-N-tetradecylamino)ethanol

N-Benzylethanolamine (7.55 g) and tetradecyl bromide (13.86 g) were reacted in the same way as in Method of Manufacturing Intermediate 1, and the product purified by silica gel chromatography (solvent:ethyl acetate:hexane (1:1)) to give 12.15 g (69.91%) of the target compound.
¹H-NMR spectrum (in deutero-chloroform): δppm:
0.88(3H,t), 1.24(22H,s), 1.48(2H,m), 2.46(2H,t), 2.61(2H,t), 3.55 (2H,t), 3.61(2H,s), 7.30(5H,m).

### Method of Manufacturing Intermediate 7: N-Benzyl-N-decyl-ethylenediamine

I. N-Benzyl-N-decyl-N-(2-ethylphthalimido)amine was obtained from 6.00 g of 2-(N-benzyl-N-decylamino)ethanol in the same way as in Method of Manufacturing Intermediate 2(I). The yield was 5.84 g (67.40%).
   ¹H-NMR spectrum (in deutero-chloroform): δppm:
   0.88(3H,t), 1.24(14H,m), 1.40(2H,br), 2.46(2H,t), 2.72(2H,t), 3.60 (2H,s), 3.77(2H,t), 7.14(5H,m), 7.72(2H,m), 7.80(2H,m).
II. The 5.84 g of product obtained in the aforesaid Method of Manufacturing Intermediate 7(I) was treated in the same way as in Method of Manufacturing Intermediate 2(II), with the exception that the reaction solution was stirred at room temperature for 20 hours instead of being heated to 70°C, and then, worked up as in Method of Manufacturing Intermediate 2(II) to obtain the target compound. The yield was 2.81 g (69.74%).

### Method of Manufacturing Intermediate 8: N-Benzyl-N-tetradecylethylenediamine

I. N-Benzyl-N-tetradecyl-N-(2-ethylphthalimido)amine was obtained from 6.00 g of 2-(N-benzyl-N-decylamine)-ethanol in the same way as in Method of Manufacturing Intermediate 2(I). The yield was 6.02 g (73.00%).
   ¹H-NMR spectrum (in deutero-chloroform): δppm:
   0.88(3H,t), 1.24(22H,m), 1.40(2H,br), 2.46(2H,t), 2.71(2H,t), 3.60 (2H,s), 3.77(2H,t), 7.15(5H,m), 7.72(2H,m), 7.80(2H,m).
II. The 6.02 g of product obtained in the aforesaid Method of Manufacturing Intermediate 8(I) was treated in the same way as in Method of Manufacturing Intermediate 2(II), with the exception that the reaction solution was stirred at room temperature for 20 hours instead of being heated to 70°C, and then, worked up as in Method of Manufacturing Intermediate 2(II) to obtain the target compound. The yield was 3.17 g (72.53%).

### Method of Manufacturing Intermediate 9: 2-Hydroxyethyl N-(2-benzyldecylaminoethyl)carbamate

N-Benzyl-N-decylaminoethylenediamine (2.00 g) and an equimolar amount (0.61 g) of ethylene carbonate were reacted in the same way as in Method of Manufacturing Intermediate 3 to give the target compound. The yield was 2.24 g (90.42%).

### Method of Manufacturing Intermediate 10: 2-Hydroxyethyl N-(2-benzyltetradecylaminoethyl) carbamate

N-Benzyl-N-tetradecylaminoethylenediamine (2.00 g) and an equimolar amount (0.51 g) of ethylene carbonate were reacted in the same way as in Method of Manufacturing Intermediate 3 to give the target compound. The yield was 2.24 g (89.24%).

### Method of Manufacturing Intermediate 11: 2-Hydroxyethyl N-(2-(2-pyridyl)ethyl carbamate

2-(2-Pyridyl)ethylamine (10.00 g) and an equimolar amount (7.21 g) of ethylene carbonate were reacted in the same way as in Method of Manufacturing intermediate 3 to give the target compound. The yield was 14.47 g (84.08%).

### Method of Manufacturing Intermediate 12: 2-Hydroxyethyl N-(2-,3- and 4-pyridylmethyl) carbamate

2-, 3- and 4-Aminomethylpyridine (each 10.00 g) were separately reacted with an equimolar amount (8.14 g) of ethylene carbonate in the same way as in Method of Manufacturing Intermediate 3 at 60°C for 7 hours. The reaction products were purified to give respectively 14.25 g (78.56%) of 2-hydroxyethyl N-(2-pyridylmethyl) carbamate (purified by recrystallization from acetone to give colorless needle-like crystals.

¹H-NMR spectrum (in deutero-chloroform): δppm:
3.15(1H,br), 3.81(2H,t), 4.23(2H,t), 4.47(2H,d), 6.20(1H,br), 7.17-7.28(2H,m), 7.67(1H,td), 8.51(1H,d)); 16.69 g (92.00%) of 2-hydroxyethyl N-(3-pyridylmethyl) carbamate (purified by silica gel chromatography (solvent: ethyl acetate:methanol = 6:1) to give an oily substance. ¹H-NMR spectrum (in deutero-chloroform): δppm: 3.76(2H,t), 4.06(1H,s), 4.17(2H,t), 4.31(2H,d), 6.38(1H,br), 7.20-7.26(1H,m), 7.62(1H,m), 8.44(2H,m); and 14.10 g (77.74%) of 2-hydroxyethyl N-(4-pyridylmethyl) carbamate (purified by recrystallization from acetone to give colorless needle-like crystals. ¹H-NMR spectrum (in deutero-chloroform): δppm: 3.06(1H,s), 3.82(2H,t), 4.24(2H,t), 4.35(2H,d), 5.79(1H,br), 7.19(2H,d), 8.50(2H,dd).

### Method of Manufacturing Intermediate 13 - 15: Manufacture of N-(2-, 3- and 4-methoxybenzyl) ethanolamines

A) Ethanolamine (4.49 g (73.42 mmol)) was added to a solution of 10.00 g (73.42 mmol) of 2-methoxybenzaldehyde in 50 ml of ethanol, and the mixture heated under reflux for 8 hours. The solvent was distilled off, and the residue distilled under reduced pressure to give 10.34 g of 2-methoxybenzilidine ethanolamine as an oil, b.p. 143°C/3.9 mm Hg, yield 78.6%,
   ¹H-NMR spectrum (in deutero-chloroform), δppm:
   2.82(1H,s), 3.74(2H,t), 3.85(3H,s), 3.89(2H,m), 6.94(2H,q), 7.38(1H,t), 7.91(1H,d), 8.75(1H,s).
   3-Methoxybenzylidene ethanolamine was prepared in the same way from 3-methoxybenzaldehyde and ethanolamine: an oil, b.p. 148°C/3.5 mm Hg, yield 71.6%,
   ¹H-NMR spectrum (in deutero-chloroform), δppm:
   3.03(1H,s), 3.74(2H,t), 3.83(3H,s), 3.91(2H,m), 6.96(1H,m), 7.30(3H,m), 8.75(1H,s).
   4-Methoxybenzylidene ethanolamine was also prepared from 4-methoxybenzaldehyde and ethanolamine: an oil, b.p. 155°C/4.6 mm Hg, yield 73.4%,
   ¹H-NMR spectrum (in deutero-chloroform), δppm:
   3.05(1H,s), 3.69(2H,t), 3.83(3H,s), 3.89(2H,m), 6.90(2H,m), 7.63(2H,d), 8.19(1H,s).
B) 2-Methoxybenzilidine ethanolamine (10.34 g (57.67 mmol)) was dissolved in 200 ml of methanol, and the mixture cooled to 5 - 10°C. Sodium boron hydroxide (3.27 g (86.51 mmol)) was added in small quantities at a time, and the mixture stirred at 10°C for 3 hours, then water (300 ml) added, the methanol distilled off, and the mixture acidified with hydrochloric acid. After washing with ether, the mixture was rendered alkaline with an aqueous solution of sodium hydroxide, washed with a small quantity of ether, and extracted with dichloromethane (7 x 50 - 60 ml). The extracts were combined, and after drying, the solvent was distilled off to give 8.81 g of N-(2-methoxybenzyl)ethanolamine as colorless crystals, yield 84.3%,
   ¹H-NMR spectrum (in heavy chloroform), δppm:
   2.74(2H,t), 2.83(2H,s), 3.63(2H,t), 3.80(2H,s), 3.84(3H,s), 6.87(3H,m), 7.24(1H,m)).

N-(3-Methoxybenzyl)ethanolamine was obtained from 3-methoxybenzylideneethanolamine in the same way as a colorless liquid, yield 84.0%,
¹H-NMR spectrum (in deutero-chloroform), δppm:
2.39(2H,s), 2.78(2H,t), 3.64(2H,t), 3.77(2H,s), 3.80(3H,s), 6.87(3H,m), 7.24(1H,m)).

N-(4-Methoxybenzyl)ethanolamine was obtained from 4-methoxybenzylideneethanolamine in the same way as colorless crystals, yield 82.6%,
¹H-NMR spectrum (in deutero-chloroform), δppm:
2.51(2H,s),
2.78(2H,t), 3.63(2H,t), 3.73(2H,s), 3.79(3H,s), 6.85(2H,d), 7.22(2H,d)).

### Method of Manufacturing Intermediate 16 - 18: Manufacture of N-(2-, 3- and 4-methoxybenzyl)ethylene diamines

Dodecyl bromide was reacted separately with the N-(2-, 3- and 4-methoxybenzyl)ethanolamines obtained in Method of Manufacturing Intermediate 13 - 15, in the same way as in Method of Manufacturing Intermediate 2 to obtain the N-dodecyl-N-(2-, 3- and 4-methoxybenzyl) ethanolamines. These compounds were then respectively reacted with phthalimide to obtain the (2-, 3- and 4-methoxybenzyl)-N-(2-phthalimidoethyl)amines, which were then respectively reacted with hydrazine hydrate so as to obtain the target compounds.

### Method of Manufacturing Intermediate 19 - 21: Manufacture of 2-hydroxyethyl N-(2-dodecyl(2-, 3- and 4-methoxybenzyl)aminoethyl)carbamates

The dodecyl-N-(2-, 3- and 4-methoxybenzyl) ethylenediamines obtained in Method of Manufacturing Intermediate 16 - 18 were respectively reacted with ethylene carbonate in the same way as in Method of Manufacturing Intermediate 3 so as to obtain the following target compounds:
2-hydroxyethyl N-(2-(dodecyl(2-methoxybenzyl) amino)ethyl)carbamate,
IR-spectrum (neat) _{cm}⁻¹: 3362, 2928, 2856, 1705, 1495, 1464, 1245, 754.
¹H-NMR spectrum (in deutero-chloroform), δppm:
0.88(3H,t), 1.25(18H,s), 1.44(2H,m), 2.43(2H,t), 2.59(2H,t), 3.26(2H,q), 3.59(2H,s), 3.80(2H,m), 3.84(3H,s), 4.19(2H,t), 5.72(1H,br), 6.92(2H,m), 7.25(2H,m).

2-hydroxyethyl N-(2-(dodecyl(3-methoxybenzyl)amino)ethyl)carbamate,
IR-spectrum (neat) _{cm}⁻¹: 3350, 2928, 2858, 1705, 1491, 1466, 1265, 1151, 1048, 777, 694.
¹H-NMR spectrum (in deutero-chloroform), δppm:
0.88(3H,t), 1.25(18H,s), 1.49(2H,m), 2.47(2H,t), 2.55(2H,t), 3.20(2H,q), 3.55(2H,s), 3.77(2H,m), 3.81(3H,s), 4.17(2H,t), 5.24(1H,br), 6.84(3H,m), 7.23(1H,t).

2-hydroxyethyl N-(2-(dodecyl(4-methoxybenzyl) amino)ethyl)carbamate,
IR-spectrum (neat) _{cm}⁻¹: 3350, 2928, 2858, 1705, 1514, 1249.
¹H-NMR spectrum (in CDCl₃), δppm: 0.88(3H,t), 1.25(18H,s), 1.46(2H,m), 2.42(2H,t), 2.52(2H,t), 3.20(2H,q), 3.50(2H,s), 3.77(2H,m), 3.80(3H,s), 4.17(2H,t), 5.20(1H,br), 6.85(2H,d), 7.19(2H,d).

### Method of Manufacturing Intermediate 22 - 24: Manufacture of N-(2-, 3- and 4-pyridylmethyl) carbamates

Each 10.0 g (92.47 mmol) of 2-, 3- and 4-aminomethyl- pyridine was heated respectively with 8.14 g (92.47 mmol) of ethylene carbonate at 60°C for 7 hours according to the Method of Manufacturing Intermediate 3, and the product purified to obtain the following target compounds:
2-hydroxyethyl N-(2-pyridylmethyl)carbamate, needle-shaped crystals (recrystallized from acetone), yield 14.25 g (78.6%),
¹H-NMR spectrum (in deutero-chloroform), δppm:
3.15(1H,br), 3.81(2H,t,J=4.5Hz), 4.23 (2H,t,J=4.5Hz, 4.47 (2H,d,J=5.7Hz), 6.20(1H,br), 7.17-7.28(2H,m), 7.67(1H,txd,J=7.7Hz), 8.51(1H,d,J=4.3Hz).

2-hydroxyethyl N-(3-pyridylmethyl)carbamate, an oil (silica gel chromatography, solvent: ethyl acetate/ methanol = 6:1 (v/v)), yield 16.7 g (92.0%),
¹H-NMR spectrum (in deutero-chloroform), δppm:
3.76(2H,t,J=4.6Hz), 4.06(1H,s), 4.17(2H,t,J=4.6Hz), 4.31(2H,d,J=5.9Hz), 6.38(1H,br), 7.20-7.26(1H,m), 7.62(1H,m), 8.44(2H,m).

2-hydroxyethyl N-(4-pyridylmethyl)carbamate, needle-shaped crystals (recrystallized from acetone), yield 14.1 g (77.7%),
¹H-NMR spectrum (in deutero-chloroform), δppm:
3.06(1H,s), 3.82(2H,t,J=4.4Hz), 4.24 (2H,t,J=4.6Hz), 4.35 (2H,d,J=6.2Hz), 5.79(1H,br), 7.19(2H,d,J=6.1Hz), 8.50 (2H,dxd,J=4.5Hz).

### Method of Manufacturing Intermediate 25: Manufacture of 2-hydroxyethyl N-(2-(2-pyridyl)ethyl)carbamates

2-(2-Pyridyl)ethylamine (10.0 g (81.9 mmol)) and 7.12 g (81.9 mmol) of ethylene carbonate were reacted together according to the Method of Manufacturing Intermediate 3 so as to obtain the target compound.

### Method of Manufacturing Intermediate 26: Manufacture of 1-dodecyl-3-(2-hydroxyethyloxycarbonylaminomethyl) pyridinium bromide

2-Hydroxyethyl N-(3-pyridylmethyl)carbamate (9.81 g (50 mmol)) obtained in Method of Manufacturing Intermediate 23 and 24.9 g (100 mmol) of dodecyl bromide were mixed together, and stirred at 120°C for 3 hours. Methanol was added, the mixture washed with hexane, the methanol distilled off, and the product purified by silica gel chromatography (solvent: acetonitrile/water = 9:1 (v/v) to give 18.16 g of the target compound (yield 81.5%).

### Method of Manufacturing Intermediate 27 - 29: Manufacture of N-(2-, 3- and 4-Chlorobenzyl) ethanolamines

2-, 3- and 4-Chlorobenzaldehydes were respectively reacted together with ethanolamine as in Method of Manufacturing Intermediate 13 - 15 to obtain the N-(2-, 3- and 4-chlorobenzylidene)ethanolamines respectively. These compounds were reduced with sodium borohydride to obtain the following target compounds as oils:
N-(2-chlorobenzyl) ethanolamine, b.p. 130-133°C/3 mm Hg, yield 81.4%, ¹H-NMR spectrum (in deutero-chloroform), δppm: 2.57(2H,s), 2.77(2H,t), 3.65(2H,t) 3.89(2H,s), 7.1-7.4(4H,m).

N-(3-chlorobenzyl) ethanolamine, b.p. 137-140°C/3 mm Hg, yield 80.7%, ¹H-NMR spectrum (in deutero-chloroform), δppm: 2.48(2H,s), 2.77(2H,t), 3.65(2H,t) 3.77(2H,s), 7.1-7.3(4H,m).

N-(4-chlorobenzyl) ethanolamine, b.p. 128-130°C/3 mm Hg, yield 70.5%, ¹H-NMR spectrum (in deutero chloroform), δppm: 2.52(2H,s), 2.75(2H,t), 3.64(2H,t) 3.76(2H,s), 7.2-7.3(4H,m).

### Method of Manufacturing Intermediate 30 - 32: Manufacture of N-(2-, 3- and 4-chlorobenzyl) ethylenediamines

The N-(2-, 3- and 4-chlorobenzyl)ethanolamines obtained in Method of Manufacturing Intermediate 27 - 29 were respectively reacted with dodecyl bromide in the same way as in Method of Manufacturing Intermediate 16 to obtain the N-(2-, 3- and 4-chlorobenzyl)-N-dodecylethanolamines. These compounds were then respectively reacted with phthalimide to obtain the N-(2-, 3- and 4-chlorobenzyl)-N-dodecyl-N-(2-phthalimidoethyl)amines, which were then respectively reacted with hydrazine hydrate so as to obtain the target compounds.

### Method of Manufacturing Intermediate 33 - 35: Manufacture of 2-hydroxyethyl N-(2-((2-, 3- and 4-chlorobenzyl)dodecylamino)ethyl)carbamate

The N-(2-, 3- and 4-chlorobenzyl) ethylenediamines obtained in Method of Manufacturing Intermediate 30 - 32 were respectively reacted with ethylene carbonate according to the Method of Manufacturing Intermediate 3 so as to obtain the following target compounds:
2-hydroxyethyl N-(2-((2-chlorobenzyl) -dodecylamino)ethyl)carbamate, yield 87.2%,
¹H-NMR spectrum (in deutero-chloroform): 0.88(3H,t), 1.25(18H,s), 1.46(2H,m), 2.46(2H,t), 2.59(2H,t), 3.22(2H,q), 3.67(2H,s), 3.78(2H,m), 4.17(2H,m), 5.30(1H,br), 7.1-7.3(2H,m), 7.3-7.5(2H,m).

2-hydroxyethyl N-(2-((3-chlorobenzyl) -dodecylamino)ethyl)carbamate, yield 84.0%,
¹H-NMR spectrum (in heavy chloroform): 0.88(3H,t), 1.25(18H,s), 1.49(2H,m), 2.43(2H,t), 2.54(2H,t), 3.21(2H,q), 3.54(2H,s), 3.80(2H,m), 4.19(2H,m), 5.15(1H,br), 7.1-7.4(4H,m).

2-hydroxyethyl N-(2-((4-chlorobenzyl) -dodecylamino)ethyl)carbamate, yield 95.2%,
¹H-NMR spectrum (in deutero-chloroform): 0.88(3H,t), 1.25(18H,s), 1.49(2H,m), 2.42(2H,t), 2.53(2H,t), 3.21(2H,q), 3.52(2H,s), 3.80(2H,m), 4.19(2H,m), 5.16(1H,br), 7.2-7.4(4H,m).

### Method of Manufacturing Intermediate 36: Manufacture of 2-hydroxyethyl N-(2-(benzyldecylamino)ethyl) carbamate

N-Benzylethanolamine was reacted with decyl bromide according to the Method of Manufacturing Intermediate 1 to obtain N-benzyl-N-decylethanolamine. This was converted to the phthalimide derivative according to the Method of Manufacturing Intermediate 2 and then treated with hydrazine to obtain N-benzyl-N-decylethylenediamine, which was then reacted with ethylene carbonate according to the Method of Manufacturing Intermediate 3 so as to obtain the target compound.

### Method of Manufacturing Intermediate 37: Manufacture of 2-hydroxyethyl N-(2-(benzyltetradecylamino)ethyl) carbamate

The same procedure as in Method of Manufacturing Intermediate 36 was followed, except that the reaction was performed using tetradecyl bromide instead of decyl bromide to obtain the target compound.

### Example 1: 2-[N-(2-Benzylhexadecylaminoethyl)-carbamoyloxy]ethyl methacrylate

2-Hydroxyethyl N-(2-benzylhexa-decylaminoethyl) -carbamate (1.46 g (3.66 mmol)) and 0.63 g (6.32 mmol) of triethylamine were dissolved in 30 ml chloroform, and to this a solution of 0.33 g (3.20 mmol) of methacryloyl chloride dissolved in 5 ml dichloromethane was added dropwise in at room temperature. The reaction mixture was stirred at room temperature for 2 hours further, and then washed with aqueous sodium hydroxide solution and then with brine. After drying (MgSO₄), the solvent was distilled off under reduced pressure, and the residue separated and purified by silica gel chromatography (solvent: dichloromethane/ether = 20:1) to give 0.73 g (43.7%) of the target compound.
IR spectrum (neat) cm⁻¹: 3382, 2928, 2856, 1725, 1638, 1510, 1456, 1168, 737, 698.
¹H-NMR spectrum (in deutero-chloroform): δppm:
0.88(3H,t), 1.26(26H,s), 1.46(2H,m), 1.95(3H,s), 2.43(2H,t), 2.53(2H,t) 3.20(2H,q), 3.55(2H,s), 4.30(4H,m), 5.14(1H,br), 5.59(1H,m), 6.14(1H,s), 7.28(5H,m).

### Example 2: 2-[N-(2-Benzyldodecylaminoethyl) -carbamoyloxy]ethyl methacrylate

The reaction was carried out as in Example 1 using 7.00 g (17.22 mmol) of 2-hydroxyethyl N-(2-benzyldodecylamino)ethyl)carbamate, 3.40 g (33.60 mmol) of triethylamine and 1.80 g (17.22 mmol) of methacryloyl chloride. The product was purified by silica gel chromatography (solvent: dichloromethane/ether = 20:1 or ether:hexane = 2:1) to give 6.62 g (yield 81.0%) of the target compound.
IR spectrum (neat) cm⁻¹: 3382, 2928, 2858, 1725, 1638, 1508, 1456, 1168, 737, 700.
¹H-NMR spectrum (in deutero-chloroform): δppm:
0.88(3H,t), 1.25(18H,s), 1.47(2H,m), 1.95(3H,s), 2.44(3H,t), 2.54(2H,t) 3.21(2H,q), 3.56(2H,s), 4.31(4H,m), 5.13(1H,br), 5.59(1H,s), 6.14(1H,s), 7.29(5H,m).

### Example 3: 2-[N-(2-Benzylhexylaminoethyl) -carbamoyloxylethyl methacrylate

The reaction was carried out as in Example 2 using 7.00 g (21.71 mmol) of 2-hydroxyethyl N-(2-benzylhexylaminoethyl)carbamate, 4.28 g (42.30 mmol) of triethylamine and 2.27 g (21.71 mmol) of methacryloyl chloride. The product was treated as in Example 2 to give 7.42 g (yield 87.5%) of the target compound.
IR spectrum (neat) cm⁻¹: 3378, 2932, 2862, 1725, 1638, 1508, 1456, 1168, 739, 700.
¹H-NMR spectrum (in heavy chloroform): δppm:
0.87(3H,t), 1.25(6H,s), 1.47(2H,m), 1.96(3H,s), 2.43(3H,t), 2.54(2H,t) 3.21(2H,q), 3.56(2H,s), 4.31(4H,m), 5.15(1H,br), 5.59(1H,s), 6.14(1H,s), 7.29(5H,m).

### Example 4 (1): [1-Butoxycarbonyl-1-methylethylene/1-(2-N-(2-benzylhexadecylaminoethyl)carbamoyloxy)ethoxycarbonyl)-1-methylethylene] copolymer (90:10 mol%)

The monomer (0.318 g (10 mol%)) obtained in Example 1 was dissolved in 10 ml of toluene, the toluene was distilled off, and the residue again dissolved in toluene (5 ml). To this, 0.772 g of butyl methacrylate and azo-bisisobutyronitrile (0.1 mol% with respect to the monomer) were added and the solution was degassed in vacuo while cooling it in a Dry-Ice bath. After dega, the vessel was stoppered tightly, and then heated at 60°C for 24 to 96 hours. The reaction mixture was added to 200 ml of ethanol and the reaction product precipitated was washed with methanol and dried in vacuo at 50°C to give 0.0955 g of the target copolymer as a light yellow resinous substance. The ¹H-NMR spectrum (in deutero-chloroform, concentration 2%) of this substance showed no absorptions that could be attributed to olefinic protons which are expected to occur in the region of δ5.6 and 6.1 ppm, thereby confirming that there was effectively none of the starting material monomer remaining in the product. A peak was found at δ7.3 ppm which could be assigned to the one due to the protons in the benzene ring. The content of the group due to the monomer of Example 3 in the product, as calculated from the area of this peak, was 10.5 mol%. Also, in the X ray photoelectron spectroscopy of a film obtained by casting a dimethylformamide solution of this reaction product on a glass plate, in addition to the strong peaks O₁ₛ, C₁ₛ, a N₁ₛ peak was observed at 400 eV. The atomic ratios on the film surface calculated from the area of this peak were N/C = 1.54%, O/C = 20.91%. The amount of the monomer unit of Example 3 in the product, as calculated from these values, was 7.6 and 10.8 mol% respectively. These values agreed well with the amount of starting material monomer.

### Example 4 (2) [1-Butoxycarbonyl-1-methylethylethylene/1-(2-(N-(2-benzylhexylaminoethyl )carbamoyloxy)ethoxycarbaboyl)-1-methylethylene]copolymer (86.9:13.1 mol%)

The same procedure as in Example 4 (1) was followed, excepting that the reaction was performed using 1.2139 g of the monomer obtained in Example 3 and 2.9268 g of butyl methacrylate and for 96 hours. The reaction mixture was treated in the same way to give 3.6171 g of the target compound as a light yellow resin.

### Example 5 (1): [1-Butoxycarbonyl-1-methylethylene/1-(2-(N-(2-benzylhexylaminoethyl)carbamoyloxy)ethoxycarbonyl)-1-methylethylene] copolymer (95:5 mol%)

The same procedure as in Example 4 was followed, except that the reaction was performed using 0.195 g (5 mol%) of the monomer obtained in Example 3 and 1.002 g of butyl methacrylate and for 96 hours. The reaction mixture was treated in the same way as in Example 4(1) to give 1.0388 g of the target compound as a light yellow resin.

### Example 5 (2) [1-Butoxycarbonyl-1-methylethylene/1-(2-(N-(2-benzylhexylaminoethyl)carbamoyloxy)ethoxycarbonyl)-1-methylethylene] copolymer (93.3:6.7 mol%)

The same procedure as in Example 4 (1) was followed, excepting that the reaction was performed using 0.6675 g of the monomer obtained in Example 3 and 3.3951 g of butyl methacrylate and for 96 hours. The reaction mixture was treated in the same way as above to give 3.7092 g of the target compound as a light yellow resinous substance.

### Example 6: [1-Butoxycarbonyl-1-methylethylene/1-(2-(N-(2-benzyldodecylaminoethyl)carbamoyloxy)ethoxycarbonyl) -1-methylethylene] copolymer (90:10 mol%)

The same procedure as in Example 4 was followed, excepting that the reaction was performed using 1.0634 g of the monomer obtained in Example 2 and 2.8652 g of butyl methacrylate and for 96 hours. The reaction solution was treated in the same way to give 3.4740 g of the target compound as a light yellow resin.

### Example 7: [1-Butoxycarbonyl-1-methylethylene/1-(2-(N-(2-benzyldodecylaminoethyl)carbamoyloxy) ethoxycarbonyl)-1-methylethylene] copolymer (95:5 mol%)

The same procedure as in Example 4 was followed, excepting that the reaction was performed using 1.1407 g of the monomer obtained in Example 2 and 6.4925 g of butyl methacrylate and for 96 hours. The reaction mixture was treated in the same way to give 7.3605 g of the target compound as a light yellow resin.

### Example 8 : [1-Butoxycarbonyl-1-methylethylene/1-(2-(N-(2-benzylhexadecylaminoethyl)carbamoyloxy) ethoxycarbonyl)-1-methylethylene] copolymer (90:10 mol%)

The same procedure as in Example 4 was followed, excepting that the reaction was performed using 1.2793 g of the monomer obtained in Example 1 and 4.1991 g of butyl methacrylate and for 96 hours. The reaction mixture was treated in the same way to give 4.8565 g of the target compound as a light yellow resin.

### Example 9: [1-Butoxycarbonyl-1-methylethylene/1-(2-(N-(2 -benzylhexadecylaminoethyl)carbamoyloxy) ethoxycarbonyl)-1-methylethylene] copolymer (95:5 mol%)

The same procedure as in Example 4 was followed, excepting that the reaction was performed using 0.5882 g of the monomer obtained in Example 1 and 4.0666 g of butyl methacrylate and for 96 hours. The reaction solution was treated in the same way to give 4.3435 g of the target compound as a light yellow resin.

### Example 10: [1-Butoxycarbonyl-1-methylethylene/1-(2-(N-(2-benzylhexadecylmethylammonioethyl)-carbamoyloxy)ethoxycarbonyl)-1-methylethylene iodide] copolymer (90:10 mol%)

The product obtained in Example 4 (60.8 mg) was dissolved in 1 ml of dimethylformamide. To this, 0.1562 g of iodo-methane was added and the mixture stirred at room temperature with the exclusion of light for 80.5 hours.

The reaction mixture was then poured into water, the resinous material precipitated was filtered, and dried at 50°C in vacuo for 15 hours to give 51.5 mg of the target copolymer as a yellow-brown solid film. A chloroform solution of this solid was cast on a PET film, and the X ray photoelectron spectrograph of it was measured. Apart from strong O₁ₛ, C₁ₛ peaks, peaks were also observed at 630.5, 619.0 and 49.0 eV due respectively to the 3d_{3/2}, 3d_{5/2} and 4d orbitals of the I atom. Peaks were also observed at 401.96 eV due to N⁺ and at 400.07 eV due to N. From the areas of these peaks, the surface element ratios were respectively O/C = 24.93; N/C = 0.93; N⁺/C = 0.64; I/C = 0.82. Compared to the calculated values assuming that all the tertiary N in the starting material copolymer were converted to quaternary N, i.e. O/C = 20.95; N/C = 0.95; N⁺/C = 0.95; I/C = 0.95, it appears that the rate of conversion to the quaternary derivative was approx. 80%.

### Example 11: [1-Butoxycarbonyl-1-methylethylene/1-(2-(N-(2-benzylhexylmethylammonio)ethyl) carbamoyloxy)ethoxycarbonyl)-1-methylethylene iodide] copolymer (93.3:6.7 mol%)

The same procedure as in Example 10 was followed, excepting that the reaction was performed using 3.7092 g of the copolymer obtained in Example 5 and 0.5376 g of iodomethane for 120 hours. The reaction mixture was treated as in Example 10 to give 3.8490 g of the target compound as a yellow-brown solid film.

### Example 12: [1-Butoxycarbonyl-1-methylethylene/1-(2-(N-(2-benzyldodecylmethylammonioethyl) carbamoyloxy)ethoxycarbonyl)-1-methylethylene iodide] copolymer (90:10 mol%)

The same procedure as in Example 10 was followed, excepting that the reaction was performed using 3.4740 g of the copolymer obtained in Example 6 and 0.9585 g of iodomethane and for 120 hours. The reaction mixture was treated as in Example 10 to give 3.6893 g of the target compound as a yellow-brown solid film.

### Example 13: [1-Butoxycarbonyl-1-methylethylene/1-(2-(N-(2-benzyldodecylmethylarnonioethyl) carbamoyloxy)ethoxycarbonyl)-1-methylethylene iodide] copolymer (95:5 mol%)

The same procedure as in Example 10 was followed, excepting that the reaction was performed using 7.3605 g of the copolymer obtained in Example 7 and 1.6872 g of iodomethane and for 120 hours. The reaction solution was treated as in Example 10 to give 7.3744 g of the target compound as a yellow-brown solid film.

### Example 14: [1-Butoxycarbonyl-1-methylethylene/1-(2-(N-(2-benzylhexadecylmethylammonioethyl) carbamoyloxy]ethoxycarbonyl]-1-methylethylene iodide copolymer (92.5:7.5 mol%)

The same procedure as in Example 10 was followed, excepting that the reaction was performed using 4.8565 g of the copolymer obtained in Example 8 and 2.0718 g of iodomethane and for 120 hours. The reaction mixture was treated as in Example 10 to give 5.0704 g of the target compound as a yellow-brown solid film.

### Example 15: [1-Butoxycarbonyl-1-methylethylene/1-(2-(N-(2-benzylhexadecylmethylammonioethyl) carbamoyloxy)ethoxycarbonyl)-1-methylethylene iodide] copolymer (96.3:3.7 mol%)

The same procedure as in Example 10 was followed, excepting that the reaction was performed using 4.3435 g of the copolymer obtained in Example 9 and 1.0517 g of iodomethane and for 120 hours. The reaction mixture was treated as in Example 10 to give 4.3903 g of the target compound as a yellow-brown solid film.

### Example 16: 2-[N-(2-Benzylhexadecylaminoethyl) -carbamoyloxy]ethyl methacrylate

2-Hydroxyethyl N-(2-(benzylhexadecylamino) -ethyl) carbamate (1.00 g (2.16 mmol)) and 0.87 g (8.65 mmol) of triethylamine were dissolved in 30 ml chloroform, and a solution of 0.23 g (21.6 mmol) of methacryloyl chloride dissolved in 5 ml dichloromethane was gradually added at room temperature with stirring. After stirring the reaction solution overnight, the solvent was distilled off. Ether was added to the residue, which was then washed with sodium bicarbonate solution and by brine successively. The ether was distilled off, and the residue purified by silica gel chromatography (solvent: ether/hexane = 2:1) to give 0.96 g of the target compound (yield 83.5%) as a light yellow oil.
IR spectrum (neat) cm⁻¹: 3364, 2928, 2858, 1738, 1506, 1468, 1156, 737, 698.
¹H-MMR spectrum (in deutero-chloroform): δppm:
0.88(3H,t), 1.18(6H,d), 1.26(26H,s), 1.46(2H,m), 2.43(2H,t), 2.53(2H,t) 3.21(2H,q), 3.56(2H,s), 4.24(4H,s), 5.12(1H,br), 7.29(5H,m).

### Example 17: 2-[N-(2-Benzyldodecylaminoethyl) -carbamoyloxy]ethyl isobutyrate

The reaction was carried out as in Example 16, but using 1.00 g of 2-hydroxyethyl N-(2-benzyldodecylaminoethyl)carbamate and 0.26 g of isobutyryl chloride in 30 ml of dichloromethane in the presence of 1.00 g of triethylamine at room temperature. The reaction mixture was treated in the same way as in Example 16, and the residue was purified by silica gel chromatography (solvent: ether/hexane = 2:1) to give 0.94 g of the target compound (yield 80.2%) as a light yellow oil.
IR spectrum (neat) cm⁻¹: 3382, 2928, 2858, 1725, 1638, 1508, 1456, 1168, 737, 700.
¹H-NMR spectrum (in deutero-chloroform): δppm:
0.88(3H,t), 1.25(18H,s), 1.47(2H,m), 1.95(3H,s), 2.44(3H,t), 2.54(2H,t) 3.21(2H,q), 3.56(2H,s), 4.31(4H,m), 5.13(1H,br), 5.59(1H,s), 6.14(1H,s), 7.29(5H,m).

### Example 18: 2-[N-(2-Benzylhexylaminoethyl) -carbamoyloxy]ethyl methacrylate

The reaction was carried out as in Example 16, but using 1.00 g of 2-hydroxyethyl N-(2-benzylhexylaminoethyl)carbamate and 0.33 g of isobutyryl chloride in 30 ml of dichloromethane in the presence of 1.25 g of triethylamine at room temperature. The reaction mixture was treated as in Example 16, and the residue was purified by silica gel chromatography (solvent: ether/hexane = 2:1) to give 1.18 g of the target compound (yield 97.0%) as a light yellow oil.
IR spectrum (neat) cm⁻¹: 3364, 2934, 2862, 1734, 1497, 1456, 1158, 739, 700.
¹H-MMR spectrum (in deutero-chloroform): δppm:
0.87(3H,t), 1.18(6H,d), 1.47(2H,m), 2.44(2H,t), 2.54(2H,t), 3.21(2H,q), 3.56(2H,s), 4.25(4H,s), 5.15(1H,br), 7.29(5H,m).

### Example 19: Benzylhexadecyl[2-(2-isobutyryloxyethoxycarbonylamino)ethyl]methylammonium iodide

The 2-[N-(2-benzyldodecylaminoethyl) -carbamoyloxy]ethyl isobutyrate obtained in Example 16, and 5 mole equivalents of iodomethane, were dissolved in ethanol, and stirred at room temperature with the exclusion of light for 7 to 9 days. The solvent was distilled off under reduced pressure, and the residue was purified by silica gel chromatography (solvent: first ethyl acetate/ether = 1:1 (v/v), and then methanol) to give 0.40 g of the target compound (yield 63.1%) as a red oil.
¹H-NMR spectrum (in deutero-chloroform): δppm:
0.88(3H,t), 1.16(6H,d), 1.25(26H,m), 1.84(2H,br), 2.56(1H,t), 3.22(3H,s), 3.42(2H,m), 3.73(2H,br t), 3.86(2H,br t), 4.24(4H,s), 4.90(2H,d), 6.45(1H, br t), 7.49(3H,m), 7.62(2H,m).

### Example 20: Benzyldodecyl[2-(2-isobutyryloxyethoxycarbonylamino)ethyl]methylammonium iodide

The 2-[N-(2-benzyldodecylaminoethyl) carbamoyloxy]ethyl isobutyrate obtained in Example 17, and 5 mole equivalents of iodomethane, were reacted in ethanol according to the method of Example 19. The reaction mixture was treated as in Example 19 to give 0.27 g of the target compound (yield 47.4%) as a red oil.
¹H-NMR spectrum (in deutero-chloroform): δppm:
0.88(3H,t), 1.15(6H,d), 1.25(18H,m), 1.83(2H,br), 2.56(1H,m), 3.22(3H,s), 3.43(2H,m), 3.73(2H,br t), 3.86(2H,br t), 4.25(4H,s),
4.90(2H,d), 6.45(1H, br t), 7.49(3H,m), 7.61(2H,m).

### Example 21: Benzylhexyl[2-(2 isobutyryloxyethoxycarbonylamino)ethyl]methyl ammonium iodide

The 2-[N-(2-benzylhexylaminoethyl)carbamoyloxy] ethyl isobutyrate obtained in Example 18, and 5 mole equivalents of iodomethane, were reacted in ethanol according to the method of Example 19. The reaction mixture was treated as in Example 19 to give 0.37 g of the target compound (yield 46.7%) as a red oil.
¹H-MMR spectrum (in deutero-chloroform): δppm:
0.88(3H,t), 1.15(6H,d), 1.33(6H,m), 1.83(2H,br), 2.56(1H,m), 3.22(3H,s), 3.42(2H,m), 3.73(2H,br t), 3.86(2H,br t), 4.25(4H,s), 4.91(2H,d), 6.47(1H, br t), 7.49(3H,m), 7.61(2H,m).

### Example 22: [1-Butoxycarbonyl-1-methylethylene/1-(2-(N-(2-benzyldodecylaminoethyl)carbamoyloxy) ethoxycarbonyl)-1-methylethylene] copolymer (99:1 mol%)

The same procedure as in Example 4 was followed, except that the reaction was performed using 0.1586 g (1 mol%) of the monomer obtained in Example 2 and 4.7055 g of butyl methacrylate and for 96 hours. The reaction mixture was treated in the same way as in Example 4 to give 4.7579 g of the target compound as a light yellow resin.

### Example 23: [1-Butoxycarbonyl-1-methylethylene/1-(2-(N-(2-benzyldodecylaminoethyl)carbamoyloxy) ethoxycarbonyl)-1-methylethylene] copolymer (97:3 mol%)

The same procedure as in Example 4 was followed, excepting that the reaction was performed using 0.4317 g of the monomer obtained in Example 2 and 4.1823 g of butyl methacrylate. As in Example 4, after evacuating the reaction vessel, the reaction was continued at 60°C for 96 hours. The reaction mixture was treated in the same way as in Example 4 to give 0.7417 g of the target compound as a light yellow resin.

### Example 24: 2-[N-(2-Benzyldecylaminoethyl) carbamoyloxy]ethyl methacrylate

The same procedure as in Example 1 was followed, except that the reaction was performed using 1.00 g (2.73 mmol) of 2-hydroxyethyl N-(2-benzyldecylaminoethyl)carbamate, 1.00 g (9.89 mmol) of triethylamine and 0.29 g (2.73 mmol) of methacryloyl chloride. The product was separated and purified by silica gel chromatography (solvent: dichloromethane/ether = 20:1 or ether/hexane = 2:1) to give 1.07 g of the target compound (yield 90.31%).
¹H-NMR spectrum (in deutero chloroform): δppm:
0.88(3H,t), 1.24(14H,s), 1.4-1.6(2H,br), 1.95(3H,s), 2.46(2H,br), 2.57(2H,br), 3.23(2H,q br), 3.60(2H,s), 4.30(4H,m), 5.1-5.4(1H,br), 5.58(1H,s), 6.14(1H,s), 7.30(5H,m).

### Example 25: 2-[N-(2-Benzyltetradecylaminoethyl) carbamoyloxy]ethyl methacrylate

The same procedure as in Example 1 was followed, excepting that the reaction was performed using 1.00 g (2.37 mmol) of 2-hydroxyethyl N-(2-benzyldecylaminoethyl)carbamate, 1.00 g (9.89 mmol) of triethylamine and 0.25 g (2.37 mmol) of methacryloyl hydrochloride. The product was separated and purified by silica gel chromatography (solvent: dichloromethane/ether = 20:1 or ether/hexane = 2:1) to give 0.81 g of the target compound (yield 69.75%).
¹H-NMR spectrum (in deutero-chloroform): δppm:
0.88(3H,t), 1.24(22H,s), 1.4-1.6(2H,br), 1.95(3H,s), 2.45(2H,br), 2.56(2H,br), 3.21(2H,br), 3.59(2H,br), 4.30(4H,m), 5.1-5.3(1H,br), 5.58(1H,s), 6.14(1H,s), 7.30(5H,m).

### Example 26: 2-[N-(2-Benzyldecylaminoethyl) carbamoyloxy]ethyl isobutyrate

The reaction was carried out as in Example 16, but using 1.00 g of 2-hydroxyethyl N-(2-benzyldecylaminoethyl)carbamate and 0.29 g of isobutyryl hydrochloride in 30 ml of dichloromethane in the presence of 1.18 g of triethylamine at room temperature. The reaction mixture was treated in the same way as in Example 16, and the residue was purified by silica gel chromatography (solvent: ether/hexane = 2:1) to give 0.80 g of the target compound (yield 67.21%) as a light yellow oil.
¹H-NMR spectrum (in deutero-chloroform): δppm:
0.88(3H,t), 1.17(6H,d), 1.25(14H,s), 1.46(2H,m), 2.43(2H,t), 2.54(2H,t) 3.21(2H,q), 3.56(2H,s), 4.24(4H,s), 5.13(1H,br), 7.29(5H,m).

### Example 27: 2-[N-(2-Benzyltetradecylaminoethyl) -carbamoyloxy]ethyl isobutyrate

The reaction was carried out as in Example 16, but using 1.00 g of 2-hydroxyethyl N-(2-benzyltetradecylaminoethyl)carbamate and 0.25 g of isobutyryl chloride in 30 ml of dichloromethane in the presence of 1.02 g of triethylamine at room temperature. The reaction mixture was treated as in Example 16, and the residue was purified by silica gel chromatography (solvent: ether/hexane = 2:1) to give 0.93 g of the target compound (yield 79.75%) as a light yellow oil.
¹H-NMR spectrum (in deutero-chloroform): δppm:
0.87(3H,t), 1.18(6H,d), 1.25(22H,s), 1.47(2H,m), 2.44(2H,t), 2.54(2H,t), 3.21(2H,q), 3.56(2H,s), 4.25(4H,s), 5.15(1H,br), 7.29(5H,m).

### Example 28: Benzyldecyl[2-(2-isobutyryloxy ethoxycarbonylamino)ethyl]methyl ammonium iodide

2-[N-(2-Benzyldecylaminoethyl)carbamoyloxy]ethyl isobutyrate (0.76 g) obtained in Example 26, and 5 mole equivalents of iodomethane, were dissolved in acetonitrile, and stirred at room temperature under shielding of light for 3 days. The solvent was distilled off under reduced pressure, and the residue was purified by silica gel chromatography (solvent:-ethyl acetate/ether = 1:1 (v/v), then methanol) to give 0.65 g of the target compound (yield 64.71%) as a red oil.
¹H-NMR spectrum (in deutero-chloroform): δppm:
0.88(3H,t), 1.15(6H,d), 1.25(14H,m), 1.7-1.9 (2H,br), 2.56(1H,m), 3.22(3H,s), 3.3-3.6 (2H,m), 3.71(2H,br), 3.85(2H,br), 4.24(4H,s), 4.91(2H,d), 6.50(1H,t), 7.48(3H,m), 7.62(2H,m).

### Example 29 Benzyltetradecyl[2-(2-isobutyryloxyethoxy-carbonylamino)ethyl]methyl ammonium iodide

2-[N-(2-Benzyltetradecylamino ethyl)carbamoyloxy]ethyl isobutyrate (0.85 g) obtained in Example 26, and 5 mole equivalents of iodomethane, were dissolved in acetonitrile, and stirred at room temperature under shielding light for 3 days. The solvent was distilled off under reduced pressure, and the residue was purified by silica gel chromatography (solvent: ethyl acetate/ether = 1:1 (v/v), then methanol) to give 0.84 g of the target compound (yield 76.92%) as a red oil.
¹H-NMR spectrum (in deutero-chloroform): δppm:
0.88(3H,t), 1.15(6H,d), 1.25(22H,m), 1.7-1.9 (2H,br), 2.56(1H,m), 3.22(3H,s), 3.3-3.6 (2H,m), 3.72(2H,m), 3.86(2H,m), 4.24(4H,s), 4.91(2H,d), 6.50(1H,t), 7.48(3H,m), 7.60(3H,m).

### Example 30 1-Decyl-2-[2-(2-isobutyryloxyethoxycarbonylamino)ethyl] pyridinium bromide

(1) 2-Hydroxyethyl N-[2-(2-pyridyl)ethyl] -carbamate (5.00 g (23.78 mmol)) and 4.70 g of triethylamine were dissolved in 300 ml of dichloromethane, and to this, a solution of 2.55 g (23.78 mmol) of isobutyryl chloride dissolved in 30 ml dichloromethane was added dropwise at 0°C. The reaction mixture was stirred at the same temperature for 3 hours, and then washed with aqueous sodium hydroxide solution and with brine successively. The solvent was distilled off, and the residue separated and purified by silica gel chromatography (solvent: ethyl acetate) to give 5.58 g of 2-[N-(2-(2-pyridyl)ethyl)carbamoyloxy]ethyl isobutyrate.
(2) 2-[N-(2-(2-Pyridyl)ethylcarbamoyloxy]ethyl isobutyrate (0.56 g) obtained in (1) was mixed with 0.89 g of 1-bromodecane, and the mixture stirred at 120°C for 8 hours. After cooling, the reaction product was washed with ether, and purified by silica gel chromatography (solvent: acetonitrile/water = 9:1) to give 0.57 g of the target compound (yield 56.83%) as a grey paste.
   ¹H-NMR spectrum (in deutero-chloroform): δppm:
   0.87(3H,t), 1.15(6H,t), 1.25(14H,m), 1.96(2H,m), 2.56(1H,m), 3.52(2H,t) 3.71(2H,m), 4.20(4H,m), 4.90 (2H,t), 6.76(1H,t), 7.94(1H,t), 8.11(1H,d), 8.39(1H,t), 9.15(1H,d).

### Example 31 2-Isobutyroxyethyl N-(2-, 3- and 4-pyridyl-methyl)carbamate

Each 5.00 g (25.48 mmol) of 2-hydroxyethyl N-(2-, 3- and 4-pyridylmethyl)carbamate was dissolved in 300 ml dichloromethane (in the case of the 3-pyridyl derivative) or tetrahydrofuran (in the case of the 2-or 4-pyridyl derivatives). To this, 5.00 g of triethylamine were added, and the mixture was cooled to -10°C. A solution of 2.72 g (25.52 mmol) of isobutyryl chloride dissolved in 30 ml dichloromethane or tetrahydrofuran was added, and the reaction mixture stirred at the same temperature for 4 to 5 hours. If the reaction was carried out in tetrahydrofuran, the tetrahydrofuran was distilled off, and the residue was taken up in dichloromethane. The resulting solution was washed with sodium bicarbonate and then with brine, and dried with anhydrous magnesium sulfate. The solvent was distilled off, and the residue purified by silica gel chromatography (solvent: ethyl acetate) to give 4.48 g of 2-isobutyryloxyethyl N-(2-pyridylmethyl)carbamate (yield 67.28%) having the following ¹H-NMR spectrum (in deutero-chloroform):
δppm: 1.16(6H,d), 2.57(1H,m), 4.29(4H,m), 4.51(2H,d), 6.00(1H,br) 7.29(2H,m), 7.70(1H,td), 8.54 (1H,dd), 5.90 g (yield 88.61%) of 2-isobutyryloxyethyl N-(3-pyridylmethyl)carbamate having the following ¹H-NMR spectrum (in deutero-chloroform):
δppm: 1.15(6H,d), 2.55(1H,m), 4.29(4H,m), 4.39(2H,d), 5.59(1H,br) 7.29(2H,m), 7.68(2H,d), 7.8 (1H,dd), 8.53(2H,m),
and 3.05 g (yield 45.81%) of 2-isobutyryloxyethyl N-(4-pyridylmethyl)carbamate having the following ¹H-NMR spectrum (in deutero-chloroform): δppm: 1.16(6H,d), 2.57(1H,m), 4.31(4H,m), 4.39(2H,d), 5.56(1H,br) 7.24(2H,d), 8.55 (2H,dd).

### Example 32: 2-(N-(3-Pyridylmethyl)carbamoyloxy)ethyl methacrylate

Each 10.0 g (51.0 mmol) of the 2-hydroxyethyl N-(3-pyridylmethyl) carbamate obtained in Method of Manufacturing Intermediate 22 to 24 and 10.0 g of triethylamine were dissolved in 300 ml dichloromethane, and cooled to 0°C. A solution of 5.90 g (56.43 mmol) of methacryloyl hydrochloride in 30 ml dichloromethane was added dropwise and the mixture stirred at 0°C for 2 hours, and then at room temperature for 18 hours. After the reaction mixture being washed with a saturated aqueous solution of sodium bicarbonate and brine, the resulting solution was dried, the solvent distilled off, and the residue purified by silica gel chromatography (solvent: ethyl acetate) to give 8.94 g (yield 66.41%) of the target compound,
¹H-MMR spectrum (in deutero-chloroform), δppm:
1.93(3H,s), 4.3-4.5(6H,m), 5.57(2H,m), 6.11(1H,s), 7.28(1H,m), 7.68(1H,d), 8.53(2H,s).

### Example 33: 1-Dodecyl-2-[2-(2-methacryloyloxyethoxycarbonylamino)ethyl]pyridinium bromide

2-Hydroxyethyl N-(2-(2-pyridyl)ethyl) -carbamate (2.10 g (10 mmol)) obtained in Method of Manufacturing Intermediate 25 was mixed with 3.24 g (13 mmol) of lauryl bromide, and the mixture stirred at 120°C for 8 hours. The reaction product was purified by silica gel chromatography (solvent: ethyl acetate/methanol = 6:1, followed by acetonitrile and then acetonitrile/water = 9:1) so as to obtain 2.50 g of 1-dodecyl-2-[2-(2-methacryloyloxyethoxycarbonylamino)ethyl]pyridinium bromide (yield 54.5%),
¹H-NMR spectrum (in deutero-chloroform), δppm:
0.87(3H,t,J=6.4Hz), 1.24(18H,s), 1.94(2H,m), 3.48(2H,br), 3.6-3.85 (5H,m), 4.05(2H,br), 4.86(2H,t,J=7.5Hz), 7.11(1H,t), 7.97(1H,t,J=6.9Hz), 8.15(1H,d,J=7.8Hz), 8.47(1H,t,J=7.2Hz), 9.12(1H,d,J=6.2Hz).

This product (0.46 g (1 mmol)) and 0.40 g (1 mmol) of triethylamine were dissolved in 30 ml dichloromethane. To this, a solution of 0.13 g (1.2 mmol) of methacryloyl chloride was added dropwise while cooling in ice, and then the reaction mixture was stirred at room temperature for 19 hours. The solvent was distilled off, the reaction product separated by silica gel chromatography (solvent: acetonitrile/water = 9:1), and then purified by Amberlite XAD-2 chromatography to give 120 mg (yield 22.8%) of the target compound,
¹H-HMR spectrum (in deutero-chloroform), δppm:
0.87(3H,t,J=6.5Hz), 1.24(18H,s), 1.93(5H,m), 3.53(2H,t,J=6.6Hz), 3.70(2H,t,J=6.1Hz), 4.25(4H,s), 4.89(2H,t,J=7.7Hz), 5.90(1H,s), 6.12(1H,s), 6.85(1H,t), 7.98(1H,t), 8.17(1H,d), 8.40(1H,t), 9.27(1H,d).

### Example 34: 1-Dodecyl-3-[2-(2-methacryloyloxyethoxycarbonylamino)ethyl]pyridinium bromide

1-Dodecyl-3-[2-(2-methacryloyloxyethoxycarbonylamino)ethyl]pyridinium bromide (10.00 g (22.45 mmol)) obtained in Method of Manufacturing Intermediate 26 and 10.80 g (100 mmol) of triethylamine were dissolved in 300 ml dichloromethane, and a solution of 2.82 g (26.94 mmol) in 50 ml dichloromethane was added dropwise at 0°C. The mixture was stirred at 0°C for 1 hour, and then at room temperature for 22 hours. After the reaction mixture was washed with water, the solvent was distilled off, and the residue separated by silica gel chromatography (solvent: acetonitrile/water = 9:1) to give 6.48 g of the target compound (yield 56.1%),
¹H-NMR spectrum (in deutero-chloroform), δppm:
0.88(3H,t), 1.24(12H,s), 1.33(6H,s), 1.93(3H,s), 2.04(2H,m), 4.30(4H,m), 4.64(2H,s), 4.78(2H,t), 5.59(1H,m), 6.12(1H,s), 7.21(1H,t), 8.00(1H,t), 8.56(1H,d), 9.05(1H,d), 9.41(1H,s).

### Example 35: [1-butoxycarbonyl-1-methylethylene/1-(2-(N-(2-(1-dodecyl-2-pyridinio)ethyl)carbamoyloxy)-ethoxycarbonyl)-1-methylethylene bromide]copolymer (90:10 mol%)

Butyl methacrylate (1.6757 g) and 0.6889 g of the 1-dodecyl-2-[2-(2-methacryloyloxyethoxycarbonylamino)ethyl]pyridinium bromide obtained in Example 25, were dissolved in 11.823 ml of dimethylformamide. To this, 2.21 mg of azoisobutyronitrile was added, and after being degassed, the mixture was heated at 60°C for 42 hours with stirring. The reaction solution was poured into 200 ml water, 500 ml water added, stirring continued, and the solution washed with water for 4 days while replacing 500 ml of the water with fresh water every day. The insoluble material was filtered off, and this product dried under reduced pressure (50°C, 1 day) to obtain 1.6259 g of the target compound.

### Example 36: Benzyldodecylethyl [2-(2-isobutyroxyethoxycarbonylamino)ethyl]ammonium iodide

2-[N-(2-Benzyldodecylamino-ethylcarbamoyloxy]ethyl isobutyrate (0.48 g (1 mmol)) obtained in Example 17 and 0.76 g (5 mmol) of iodomethane were dissolved in 10 ml acetonitrile, and the solution stirred with the exclusion of light at 50°C for 3 days. The solvent was distilled off under reduced pressure, the product was subjected to silica gel chromatography (solvent: ethyl acetate followed by methanol), and the solvent in the methanol fraction was distilled off to give a red oil. This was further purified by silica gel chromatography using acetonitrile/water (9:1) as solvent to give 0.23 g of the target compound as a light yellow wax, yield 35.65%,
¹H-NMR spectrum (in deutero-chloroform), δppm:
0.88(3H,t), 1.16(6H,d), 1.26(18H,s), 1.50(3H,t), 1.84(2H,br), 2.57(1H,m), 3.15-3.35(2H,br), 3.35-3.70(4H,m), 3.90(2H,d), 4.24(4H,s), 4.77(2H,s), 6.63(1H,t), 7.40-7.60(5H,m).

### Example 37: Benzyldodecyl[2-(2-isobutyryloxyethoxycarbonylamino)ethyl]ammonium chloride

Benzyldodecyl[2-(2-isobutyroxyethoxy -carbonylamino)ethyl]methylammonium iodide (0.68 g) obtained in Example 20 was dissolved in a 70% aqueous solution of methanol, and the resulting solution passed through a Cl⁻Amberlite IRA-410 column. The column was washed with methanol, the eluates from the column were combined, the solvent was distilled off, and the residue purified by silica gel chromatography with acetonitrile/water (9:1) as eluent, to give 0.40 g of the target compound as a white wax, yield 69.0%,
¹H-NMR spectrum (in deutero-chloroform), δppm:
0.88(3H,t), 1.14(6H,d), 1.25(18H,s), 1.6-1.9(2H,br), 2.55(1H,m), 3.24(3H,s), 3.3-3.6(2H,m), 3.70(2H,br), 3.84(2H,br), 4.23(4H,s), 4.95(2H,q), 7.46(4H,m), 7.61(2H,m).

| Elemental analysis: As C₂₉H₅₁N₂O₄Cl, | | | | |
|---|---|---|---|---|
| Calculated: | C:66.07; | H:9.75; | N:5.31; | Cl:6.72 |
| Experimental: | C:66.09; | H:9.75; | N:5.28; | Cl:6.55. |

### Example 38: Benzyldodecyl[2-(2-isobutyryloxyethoxycarbonylamino)ethyl]methylammonium bromide

Benzyldodecyl[2-(2-isobutyroxyethoxycarbonylamino)ethyl]methylammonium iodide (0.68 g) obtained in Example 20 was dissolved in 70% aqueous methanol, and the resulting solution passed through a Amberlite IRA-410 column treated with lithium bromide. The target compound was obtained as a white wax, yield 61.3%,
¹H-NMR spectrum (in deutero-chloroform), δppm:
0.88(3H,t,J=6.4Hz), 1.15(6H,d,J=7.0Hz), 1.25(18H,s), 1.15-1.9(2H,br), 2.55(1H,m,J=7.0Hz), 3.24(3H,s), 3.3-3.6(2H,m), 3.70(2H,br), 3.86(2H,br), 4.23(4H,s), 4.97(2H,q), 6.98(1H,t,J=5.4Hz), 7.47(3H,m), 7.63(2H,m).

| Elemental analysis: As C₂₉H₅₁N₂O₄Br, | | | | |
|---|---|---|---|---|
| Calculated: | C:60.93; | H:8.99; | N:4.90; | Br:13.98 |
| Experimental: | C:60.21; | H:8.98; | N:4.91; | Br:14.09 |

### Examples 39 - 41: 1-Dodecyl, 1-tetradecyl- and 1-hexadecyl-2-[2-(2-isobutyryloxyethoxycarbonylaminoethyl]pyridinium bromide

Two mmol of the 2-[N-(2-(2-pyridyl)ethyl) -carbamoyloxy]ethyl isobutyrate obtained in Example 30(1) was mixed with 4 mmol each of the corresponding alkyl bromide, and the mixture heated with stirring at 120°C for 8 hours. After cooling, ether was added, and after stirring and, leaving to stand the ether layer was removed, and the residue purified by silica gel chromatography with acetonitrile/water (9:1) as solvent to give each of the following target compounds:
1-dodecyl derivative, yield 66.1%,
¹H-NMR spectrum (in deutero-chloroform), δppm:
0.88(3H,t), 1.16(6H,d), 1.25(18H,s), 2.00(2H,br), 2.57(1H,m), 3.52(2H,t), 3.72(2H,t), 4.20(4H,s), 4.90(2H,t), 6.76(1H,t), 7.93(1H,t), 8.11(1H,d), 8.38(1H,t), 9.13(1H,d).

1-tetradecyl derivative, yield 73.5%,
¹H-NMR spectrum (in deutero-chloroform), δppm:
0.88(3H,t), 1.15(6H,d), 1.25(22H,s), 2.00(2H,br), 2.56(1H,m), 3.52(2H,t), 3.71(2H,t), 4.20(4H,s), 4.91(2H,t), 6.79(1H,t), 7.96(1H,t), 8.12(1H,d), 8.37(1H,t), 9.19(1H,d).

1-hexadecyl derivative, yield 29.9%,
¹H-NMR spectrum (in deutero-chloroform), δppm:
0.88(3H,t), 1.16(6H,d), 1.25(26H,s), 1.97(2H,br), 2.57(1H,m), 3.53(2H,t), 3.71(2H,t), 4.19(4H,s), 4.91(2H,t), 6.80(1H,t), 7.95(1H,t), 8.12(1H,d), 8.39(1H,t), 9.19(1H,d).

### Examples 42 - 45: 1-Decyl-, 1-dodecyl-, 1-tetradecyl-and 1-hexadecyl-2-(2-isobutyryloxyethoxycarbonylaminomethyl)pyridinium bromide

2-[N-(2-Pyridylmethyl)carbamoyloxy]ethyl isobutyrate obtained in Example 31 was reacted with the corresponding alkyl bromide according to the method of Example 30, and the reaction mixture treated in the same way as in Examples 39 - 41 to obtain the following target compounds:
1-decyl derivative, yield 46.2%,
¹H-NMR spectrum (in deutero-chloroform), δppm:
0.88(3H,t), 1.16(6H,d), 1.25(14H,s), 1.98(2H,m), 2.57(1H,m), 4.27(4H,s), 4.91(4H,t), 7.59(1H,t), 7.94(1H,t), 8.26-8.45(2H,m), 9.20(1H,d).

1-dodecyl derivative, yield 14.6%,
¹H-NMR spectrum (in deutero-chloroform), δppm:
0.88(3H,t), 1.16(6H,d), 1.25(18H,s), 1.98(2H,m), 2.58(1H,m), 4.28(4H,s), 4.92(4H,m), 7.58(1H,t), 7.92(1H,t), 8.25-8.50(2H,m), 9.12(1H,d).

1-tetradecyl derivative, yield 23.0%,
¹H-NMR spectrum (in deutero-chloroform), δppm:
0.88(3H,t), 1.17(6H,d), 1.25(22H,s), 1.97(2H,m), 2.58(1H,m), 4.27(4H,s), 4.92(4H,m), 7.56(1H,t), 7.94(1H,t), 8.25-8.50(2H,m), 9.13(1H,d).

1-hexadecyl derivative, yield 19.3%,
¹H-NMR spectrum (in deutero-chloroform), δppm:
0.88(3H,t), 1.14(6H,d), 1.25(26H,s), 1.97(2H,m), 2.58(1H,m), 4.27(4H,s), 4.92(4H,m), 7.59(1H,t), 7.91(1H,t), 8.25-8.50(2H,m), 9.12(1H,d).

### Examples 46 - 49: 1-decyl-, 1-dodecyl-, 1-tetradecyl-and 1-hexadecyl-3-(2-isobutyryloxyethoxycarbonylaminomethyl)pyridinium bromide

2-[N-(3-Pyridylmethyl)carbamoyloxy]ethyl isobutyrate obtained in Example 31 was reacted with the corresponding alkyl bromide according to the method of Example 30, and the reaction mixture treated in the same way as in Examples 39 - 41 to obtain the following target compounds:
1-decyl derivative, yield 90.8%,
¹H-NMR spectrum (in deutero-chloroform), δppm:
0.87(3H,t), 1.16(6H,d), 1.25(14H,m), 2.05(2H,m), 2.57(1H,m), 4.26(4H,s), 4.65(2H,d), 4.78(2H,t), 7.18(1H,t), 8.00(1H,t), 8.57(1H,d), 9.05(1H,d), 9.45(1H,s).

1-dodecyl derivative, yield 91.3%,
¹H-NMR spectrum (in deutero-chloroform), δppm:
0.88(3H,t), 1.16(6H,d), 1.24(18H,m), 2.05(2H,m), 2.58(1H,m), 4.26(4H,s), 4.66(2H,d), 4.77(2H,t), 7.17(1H,t), 8.00(1H,t), 8.57(1H,d), 9.03(1H,d), 9.44(1H,s).

1-tetradecyl derivative, yield 71.6%,
¹H-NMR spectrum (in deutero-chloroform), δppm:
0.88(3H,t), 1.16(6H,d), 1.25(22H,m), 2.05(2H,m), 2.58(1H,m), 4.26(4H,s), 4.65(2H,d), 4.77(2H,t), 7.18(1H,t), 7.99(1H,t), 8.56(1H,d), 9.00(1H,d), 9.46(1H,s).

1-hexadecyl derivative, yield 64.7%,
¹H-NMR spectrum (in deutero-chloroform), δppm:
0.88(3H,t), 1.16(6H,d), 1.25(26H,m), 2.05(2H,m), 2.58(1H,m), 4.26(4H,s), 4.64(2H,d), 4.77(2H,t), 7.18(1H,t), 8.00(1H,t), 8.56(1H,d), 9.03(1H,d), 9.44(1H,s).

### Examples 50 - 53: 1-Decyl-, 1-dodecyl-, 1-tetradecyl-and 1-hexadecyl-4-(2-isobutyryloxyethoxycarbonylaminomethyl)pyridinium bromide

2-[N-(4-Pyridylmethyl)carbamoyloxy]ethyl isobutyrate obtained in Example 31 was reacted with the corresponding alkyl bromide according to the method of Example 30, and the reaction mixture treated in the same way as in Examples 39 - 41 to obtain the following target compounds:
1-decyl derivative, yield 97.4%,
¹H-NMR spectrum (in deutero-chloroform), δppm:
0.87(3H,t), 1.16(6H,d), 1.25(14H,m), 2.00(2H,m), 2.58(1H,m), 4.26(4H,s), 4.69(2H,d), 4.85(2H,t), 7.26(1H,t), 8.11(2H,d), 9.10(2H,d).

1-dodecyl derivative, yield 98.0%,
¹H-NMR spectrum (in deutero-chloroform), δppm:
0.88(3H,t), 1.16(6H,d), 1.25(18H,m), 2.00(2H,m), 2.58(1H,m), 4.26(4H,s), 4.69(2H,d), 4.84(2H,t), 7.22(1H,t), 8.11(2H,d), 9.10(2H,d).

1-tetradecyl derivative, yield 90.1%,
¹H-NMR spectrum (in deutero-chloroform), δppm:
0.88(3H,t), 1.16(6H,d), 1.25(22H,m), 1.99(2H,m), 2.58(1H,m), 4.26(4H,s), 4.69(2H,d), 4.84(2H,t), 7.21(1H,t), 8.11(2H,d), 9.09(2H,d).

1-hexadecyl derivative, yield 92.7%,
¹H-NMR spectrum (in deutero-chloroform), δppm:
0.88(3H,t), 1.16(6H,d), 1.25(26H,m), 1.99(2H,m), 2.58(1H,m), 4.26(4H,s), 4.69(2H,d), 4.84(2H,t), 7.20(1H,t), 8.11(2H,d), 9.08(2H,d).

### Examples 54 - 56: Dodecyl(2-, 3- and 4-methoxybenzyl) (2-(2-isobutyryloxyethoxycarbonylamino)methylammonium iodide

A) Each 1.42 g (3.26 mmol) of the 2-hydroxyethyl N-(2-(dodecyl(2-, 3- and 4-methoxybenzyl)amino)ethyl) carbamates obtained in Method of Manufacturing Intermediates 19 to 21 was dissolved together with 1.32 g of triethylamine in 30 ml dichloromethane, and a solution of 0.38 g (3.58 mmol) isobutyryl chloride in 5 ml dichloromethane was added dropwise at room temperature. After stirring for 2 hours, the solvent was distilled off, and the product dissolved in ether. This solution was washed with a saturated solution of sodium bicarbonate and with brine, and after drying (Na₂SO₄), the solvent was distilled off and the residue purified by silica gel chromatography (solvent: dichloromethane/ethyl acetate = 10:1 - 5:1 (v/v)) so as to obtain 1.15 g (yield 69.8%), 1.12 g (yield 71.4%) and 1.15 g (yield 76.8%) of 2-[N-(2-(dodecyl(2-, 3- and 4-methoxybenzyl) amino)ethyl)carbamoyloxy]ethyl isobutyrate respectively.
B) Each 0.45 g (0.89 mmol) of these products was respectively dissolved in 10 ml of acetonitrile, 0.62 g of methyl iodide was added to the solution, and the mixture stirred under shielding of light at room temperature for 65 hours. The solvent was removed, and the residue purified by silica gel chromatography (solvent: acetonitrile/water = 9:1) so as to obtain 0.38 g (66.1%), 0.30 g (52.2%) and 0.30 g (52.2%) of the target compounds respectively:
   2-methoxybenzyl derivative:
   ¹H-NMR spectrum (in deutero-chloroform), δppm:
   0.88(3H,t), 1.16(6H,d), 1.26(14H,s), 1.35(4H,s), 1.7-1.9(2H,br), 2.57(1H,m), 3.17(3H,s), 3.3-3.6(2H,m), 3.74(2H,q), 3.85(2H,m), 3.91(3H,s), 4.25(4H,s), 4.71(2H,q), 6.34(1H,t), 6.9-7.1(2H,m), 7.4-7.6(2H,m). 3-methoxybenzyl derivative:
   ¹H-NMR spectrum (in deutero-chloroform), δppm:
   0.88(3H,t), 1.15(6H,d), 1.25(14H,s), 1.33(4H,s), 1.7-2.0(2H,br), 2.56(1H,m), 3.17(3H,s), 3.3-3.6(2H,m), 3.72(2H,br), 3.85(5H,m), 4.25(4H,s), 4.85(2H,q), 6.54(1H,t), 6.9-7.4(4H,m).
   4-methoxybenzyl derivative:
   ¹H-NMR spectrum (in deutero-chloroform), δppm:
   0.88(3H,t), 1.15(6H,d), 1.25(14H,s), 1.32(4H,s), 1.7-2.0(2H,br), 2.56(1H,m), 3.18(3H,s), 3.3-3.5(2H,m), 3.67(2H,br), 3.83(5H,s), 4.25(4H,s), 4.81(2H,q), 6.52(1H,t), 6.94(2H,d), 7.53(2H,d).

### Examples 57 - 59: Dodecyl(2-, 3- and 4-chlorobenzyl) (2-(2-isobutyroxyethoxycarbonylamino)methylammonium iodide

Each 2-hydroxyethyl N-(2-(dodecyl(2-, 3- and 4-chlorobenzyl)amino)ethyl) carbamate obtained in Method of Manufacturing Intermediates 33 to 35 was reacted according to the method of Examples 54 - 56 so as to obtain the target compound:
2-chlorobenzyl derivative, yield 88.9%,
¹H-NMR spectrum (in deutero-chloroform), δppm:
0.88(3H,t), 1.16(6H,d), 1.25(14H,s), 1.36(4H,s), 1.8-2.1(2H,br), 2.56(1H,m), 3.28(2H,s), 3.3-3.5(2H,m), 3.6-3.8(2H,m), 3.89(2H,br), 4.25(4H,s), 5.00(2H,br), 6.50(1H,t), 7.4-7.6(3H,m), 8.00(1H,m).

3-chlorobenzyl derivative, yield 88.8%,
¹H-NMR spectrum (in deutero-chloroform), δppm:
0.88(3H,t), 1.16(6H,d), 1.25(14H,s), 1.33(4H,br), 1.7-2.0(2H,br), 2.56(1H,m), 3.25(2H,s), 3.3-3.6(2H,m), 3.77(2H,br), 3.87(2H,br), 4.25(4H,s), 5.00(2H,q), 6.43(1H,t), 7.45(2H,m), 7.59(1H,s), 7.65(1H,s). 4-chlorobenzyl derivative, yield 79.8%,
¹H-NMR spectrum (in deutero-chloroform), δppm:
0.88(3H,t), 1.16(6H,d), 1.25(14H,s), 1.31(4H,br), 1.7-2.0(2H,br), 2.56(1H,m), 3.21(2H,s), 3.3-3.6(2H,m), 3.70(2H,br), 3.89(2H,br), 4.25(4H,s), 5.02(2H,br), 6.40(1H,t), 7.41(2H,d), 7.64(2H,d).

### Example 60: 2-(N-(2-Benzyldecylaminoethyl) -carbamoyloxy)-3-hydroxypropyl isobutyrate and 3-(N-(2-benzyldecylaminoethyl)carbamoyloxy)-2-hydroxypropyl isobutyrate (a mixture)

A) Glycidyl isobutyrate (7.21 g (50 mmol)) and 0.52 g (5 mmol) of lithium bromide monohydrate were dissolved in 50 ml of N-methylpyrrolidone, and the solution was heated at 100°C for 3 hours while passing carbon dioxide under atmospheric pressure. The solvent was distilled off, and the residue purified by silica gel chromatography (solvent: dichloromethane - dichloromethane/ethyl acetate (2:1)) to give 3.50 g (37.8%) of 3-isobutyryloxy-1.2-propylene carbonate.
B) This product from A) was then reacted with an equimolar amount of benzyldodecylamine at 60°C for 4 hours, and the reaction product purified by silica gel chromatography to give the target compound.

### Example 61: Benzyldecyl(2-(3-isobutyryloxy-2-hydroxypropyloxycarbonylamino)ethyl)methyl-ammonium iodide and benzyldecyl(2-(2-isobutyryloxy-3-hydroxypropyloxycarbonylamino)-ethyl)methylammonium iodide (a mixture)

3.80 g (7.50 mmol) of the product obtained in Example 60 was dissolved in 40 ml acetonitrile, 5.22 g (37.5 mmol) of methyl iodide was added, and the resulting mixture was stirred at room temperature for 72 hours with the exclusion of light.

The solvent and excess methyl iodide were distilled off under reduced pressure, and the residue purified by silica gel chromatography (solvent: acetonitrile/water = 9:1) to give 3.93 g of the target compound (yield 80.6%).

### Example 62: 2-(N-(3-Pyridylmethyl)carbamoyloxy-3 -hydroxypropyl isobutyrate and 3-(N-(3-pyridylmethyl) -carbamoyloxy)-2-hydroxypropyl isobutyrate (a mixture)

3-Isobutyroxy-1.2-propylene carbonate obtained from glycidyl isobutyrate as described in Example 60-A), 1.70 g (9.00 mmol) of the product was reacted with 0.97 g (9.00 mmol) of 3-aminomethylpyridine at 60°C for 4 hours, and the purified product was further purified by silica gel chromatography (solvent: ethyl acetate) to give 2.60 g of the target compound (yield 97.4%).

### Example 63: 1-Dodecyl-3-(3-isobutyryloxy-2-hydroxypropyloxycarbonylamino)methyl-pyridinium bromide and 1-dodecyl-3-(2-isobutyryloxy-3-hydroxypropyloxycarbonylamino)methylpyridinium bromide (a mixture)

The compound obtained in Example 62 (2.60 g (8.76 mmol)) and 4.37 g (17.52 mmol) of lauryl bromide were mixed and blended together at 120°C for 3 hours. The reaction product was purified by silica gel chromatography (solvent: acetonitrile/water = 9:1)) to give 3.08 g of the target compound (yield 54.4%).

### Examples 64 - 68: [1-Butoxycarbonyl-1-methylethylene/1-(2-(N-(2-alkylbenzylaminoethyl)carbamoyloxy) -ethoxycarbonyl)-1-methylethylene] copolymer ((100-x):x mol%)

A predetermined amount of butyl methacrylate (monomer 1) and a predetermined amount of a predetermined 2-(N-(2-alkylbenzylaminoethyl)carbamoyloxy)ethyl methacrylate (monomer 2), were dissolved in an amount of toluene such that the total concentration of soluble materials was approx. 20 weight %. A predetermined amount of 2,2'-azobisisobutyronitrile (AIBN) was added, and the solution was intensely cooled, and degassed. Then, vessel was sealed and heated at 60°C for a predetermined time. The reaction mixture was then treated as described in Example 4(1) so as to obtain the target compounds. Table 1 shows the type of alkyl group and value of x (mol%) in the structures of the compounds manufactured by the aforementioned method, the amounts of monomer 1 and monomer 2 used (g), amount of AIBN used (mg), reaction time (hours), amount of copolymer obtained (g), and yield (%). The whole products from Examples 66, 67 were immediately used as starting materials for Examples 70, 71.

**Table 1**

| Ex. No. | Alkyl group | x | Monomer 1 | Monomer 2 | AIBN | Rct. time | Copolymer | |
|---|---|---|---|---|---|---|---|---|
| | | | | | | | extracted | Yld. |
| 64 | Tetradecyl | 10 | 1.0562 | 0.4147 | 1.35 | 92 | 1.1958 | 81.2 |
| 65 | Dodecyl | 100 | 0 | 0.9175 | 3.15 | 48 | 0.7417 | 80.8 |
| 66 | Dodecyl | 7 | 2.0197 | 0.5061 | 2.65 | 48 | - | - |
| 67 | Dodecyl | 4 | 2.4570 | 0.3410 | 3.06 | 48 | - | - |
| 68 | Decyl | 10 | 1.3572 | 0.4742 | 1.73 | 44 | 1.4538 | 79.3 |

### Examples 69 - 75: [1-Butoxycarbonyl-1-methylethylene/1-(2-(N-(2-alkylbenzylmethylammonioethyl) carbamoyloxy)ethoxycarbonyl)-1-methylethylene iodide] copolymer ((100-x):x mol%)

Predetermined amounts of the copolymers having a tertiary amino group obtained in Example 4(2) and Examples 64 - 68 (prepolymer), were respectively dissolved in a predetermined amount of N,N'-dimethylformamide (DMF), a predetermined amount of methyl iodide was added, and made to react according to the method of Example 10. The reaction product was then treated so as to obtain the target quartenary ammonium derivative copolymer (quaternary polymer) compounds. Table 2 shows the types of alkyl group and value of x (mol%) in the structures of the compounds manufactured by the aforementioned method, the amount of prepolymer and methyl iodide used (g), amount of DMF used (ml), reaction time (hours), and amount of quaternary polymer obtained (g).

**Table 2**

| Ex. No. | Alkyl group | x | Prepolymer | Methyl iodide | DMF | Rct. time | Quart. polymer extracted |
|---|---|---|---|---|---|---|---|
| 69 | Tetradecyl | 10 | 0.7996 | 1.9353 | 40 | 81 | 0.8698 |
| 70 | Dodecyl | 7 | - | 1.5817 | 20 | 192 | 1.8991 |
| 71 | Dodecyl | 4 | - | 1.3476 | 20 | 192 | 2.0585 |
| 72 | Dodecyl | 3 | 2.2218 | 0.210 | 25 | 96 | 1.9339 |
| 73 | Dodecyl | 1 | 2.3172 | 0.2 | 25 | 96 | 2.1075 |
| 74 | Decyl | 10 | 0.7049 | 0.2898 | 35 | 96 | 0.7540 |
| 75 | Hexyl | 13.1 | 3.6171 | 1.0291 | 36 | 135 | 3.8466 |

### Examples 76 - 77: [1-Butoxycarbonyl-1-methylethylene/1-(2-(N-(1-dodecyl-3-pyridiniomethyl)-carbamoyloxy)ethoxycarbonyl)-1-methylethylene bromide] copolymer (90:10 and 95:5 mol%)

Each 5.2726 g and 1.6055 g of the 2-(N-(1-dodecyl-3-pyridiniomethyl)carbamoyloxy)ethyl methacrylate obtained in Example 34, and each 13.1404 g and 8.4447 g of butyl methacrylate, were dissolved respectively in an amount of DMF such that the concentration of soluble material was approx. 20%, and each 16.86 mg and 10.3 mg of AIBN were added respectively. The reaction mixture was intensely cooled and degassed. Then, the vessel was sealed, and heated at 60°C for 42 hours. The reaction mixture was concentrated under reduced pressure, and the residue poured in to 500 ml water. The resulting precipitate was washed well with water to give 18.4299 g (yield 81.8%) and 10.0605 g (yield 81.6%) of the target compound respectively.

### Example 78: [1-Butoxycarbonyl-1-methylethylene/1-(2-(N-(1-dodecyl-3-pyridinioethyl)carbamoyloxy)ethoxycarbonyl)-1-methylethylene bromide] copolymer (90:10 mol%)

1-Dodecyl-2-[2-(2-methacryloyloxyethoxycarbonylamino)ethyl]pyridinium bromide (0.6889 g) obtained in Example 33 and 1.6757 g of butyl methacrylate were dissolved in 12 ml of DMF, and 2.21 mg of AIBN was added. The reaction mixture was intensely cooled and degassed. Then, the vessel was sealed, and heated at 60°C for 41.5 hours with stirring. The reaction mixture was poured into 200 ml water, and stirred. Stirring was continued for 4 days while replacing 500 ml of the water with fresh one every day. The resulting precipitate was collected by filtration and dried at 50°C under reduced pressure for 1 day to give 1.6259 g (yield 68.7%) of the target compound.

### Examples 79 - 80: [1-Butoxycarbonyl-1-methylethylene/1-(2-(N-(3-pyridylmethyl)carbamoyloxy)ethoxycarbonyl) -1-methylethylene]copolymer (90:10 and 95:5 mol%)

Each 5.833 g and 3.1107 g of the 2-(N-(3-pyridylmethyl)carbamoyloxy)ethyl methacrylate obtained in Example 32, and 28.2439 g and 31.81 g of butyl methacrylate, were dissolved respectively in an amount of DMF such that the concentration of soluble material was approx. 20%, and 36.2 mg and 38.7 mg of AIBN were added respectively. The reaction mixture was intensely cooled, and degassed. Then, the vessel was sealed, and heated at 60°C for 47 hours and 111 hours respectively with stirring. The reaction solution was poured into methanol, the resulting precipitate washed with methanol, collected by filteration, and dried to give 28.5255 g and 31.00 g of the target compound respectively.

### Examples 81 - 85: [1-(2-Hydroxyethoxycarbonyl)-1-methylethylene/1-(2-(N-(2-alkylbenzylmethylammonioethyl)carbamoyloxy)ethoxycarbonyl)-1-methylethylene iodide]copolymer (100-x):x mol%)

Predetermined amounts of 2-hydroxyethyl methacrylate (monomer 3) and of a predetermined amount of 2-(N-(2-alkylbenzylaminoethyl)carbamoyloxy)ethyl methacrylate (monomer 2) were dissolved in an amount of DMF such that the total concentration of soluble material was approx. 20%. A predetermined amount of AIBN was added, and then, the reaction mixture intensely cooled and degassed. Then, the vessel was sealed, and the mixture heated at 60°C for 68 hours. Next, the reaction mixture was diluted with the same amount of DMF as used hereinabove, a predetermined amount of methyl iodide was added, the vessel sealed, and the solution stirred at room temperature for 3 days. The reaction mixture was evaporated to dryness under reduced pressure, the residual copolymer broken up into small pieces, and washed well with water over a period of 2 weeks so as to obtain the target quaternary ammonium derivative copolymer (quaternary polymer) compounds. Table 3 shows the type of alkyl group and value of x (mol%) in the structures of the compounds manufactured by the aforementioned method, the amounts of monomer 3 and monomer 2 used (g), amount of AIBN used (ml), reaction time (hours), amount of methyl iodide (MeI) used (g), and amount of quaternary polymer obtained (g).

**Table 3**

| Ex. No. | Alkyl group | x | Monomer 3 | Monomer 2 | AIBN | Methyl iodide | Quart. polymer extracted |
|---|---|---|---|---|---|---|---|
| 81 | Hexadecyl | 5 | 2.5236 | 0.5417 | 3.32 | 0.7378 | 2.7192 |
| 82 | Dodecyl | 10 | 1.3738 | 0.555 | 1.94 | 1.0281 | 1.7275 |
| 83 | Dodecyl | 5 | 1.7125 | 0.3277 | 2.27 | 1.0287 | 1.6724 |
| 84 | Hexyl | 10 | 4.3068 | 1.4359 | 6.04 | 2.6267 | 4.9093 |
| 85 | Hexyl | 5 | 4.8059 | 0.7590 | 6.38 | 1.442 | 4.4589 |

### Examples 86: Blend polymer of [1-butoxycarbonyl-1-methylethylene/1-(2-(N-2-dodecylbenzylmethylammonioethyl)carbamoyloxy)ethoxycarbonyl)-1-methylethylene iodide] copolymer (95:5 mol%) and [2-hydroxyethyl methacrylate] polymer

First, 2-hydroxyethyl methacrylate polymer was prepared. 2-Hydroxyethyl methacrylate (5.2913 g) was dissolved in 55 ml DMF, and 6.90 mg of AIBN added. The reaction mixture was intensely cooled, air was removed from the reaction vessel, and the mixture heated at 60°C for 48 hours. The reaction solution was then evaporated to dryness under reduced pressure, and the residue taken up in 500 ml water. The resulting precipitate was washed with water over a period of 2 weeks, collected by filtration, and dried under reduced pressure to give 4.8273 g of product. This product was redissolved in 25 ml DMF, the solution again evaporated to dryness under reduced pressure, and, the residue was freeze-dried after being washed with water for 1 day (yield 4.2705 g, 89.1%).

Each 60.11 mg, 36.29 mg and 12.49 mg of the quaternary polymer obtained according to the method of Example 13, and each 59.69 mg, 81.58 mg and 107.28 mg of 2-hydroxyethyl methacrylate) polymer prepared as described hereinabove, were dissolved, respectively each in 12 ml of methanol so as to obtain the desired blend polymer solutions.

### [Experimental Examples]

### Microbicidal tests

The bactericidal action of the compounds of this invention was confirmed by casting a solution of the compound onto glass or a plastic film, by allowing it to come into contact with a suspension containing a fixed number of a microorganism i.e. (Staphylococcus aureus), and by determining the residual viability of the microorganism after predetermined time.

### Experiment 1

### (1) Method

### 1. Sample preparation

Each 1 ml of 1% chloroform solution of the polymers obtained in Examples 6, 12 and 13 was introduced, respectively, in a Pyrex glass testtube. The solvent was distilled off by means of a rotary evaporator so as to form a coating of the polymer on the inside surface of the testtube, which was then dried at 50°C for 2 days.

### 2. Preparation of bacterial suspension

Staphylococcus aureus IFO-12732 was cultured in SCD medium (Nihon Seiyaku) at 35°C for 24 hours. This culture was diluted with sterilized deionised water so as to give a viable cell number of 10²-10⁸ cfu/ml in the final suspension.

### 3. Measurement of bactericidal activity

One ml of the suspension obtained in 2 was added to the testtube prepared in 1, and shaken at 25°C (200 s.p.m.). The number of viable cells in the suspension after predetermined times (0 min, 30 min, 1 hour, 2 hours) was measured by spreading each suspension on SCDLP agar plate and incubating it at 35°C for 48 hours.

### (2) Results

The results obtained are shown in Table 4 and Table 5. Table 5 shows the results of the confirming test performed with more comparison samples.

**Table 4**

| Coating compound | Viability (cfu/tube) | | | |
|---|---|---|---|---|
| Example No. | 0 min | 30 min | 1 hour | 2 hours |
| Example 12 | 64 | 15 | 7 | 2 |
| Example 13 | 63 | 25 | 15 | 2 |
| Example 6 | 56 | 19 | 8 | 1 |
| none | 62 | 63 | 51 | 53 |

**Table 5**

| Coating compound | Viability (cfu/tube) | | | |
|---|---|---|---|---|
| Example No. | 0 min | 30 min | 1 hour | 2 hours |
| Example 12 | 71 | 10 | 3 | 2 |
| Example 13 | 63 | 30 | 12 | 6 |
| Example 6 | 69 | 32 | 17 | 5 |
| *1 | 64 | 68 | 72 | 64 |
| *2 | 63 | 67 | 65 | 63 |
| *1: Coated with poly(butyl methacrylate) *2: None | | | | |

From [Table 4 and Table 5], it is clear that the compounds of this invention have excellent bactericidal activity.

### Experiment 2

The same procedure as in Experiment 1 was followed, except that Pseudomonas aeruginosa IFO-12689 was used instead of Staphylococcus aureus. This culture was diluted with sterilized, deionized water so as to give a bacterial suspension of 10⁴ cfu/ml. The results obtained are shown in Table 6.

**Table 6**

| Coating compound | Viability (cfu/tube) | | | | |
|---|---|---|---|---|---|
| Example No. | 0 min | 15 min | 30 min | 1 hour | 2 hours |
| Example No. 12 | 1.4x10⁴ | 5.1x10³ | 2.7x10³ | 8.4x10² | 1.6x10² |
| Example No. 13 | 1.4x10⁴ | 1.5x10³ | 4.0x10² | 25 | 0 |
| Example No. 14 | 1.1x10⁴ | 6.6x10² | 1.4x10² | 32 | 0 |
| Example No. 15 | 1.1x10⁴ | 1.4x10³ | - | 48 | 1 |
| Example No. 78 | 1.1x10⁴ | 6.9x10² | 1.0x10² | 5 | 0 |
| none | 1.4x10⁴ | 9.0x10³ | 8.6x10³ | 8.7x10³ | 8.4x10³ |

### Experiment 3

The same procedure as in Experiment 1 was followed, except that Paecilomyes variotii IFO-30539 and Aspergillus Niger IFO-4407 were used. Both fungi were cultured on agar stants containing potato infusion and dextrose (Potato Dextrose Agar (Difco Laboratories (USA)). Ten ml of Aerisol OT (American Cyanamid) was then added, and spores and bacterial filaments were scraped off the surface. The suspension obtained was vigorously shaken to separate the spores from the filaments. The resulting suspension was filtered using sterilized cotton to obtain a spore suspension, which was diluted with sterilized water so as to give a predetermined spore concentration (i.e. 10⁴ cfu/ml) in the final suspension. The number of viable cells was measured by incubating this solution on PDA agar plate at 24°C for 48 hours. Tables 8 and 9 show the results obtained with Paecilomyes variotii IFO-30539 and Aspergillus niger IFO-4407, respectively.

**Table 7**

| Coating compound | Viability (cfu/tube) | | | | |
|---|---|---|---|---|---|
| Example No. | 0 min | 15 min | 30 min | 1 hour | 2 hours |
| Example 12 | 1.3x10⁴ | 8.6x10² | 68 | 9 | 2 |
| none | 1.3x10⁴ | 1.5x10⁴ | 1.3x10⁴ | 1.3x10⁴ | 1.1x10⁴ |

**Table 8**

| Coating compound | Viability (cfu/tube) | | | | |
|---|---|---|---|---|---|
| Example No. | 0 min | 15 min | 30 min | 1 hour | 2 hours |
| Example 12 | 8.0x10³ | 9.2x10² | 4.5x10² | 2.7x10² | 43 |
| none | 8.0x10³ | 7.0x10³ | 5.1x10³ | 7.9x10³ | 6.4x10³ |

### Experiment 4

Pseudomonas aeruginosa IFO-12689 was cultured as described in Experiment 2, and this culture was diluted with sterilized, deionized water so as to give a bacterial cell suspension of approx. 10⁶ cfu/ml. Bactericidal activity of the polymers of this invention was tested as in Experiments. The results obtained are shown in Table 9.

**Table 9**

| Coating compound | Viability (cfu/tube) | | | | |
|---|---|---|---|---|---|
| Example No. | 0 min | 15 min | 30 min | 1 hour | 2 hours |
| Example 12 | 6.5x10⁵ | 5.1x10⁴ | 2.0x10⁴ | 1.2x10³ | 33 |
| Example 78 | 6.5x10⁵ | 1.6x10⁵ | 7.7x10⁴ | 5.9x10³ | 3.8x10² |
| Example 76 | 6.5x10⁵ | 1.0x10⁵ | 1.3x10⁴ | 1.9x10² | 0 |
| Example 77 | 6.5x10⁵ | 6.6x10⁴ | 1.8x10⁴ | 1.2x10³ | 63 |

### Experiment 5

A test sample was prepared by impregnating a piece of gauze (approx. 17x17 cm, approx. 0.95 g) with One ml of a 0.5% methanol solution (corresponding to approx. 5 mg) of the compound obtained in Example 12. This was dried at 50°C under reduced pressure for 2 days introduced into a conical flask (100 ml) containing 20 ml of a bacterial cell suspension of approx. 10⁶ cfu of Staphylococcus aureus IFO-12732 by means of a sterilized stick. Incubation was carried out on a reciprocal shaker at a rate of 100 s.p.m. at 25°C for 2 hours. 1 ml of the test solution was then removed, spreaded on SCDLP agar plate, and incubated. The residual no. of viable bacterial cells was measured. Table 10 shows the results obtained. All the data are the average of triplicate flasks. As controls, both a piece of gauze impregnated with 1 ml of methanol and of untreated gauze were used. The initial no. of bacteria was measured to be 8.1x10⁵ cfu/ml.

**Table 10**

| Sample | Viability after 2 hours (cfu/ml) |
|---|---|
| Gauze impregnated with the compound of Example 12 | 10 |
| Gauze impregnated with methanol* | 2.6x10⁴ |
| Untreated gauze* | 3.5x10⁴ |

| | |
|---|---|
| *Controls | |

### Experiment 6

One ml of suspension containing 9.0x10⁵ cfu of Staphylococcus aureus IFO-12732 was allowed to contact with a coating of the polymer blend prepared from the solution of polymers obtained in Example 86 on a glass testtube, at 25°C with shaking (200 s.p.m.) in the same way as in Experiment 1 for 2 hours. The residual no. of viable bacterial cells was measured and found to be 0.

The compound (I) of this invention are excellent antibacterial and antifungal materials with a high level of safety.

## Claims

1. A compound represented by the following formula [1]: 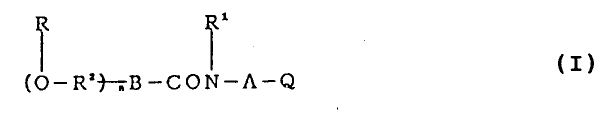 (wherein R is an acyl group which may or may not be substituted, R¹ is a hydrogen atom, a C₁₋₁₀ hydrocarbon group which may or may not be substituted, or an acyl group which may or may not be substituted, R² is a straight chain or branched C₁₋₁₂ alkylene group which may or may not be substituted, A is a straight chain or branched C₁₋₁₀ alkylene group, B is O, NH or S, Q is a tertiary amino or quaternary ammonium group, a nitrogen-containing heterocyclic group which may or may not be substituted, or a nitrogen-containing heterocyclic ammonium group which may or may not be substituted, and n is an integer from 1 to 10).

2. A compound as defined in claim 1 comprising a repeating unit represented by the formula (I), and R is: 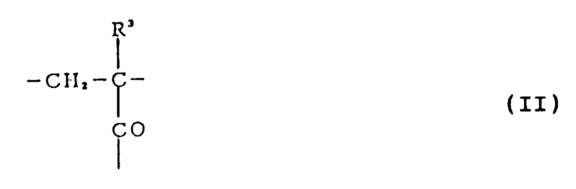 (wherein R³ is a hydrogen atom, or a straight chain or branched C₁₋₆ alkyl group which may or may not be substituted).

3. A compound as defined in claim 1 comprising a repeating unit represented by the formula (I), and R is: 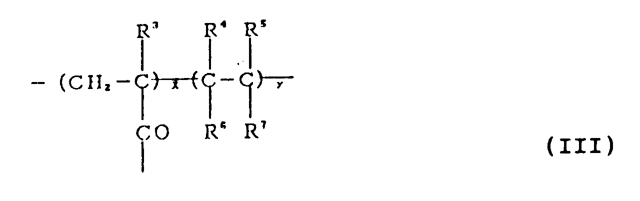 (wherein R³ is a hydrogen atom or a straight chain or branched C1-6 alkyl group, R⁴ is a hydrogen atom, or a straight chain or branched C₁₋₆ alkyl group, R⁵, R⁶, R⁷ are identical or different, and may each respectively be a hydrogen atom, a straight chain or branched C₁₋₆ alkyl group, halogen atom (i.e. chlorine, bromine, iodine or fluorine), cyano group, C₁₋₁₈ alkyl or cycloalkyloxy-carbonyl group, carboxyl group, nitrogen-containing cyclic group which may or may not be substituted, -COOR⁵¹ (wherein R⁵¹ is a hydrogen atom or a hydrocarbon group which may or may not be substituted), -CONR⁵²R⁵³ (wherein R⁵², R⁵³ are respectively a hydrogen atom or a C₁₋₁₀ hydrocarbon group which may or may not be substituted, and R⁵², R⁵³ together form a 4 to 9 membered saturated heterocyclic group with the adjacent nitrogen atom wherein said group may or may not be substituted), -0R⁵⁵ (wherein R⁵⁵ is a hydrogen atom or a C₁₋₁₀ hydrocarbon group which may or may not be substituted), -O-(CH₂CH₂O)ₘ-CH₂CH₂Z (wherein m is an integer from 1 to 10, Z is a hydroxyl, amino, lower acylamino or lower alkoxy group, or a halogen atom), -OCOR⁵⁶ (wherein R⁵⁶ is a hydrogen atom or a C₁₋₁₀ hydrocarbon group which may or may not be substituted), or a group represented by the formula: 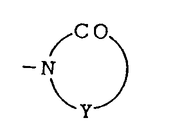 (wherein Y is a C₃₋₁₁ alkylene group which may or may not be substituted), R⁵ and R⁷ may together form a cyclic acid anhydride structure, an N-substituted cyclic imido structure (the substituting group being C₁₋₁₀ alkyl, C₃₋₁₀ cycloalkyl, C₆₋₁₀ aryl, C₇₋₁₀ cycloalkylalkyl or aralkyl) or an unsubstituted cyclic imido structure, and x, y are mole percent).

4. A compound as defined in claim 1 wherein R has the formula: 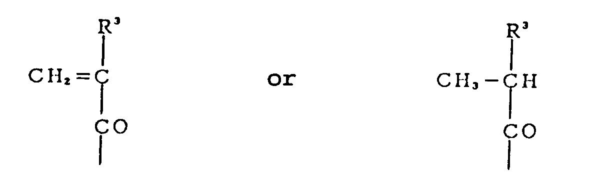 (wherein R³ is a hydrogen atom, or a straight chain or branched C₁₋₆ alkyl group which may or may not be substituted).

5. A compound as defined in claim 1 wherein R¹ is a hydrogen atom, a straight chain or branched C₁₋₁₀ alkyl group, a C₇₋₁₀ aralkyl group, or a straight chain or branched C₂₋₉ alkylcarbonyl group.

6. A compound as defined in claim 1 wherein R¹ is a hydrogen atom, a straight chain or branched C₁₋₄ alkyl group, or a straight chain or branched C₂₋₅ alkylcarbonyl group.

7. A compound as defined in claim 1 wherein R² is a straight chain or branched C₁₋₆ alkylene group.

8. A compound as defined in claim 2 wherein R³ is a hydrogen atom or a methyl group.

9. A compound as defined in claim 3 wherein R⁴ is a hydrogen atom or a methyl group.

10. A compound as defined in claim 3 wherein R⁵ is a hydrogen atom, a cyano group, a halogen atom (i.e. fluorine, chlorine, bromine or iodine), a straight chain or branched C₁₋₆ alkyl group or a C₆₋₁₀ aryl group, or an unsaturated nitrogen-containing 5 to 9 membered cyclic group wherein one or more nitrogen atoms may or may not be substitued, -COOR^{51'} (wherein R^{51'} is a hydrogen atom, a C₁₋₈ alkyl group or a C₇₋₁₀ aralkyl group), -CONR^{52'}R^{53'} (wherein R^{52'}, R^{53'} are both a hydrogen atom, or a C₁₋₆ alkyl, C₃₋₁₀ cycloalkyl, C₇₋₁₀ aralkyl or C₃₋₁₀ cycloalkylalkyl group, and R^{52'}, R^{53'} together form a 4 to 9 membered saturated heterocyclic group with the adjacent nitrogen atom wherein said groups may or may not be substituted), -OR^{55'} (wherein R^{55'} is a hydrogen atom, or a C₁₋₁₀ alkyl, C₆₋₁₀ aryl or C₇₋₁₀ aralkyl group), - OCOR^{56'} (wherein R^{56'} is a C₁₋₁₀ alkyl, C₆₋₁₀ aryl or C₇₋₁₀ aralkyl group), or a group represented by the formula: 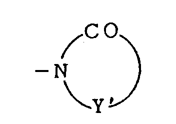 (wherein Y' is a methylene or ethylene group which may or may not be substituted).

11. A compound as defined in claim 3 wherein R⁵ is a hydrogen atom, a C₁₋₆ alkyl group, -COOR^{51''} (wherein R^{51''} is a C₁₋₆ alkyl group which may or may not be substituted by hydroxyl), or a C₆₋₁₀ aryl group.

12. A compound as defined in claim 1 wherein A is a straight chain or branched C₁₋₆ alkylene group.

13. A compound as defined in claim 1 wherein B is O.

14. A compound as defined in claim 1 wherein Q is a group represented by the formula: 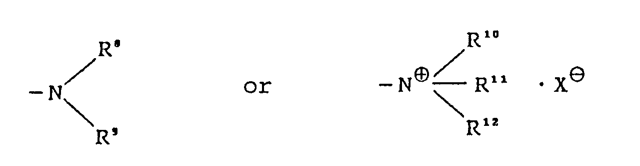 (wherein R⁸, R⁹, R¹⁰, R¹¹ and R¹² are hydrocarbon groups which may or may not be substituted, and X is an anionic group).

15. A compound as defined in claim 1 wherein Q is a 5 or 6 membered saturated or unsaturated nitrogen-containing heterocyclic group which may or may not be substituted, or a nitrogen-containing heterocyclic ammonium group which may or may not be substituted, said groups being represented by the following formula: 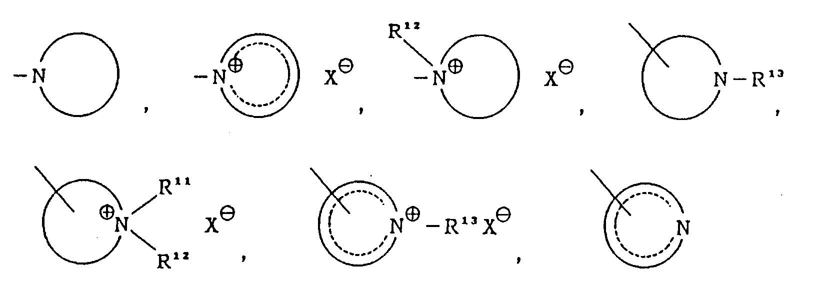 (wherein R¹¹, R¹², R¹³ are hydrocarbon groups which may or may not be substituted, and X is an anionic group).

16. A compound as defined in claim 1 wherein n is an integer from 1 to 3.

17. A compound as defined in claim 3 wherein the ratio x:y lies in the range from 100:0 to 0.01:99.99.

18. A compound as defined in claim 1 wherein the acyl group represented by R may be a straight chain or branched C₂₋₁₉ alkylcarbonyl, C₃₋₁₁ alkenylcarbonyl, C₄₋₈ cycloalkyl carbonyl, C₃₋₇ cyclolalkyl - C₂₋₇ alkylcarbonyl, C₇₋₁₁ aryl carbonyl, C₈₋₁₁ aralkyl carbonyl, C₉₋₁₂ aralkenyl carbonyl, straight chain or branched C₁₋₇ alkylsulfonyl, C₆₋₁₄ aryl sulfonyl, or C₇₋₁₀ aralkyl sulfonyl group, or a 5 to 9 member heterocyclic carbonyl group containing 1 to 3 atoms other than carbon such as O, N or S.

19. A compound as defined in claim 1 wherein R is a straight chain or branched C₂₋₁₁ alkylcarbonyl, or a C₃₋₁₁ alkenyl carbonyl group.

20. A method of manufacturing a compound as defined in claim 1 characterized in that the compound represented by the formula: 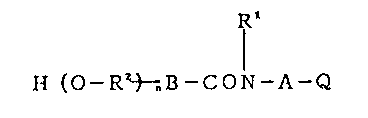 (wherein R¹ is a hydrogen atom, a C₁₋₁₀ hydrocarbon group which may or may not be substituted, or an acyl group which may or may not be substituted, R² is a straight chain or branched C₁₋₁₂ alkylene group which may or may not be substituted, A is a straight chain or branched C₁₋₁₀ alkylene group, B is O, NH or S, Q is a tertiary amino or quaternary ammonium group, a nitrogen-containing heterocyclic group which may or may not be substituted, or a nitrogen-containing heterocyclic ammonium group which may or may not be substituted, and n is an integer from 1 to 10), is made to undergo an acylation reaction, and then, if necessary, an alkylation reaction.

21. A method of manufacturing a compound as defined in claim 1 characterized in that a compound represented by the formula:
R-(O-R²-)ₙ-BH
(wherein R is an acyl group which may or may not be sustituted, R² is a straight chain or branched C₁₋₁₂ alkylene group which may or may not be substituted, B is O, NH or S), and a compound represented by the formula:
OCN-A-Q
(wherein A is a straight chain or branched C₁₋₁₀ alkylene group, and Q is a tertiary amino or quaternary ammonium group, a nitrogen-containing heterocyclic group which may or may not be substituted, or a nitrogen-containing heterocyclic ammonium group which may or may not be substituted), or a salt of the same, are reacted together, and then, if necessary, the product is made to undergo an alkylation reaction.

22. A method of manufacturing a compound as defined in claim 1 characterized in that a compound represented by the formula:
R-(O-R²-)ₙ-OH
(wherein R is an acyl group which may or may not be sustituted, R² is a straight chain or branched C₁₋₁₂ alkylene group which may or may not be substituted, and n is an integer from 1 - 10), and a compound represented by the formula: 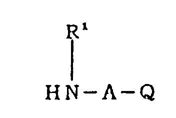 (wherein R¹ is a hydrogen atom, a C₁₋₁₀ hydrocarbon group which may or may not be substituted or an acyl group which may or may not be substituted, A is a straight chain or branched C₁₋₁₀ alkylene group, and Q is a tertiary amino or quaternary ammonium group, a nitrogen-containing heterocyclic group which may or may not be substituted, or a nitrogen-containing heterocyclic ammonium group which may or may not be substituted), or a salt of the same, are reacted, and then, if necessary, the product is made to undergo an alkylation reaction.

23. A method of manufacturing a compound as defined in claim 1 characterized in that a compound represented by the formula:
R-(O-R²-)ₙ-NCO
(wherein R is an acyl group which may or may not be substituted, R² is a straight chain or branched C₁₋₁₂ alkylene group which may or may not be substituted, and n is an integer from 1 - 10), and a compound represented by the formula: 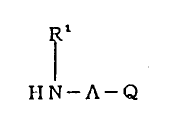 (wherein R¹ is a hydrogen atom, a C₁₋₁₀ hydrocarbon group which may or may not be substituted or an acyl group which may or may not be substituted, A is a straight chain or branched C₁₋₁₀ alkylene group, and Q is a tertiary amino or quaternary ammonium group, a nitrogen-containing heterocyclic group which may or may not be substituted, or a nitrogen-containing heterocyclic ammonium group which may or may not be substituted), or a salt of the same, are reacted, and then, if necessary, the product is made to undergo an alkylation reaction.

24. A method of manufacturing a compound as defined in claim 2 characterized in that a compound represented by the formula: 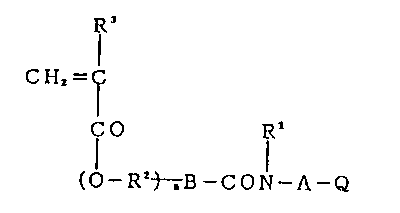 (wherein R¹ is a hydrogen atom, a C₁₋₁₀ hydrocarbon group which may or may not be substituted or an acyl group which may or may not be substituted, R² is a straight chain or branched C₁₋₁₂ alkylene group which may or may not be substituted, R³ is a hydrogen atom or a straight chain or branched C₁₋₆ alkyl group which may or may not be substituted, A is a straight chain or branched C₁₋₁₀ alkylene group, B is O, NH or S, Q is a tertiary amino or quaternary ammonium group, a nitrogen-containing heterocyclic group which may or may not be substituted, or a nitrogen-containing heterocyclic ammonium group which may or may not be substituted, and n is an integer from 1 to 10), is made to undergo a polymerization reaction either in the presence or in the absence of copolymerizable compounds, and then, if necessary, the product is made to undergo an alkylation reaction.

25. A method of manufacturing a compound as defined in claim 3 characterized in that a compound represented by the formula: 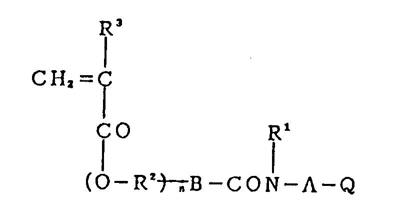 (wherein R¹ is a hydrogen atom, a C₁₋₁₀ hydrocarbon group which may or may not be substituted or an acyl group which may or may not be substituted, R² is a straight chain or branched C₁₋₁₂ alkylene group which may or may not be substituted, R³ is a hydrogen atom or a straight chain or branched C₁₋₆ alkyl group which may or may not be substituted, A is a straight chain or branched C₁₋₁₀ alkylene group, B is O, NH or S, Q is a tertiary amino or quaternary ammonium group, a nitrogen-containing heterocyclic group which may or may not be substituted, or a nitrogen-containing heterocyclic ammonium group which may or may not be substituted, and n is an integer from 1 to 10), is made to undergo a polymerization reaction with a compound represented by the formula: 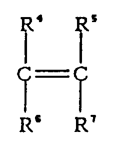 (wherein R⁴ is a hydrogen atom, or a straight chain or branched C₁₋₆ alkyl group, R⁵, R⁶, R⁷ are identical or different, and each may respectively be a hydrogen atom, a straight chain or branched C₁₋₆ alkyl group, halogen atom (i.e. chlorine, bromine, iodine or fluorine), cyano group, C₁₋₁₈ alkyl or cycloalkyloxycarbonyl group, carboxyl group, nitrogen-containing cyclic group which may or may not be substituted, -COOR⁵¹ (wherein R⁵¹ is a hydrogen atom or a hydrocarbon group which may or may not be substituted), -CONR⁵²R⁵³ (wherein R⁵², R⁵³ may be same or different and each may be a hydrogen atom or a C₁₋ ₁₀ hydrocarbon groups which may or may not be substituted, and R⁵², R⁵³ together form a 4 to 9 membered saturated heterocyclic group with the adjacent nitrogen atom wherein said groups may or may not be substituted), -0R⁵⁵ (wherein R⁵⁵ is a hydrogen atom or a C₁₋₁₀ hydrocarbon group which may or may not be substituted), -O-(CH₂CH₂O)ₘ-CH₂CH₂Z (wherein m is an integer from 1 to 10, Z is a hydroxyl, amino, lower acylamino or lower alkoxy group, or a halogen atom), -OCOR⁵⁶ (wherein R⁵⁶ is a hydrogen atom or a C₁₋₁₀ hydrocarbon group which may or may not be substituted), or a group represented by the formula: 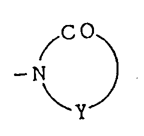 (wherein Y is a C₃₋₁₁ alkylene group which may or may not be substituted), and R⁵ and R⁷ may together form a cyclic acid anhydride structure, an N-substituted cyclic imido structure (the substitutent being C₁₋₁₀ alkyl, C₃₋₁₀ cycloalkyl, C₆₋₁₀ aryl, C₇₋₁₀ cycloalkylalkyl or aralkyl) or an unsubstituted cyclic imido structure).

26. An antibacterial, antifungal composition characterized in that it contains at least one type of compound as defined in claim 1, 2, 3 or 4.
